(19) Europäisches Patentamt / European Patent Office / Office européen des brevets

(11) **EP 4 644 408 A1**

(12) **EUROPEAN PATENT APPLICATION**

(43) Date of publication:
**05.11.2025   Bulletin 2025/45**

(21) Application number: 25170849.1

(22) Date of filing: **15.04.2025**

(51) International Patent Classification (IPC):
*C07K 14/325* (2006.01)     *C12N 11/00* (2006.01)
*C12N 15/62* (2006.01)      *C12P 13/04* (2006.01)

(52) Cooperative Patent Classification (CPC):
**C07K 14/325; C12N 11/00; C12N 15/62;
C12P 13/04**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:   **19.04.2024   US 202463636314 P**

(71) Applicant: **The Chinese University of Hong Kong
Office of
Research and Knowledge Transfer Services
(ORKTS)
Sha Tin, New Territories Hong Kong (HK)**

(72) Inventors:
• **CHAN, Michael
Kowloon (CN)**

• **LEE, Marianne
Kowloon (CN)**
• **YEKTA, Reza
Shatin (CN)**
• **XIONG, Xu
Fanling (CN)**

(74) Representative: **Geling, Andrea
ZSP Patentanwälte PartG mbB
Hansastraße 32
80686 München (DE)**

Remarks:
The complete document including Reference
Table(s) and the Sequence Listing(s) can be
downloaded from the EPO website

(54) **POLYETHYLENIMINE-MODIFIED CRY PROTEINS AND THEIR USE**

(57)    The present invention provides a system for immobilizing recombinantly produced proteins, especially enzymes useful for catalyzing various chemical reactions, and cofactors required for the reactions by way of polyethylenimine (PEI)-modified protein crystals. Related compositions and as well as methods of making and using the immobilized proteins and cofactors are also described.

EP 4 644 408 A1

**Description**

RELATED APPLICATIONS

This application claims priority to U.S. Provisional Application No. 63/636,314, filed April 19, 2024, the contents of which are hereby incorporated by reference in the entirety for all purposes.

BACKGROUND OF THE INVENTION

In many medical and industrial contexts the use of recombinant proteins is becoming increasingly more important. The production, isolation, use, and potential reuse of recombinant proteins, especially enzymes, remain in need of improvement for better quality, efficiency, and stability. For instance, enzyme immobilization can facilitate easy removal and subsequent reuse of enzymes during multiple rounds of catalysis. In many cases, immobilization also improves the stability of enzymes against many industrial conditions such as high temperatures and organic solvents. Immobilization by entrapment is particularly attractive since it does not involve any modifications to the enzyme structure, increasing the chance for high activity retention and native enzyme conformation. Thus, there exists a need for new and effective means for producing recombinant proteins, such as enzymes, in immobilized form. The present invention fulfills this and other related needs.

BRIEF SUMMARY OF THE INVENTION

In a first aspect, this invention provides a polyethylenimine (PEI)-modified protein crystal. The protein crystal may be formed by a Cry protein, a crystal-forming variant of a Cry protein, a crystal-forming fragment of a Cry protein, or a fusion polypeptide comprising the Cry protein, the crystal-forming variant, or the crystal-forming fragment.

In some embodiments, the Cry protein in the claimed PEI-modified protein crystal is Cry3Aa or Cry1Ab. In some embodiments, the PEI-modified protein crystal is formed by a fusion polypeptide comprising (1) a Cry protein, a crystal-forming variant of the Cry protein, or a crystal-forming fragment of the Cry protein, which is fused to (2) a heterologous protein. In some embodiments, the PEI-modified protein crystal is formed by a Cry protein, a crystal-forming variant of the Cry protein, or a crystal-forming fragment of the Cry protein and comprises a heterologous protein entrapped within the protein crystal. In some embodiments, the PEI-modified protein crystal comprises a fused or trapped heterologous protein, which is an enzyme, for example, a formate dehydrogenase (FDH), leucine dehydrogenase (LDH), human arginase (hArg), ornithine decarboxylase (ODC), alcohol dehydrogenase (ADH), mandelate dehydrogenase (MDH), carbonyl reductase (CBR), or reductive aminase (RedAm), or cytochrome P450 (CYP or P450), including a functional variant of any of the above. In some embodiments, the fusion polypeptide in the claimed PEI-modified protein crystal comprises a Cry protein, a crystal-forming variant of the Cry protein, or a crystal-forming fragment of the Cry protein fused to an enzyme. For example, the fusion polypeptide is a Cry3Aa-ADH fusion, Cry3Aa-FDH fusion, Cry3Aa-LDH fusion, Cry3Aa-MDH fusion, Cry3Aa-P450 fusion, Cry1Ab-hArg fusion, Cry1Ab-ODC fusion, or Cry1Ab-P450 fusion, including Cry-fusion of functional variants of these enzymes. In some embodiments, the PEI-modified protein crystal is bound with a cofactor required for a chemical reaction catalyzed by the enzyme. In some embodiments, the cofactor is a phosphorylated cofactor. For example, the phosphorylated cofactor is nicotinamide adenine dinucleotide (NADH), flavin adenine dinucleotide (FADH), or pyridoxal phosphate (PLP). In some embodiments, the PEI-modified protein crystal comprises an enzyme, which is an ADH, FDH, LDH, MDH, CBR, RedAm, or P450, and is bound with the phosphorylated cofactor NADH. In some embodiments, the PEI-modified protein crystal comprises the enzyme, an ODC or a functional variant of ODC, and is bound with the phosphorylated cofactor PLP. In some embodiments, the PEI-modified protein crystal comprises two fusion polypeptides, for example, Cry3Aa-FDH and Cry3Aa-ADH; or Cry3Aa-FDH and Cry3Aa-LDH.

In a second aspect, the present invention provides a composition comprising the PEI-modified protein crystal described above and herein. In some embodiments, the protein crystal is embedded in agarose, *e.g.*, agarose beads. In some embodiments, the composition comprises a PEI-modified protein crystal, which comprises an enzyme capable of catalyzing a substrate in a chemical reaction and a cofactor required for the reaction, and wherein composition further comprises the substrate. For example, the protein crystal is formed by a fusion polypeptide comprising a Cry protein fused to an ADH, FDH, LDH, MDH, or P450. In some embodiments, the PEI-modified protein crystal is formed by a fusion polypeptide comprising a Cry protein fused to an hArg or ODC. In some embodiments, the PEI-modified protein crystal is formed by two fusion polypeptides, *e.g.*, Cry3Aa-FDH and Cry3Aa-ADH); or Cry3Aa-FDH and Cry3Aa-LDH. In some embodiments, the PEI-modified protein crystal is bound with NADH. In some embodiments, the PEI-modified protein crystal is bound with PLP.

In a third aspect, the present invention provides a method for performing a chemical reaction. The method comprises the step of incubating the PEI-modified protein crystal of this invention as described above and herein, which comprises an enzyme, with a substrate to the enzyme under conditions permissible for the substrate to be catalyzed by the enzyme in the chemical reaction. For example, the enzyme is a formate dehydrogenase (FDH), leucine dehydrogenase (LDH), human arginase (hArg), ornithine decarboxylase (ODC), alcohol dehydrogenase (ADH), mandelate dehydrogenase (MDH), carbonyl reductase (CBR), or reductive aminase (RedAm), or cytochrome P450 (CYP or P450), including any functional variants of the above. In some embodiments, the PEI-modified protein crystal comprises an enzyme, which is an ADH, FDH, LDH, MDH, CBR, RedAm, or P450, including a functional variant thereof, and the protein crystal is bound with NADH. In some embodiments, the PEI-modified protein crystal comprises the enzyme, an ODC or its functional variant, and the protein crystal is bound with PLP. In some embodiments, the PEI-modified protein crystal comprises two fusion polypeptides, *e.g.*, Cry3Aa-FDH and Cry3Aa-ADH, or Cry3Aa-FDH and Cry3Aa-LDH. In some embodiments, the method further comprises, after the reaction is completed, a step of removing the reaction product and reusing the crystal in a subsequent reaction.

In a fourth aspect, the present invention provides an *in vitro* method for immobilizing a cofactor by incubating the cofactor in a solution with the PEI-modified protein crystal of this invention as described above and herein and allowing the cofactor to bind to the protein crystal. In some embodiments, the cofactor is NADH or PLP. In some embodiments, the method further comprises, after the cofactor is bound to the protein crystal, a step of shaking or stirring or otherwise agitating (*e.g.,* by mechanical or manual means) the solution to release the cofactor from the protein crystal. In some embodiments, the method further comprises, after the cofactor is released from the protein crystal, a step of discontinuing shaking or stirring or otherwise agitating the solution to allow the cofactor to again bind to the protein crystal.

In a fifth aspect, this invention provides a method of delivering a protein of interest to a target cell by way of contacting the target cell with a pre-formed complex between the protein of interest and a PEI-modified protein crystal as described herein, especially in the first aspect of this invention as described above. In some embodiments, the protein crystal is a PEI-modified Cry3Aa protein crystal. In some embodiments, the protein of interest is a negatively charged protein or a protein comprising a negatively charged domain or connected with a negatively charged moiety, for example, a Cas9 ribonucleoprotein (RNP) pre-loaded with at least one small guide RNA (sgRNA). In some embodiments, this delivery method further includes, before the contacting step, a step of incubating the protein of interest with the PEI-modified protein crystal to allow them to bind with each other to form a complex. In some embodiments, the protein of interest is a nuclear protein, and wherein the contacting step is performed in the presence of an endosomolytic agent, such as chloroquine.

BRIEF DESCRIPTION OF THE DRAWINGS

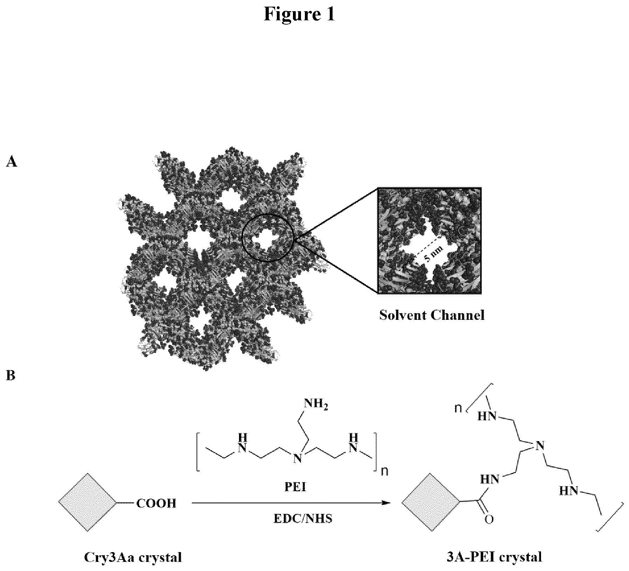

Figure 1. PEI mediated cationization of Cry3Aa crystals. Figure 1(A) shows the distribution of negatively charged residues (Asp+Glu) in red color and bulky shape throughout Cry3Aa crystal (PDB code: 1DLC). Figure 1(B) represents EDC/NHS mediated coupling of PEI with the carboxylic acid present in Cry3Aa crystals (Asp, Glu, and the C-terminus).

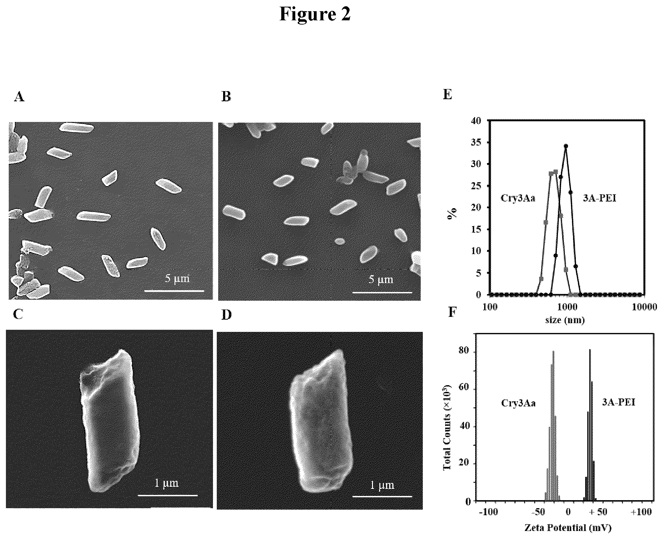

Figure 2. Characterization of 3A-PEI crystals: Fig. 2(A-D) SEM imaging of Cry3Aa (a) and 3A-PEI (b) crystals at low magnifications (5000×), (c) a single Cry3Aa crystal, and (d) a single 3A-PEI crystal with 150000× magnifications. Fig. 2(E) Size distribution and Fig. 2(F) Zeta potential of Cry3Aa crystals before and after PEI mediated cationization using a MALVERN Zetasizer Nano.

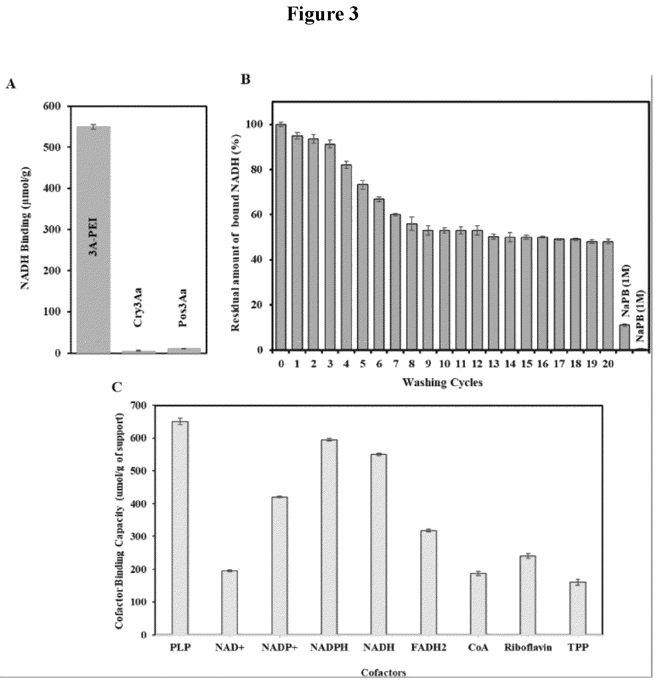

Figure 3. Binding and retention of cofactors in 3A-PEI crystals: Fig. 3A displays the NADH binding capacity of 3A-PEI, Cry3Aa and Pos3Aa crystals. Fig. 3B reveals the retention of NADH bound to 3A-PEI crystals over multiple washing cycles with NaPB (10 mM, pH:7.0). Bound NADH to 3A-PEI crystals were completely removed finally using 1M NaPB (pH:7.0). Fig. 3C indicates the binding capacity of cofactors (PLP, $NAD^+$, $NADP^+$, NADPH, $FADH_2$, CoA, Riboflavin, and TPP) in comparison with NADH. Among the cofactors, PLP exhibits the strongest binding affinity (650 $\pm 2$ $\mu$mol/g of support), whereas TPP demonstrates the weakest binding affinity (160$\pm 1$ $\mu$mol/g of support) towards 3A-PEI crystals.

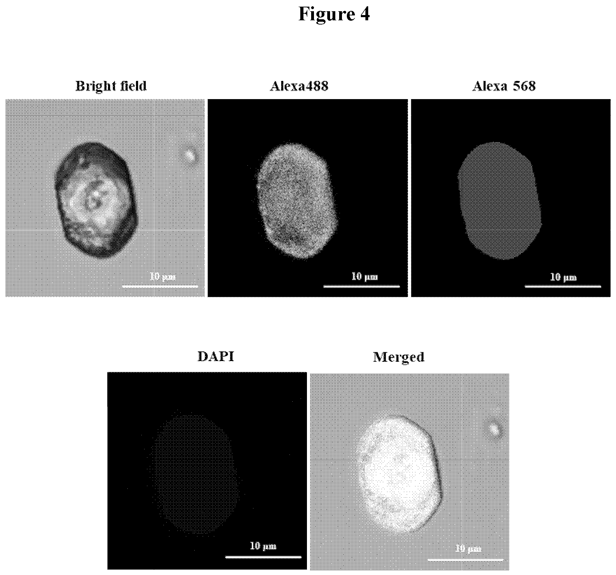

Figure 4. Confocal fluorescence microscopy of NADH loaded 3A-PEI crystal. Cry3Aa proteins was labeled with Alexa568 (red color), PEI polymer tagged with Alexa488 (green color), and bound NADH molecules exhibited blue fluorescence. The crystal was grown under *in vitro* condition.

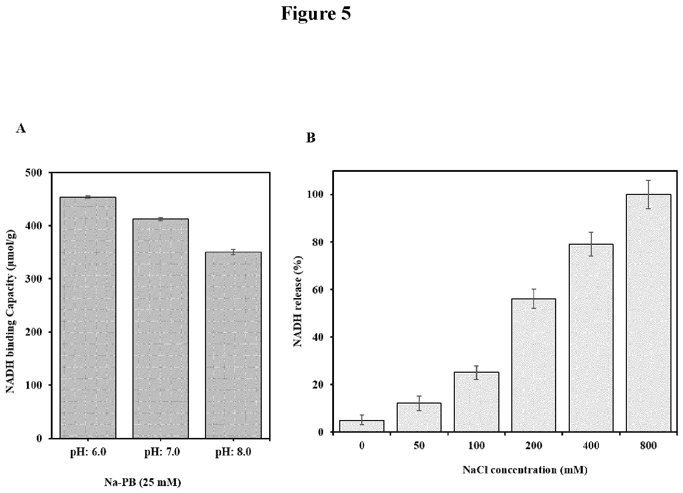

Figure 5. The effects of pH and ionic strength on NADH binding into 3A-PEI crystals. Fig. 5A indicates the effect of pH on NADH binding capacity of 3A-PEI crystals in 25 mM Na-PB. Fig. 5B displays the effect of different concentrations of NaCl (0-800 mM) on the release rate of NADH from 3A-PEI crystals in Na-PB buffer (25 mM, pH:7.0).

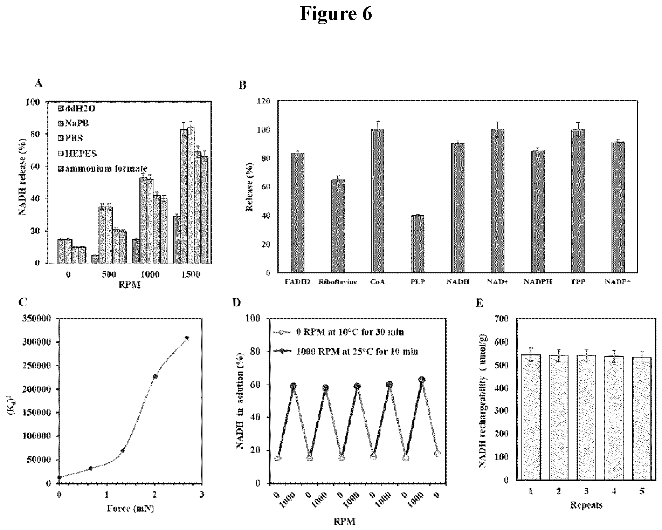

**Figure 6. The role of orbital shaking on binding and release of cofactors within 3A-PEI crystals. Fig. 6A** displays the effect of different mechanical stirring (0-2000 RPM) on the release rate of NADH in different solutions from 3APEI crystals. The buffer concentrations for all buffers were standardized to 10 mM, and the pH values were adjusted to 7.0, with the exception of ammonia formate, which was adjusted to a pH of 8.0. It is shown that by increasing the mechanical stirring, the NADH release rate rises remarkably, while when the mechanical stirring is not applied, around 15% release for phosphate-based buffers and near to 10% for HEPES and ammonia formate buffers can be seen due to lixiviation. **Fig. 6B** indicates the release of phosphorylated cofactors from 3A-PEI crystals into the solution under the mechanical shaking (1000 rpm) in sodium phosphate buffer (10 mM, pH 7.0). **Fig. 6C** reveals the association of relative force vs $(K_d)^2$, which are listed in Table 2. The results the when the relative force reach to almost 1.8mN, 50% of bound NADH molecules can be released from 3APEI crystals in Na-PB buffer (10mM, pH:7.0), while when the force removed the $(K_d)^2$ value decrease significantly, indicating association. **Fig. 6D** illustrates the rebinding of the NADH molecules in the same solution after elimination of mechanical force by decrease the shaking speed from 1000 rpm to 0 rpm. Release and rebinding of NADH molecules can be adjusted by the shaking speed, although the role of temperature for binding stabilization should not be neglected. **Fig. 6E** indicates the rechargeability of 3A-PEI crystals after complete elimination of bound NADH molecules by using high concentration of salt.

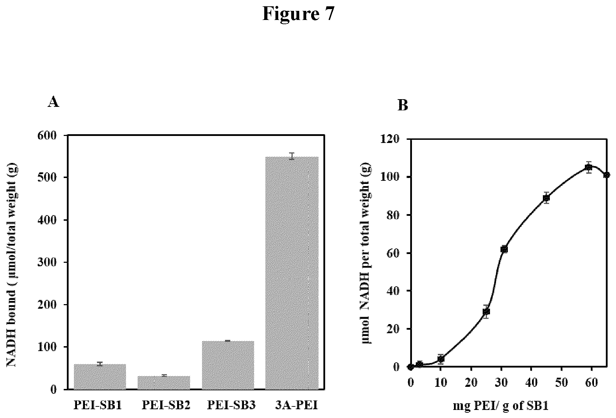

**Figure 7. Comparison of NADH binding capacity into different SB-PEI beads. Fig. 7A** indicates the binding capacity of NADH to PEI-SB1, PEI-SB2, and PEI-SB3 beads with the same amount of anchored PEI. It is obvious that PEI-SB3 beads have almost twofold higher NADH binding capacity in relative to the others. **Fig. 7B** displays the relationship of PEI anchored to the SBs and their capacity for NADH immobilization. By increase the amount of bound PEI from 0-59 mg/g of support, the NADH binding was considerably increased, through further increase ( more than 60 mg/g) can reduce the NADH immobilization due to probably less volume for NADH occupation.

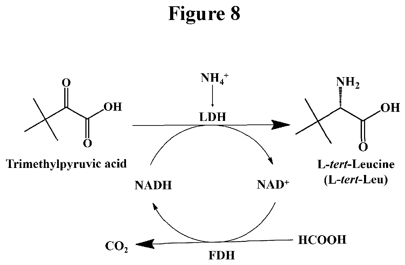

**Figure 8** illustrates regeneration of NADH molecules by oxidation of formic acid into $CO_2$.

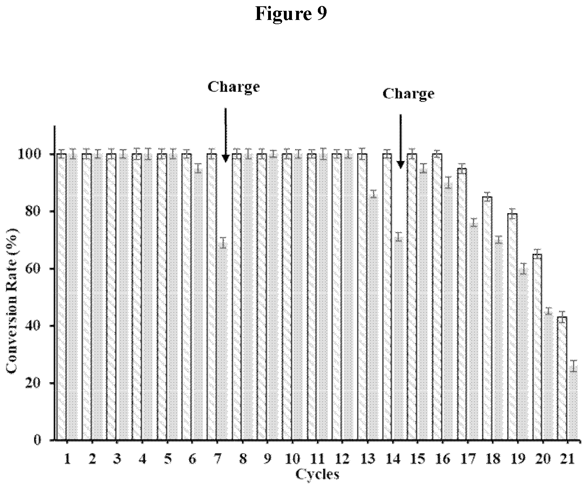

**Figure 9 Recyclability of 3AFL+3A-PEI-NADH crystals for biosynthesis of L-*tert* Leu.** 3AFL with access to free NADH is shown in pattern bar, while 3AFL+3APEI-NADH mixtures is indicated solid bar. The reactions were performed in a thermomixer (Eppendorf ThermoMixer® C) under mechanical stirring (1000 RPM) at 25°C in a reaction buffer contains 25 mM TMP in 30 mM ammonia formate (pH:8.0) with subsequent stationary state for 30 min at 10°C. The amount of NADH was added to each cycle in 3AFL reaction was 0.5 μmol, while this value was 0.5 μmol at first cycle and 0.2 μmol at cycle 7 and cycle 14 for 3AFL+3APEI-NADH mixture.

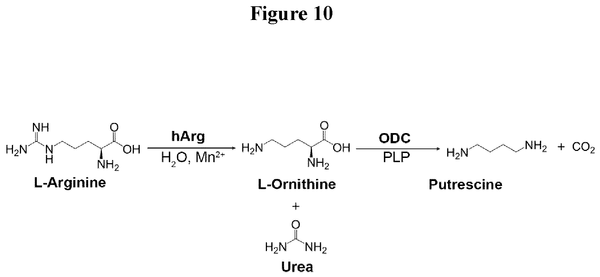

**Figure 10.** Scheme for the hArg and ODC-mediated conversion of L-arginine to putrescine. In this pathway, hArg acts to convert L-arginine to produce L-ornithine, which is then transformed to putrescine by PLP-dependent ODC.

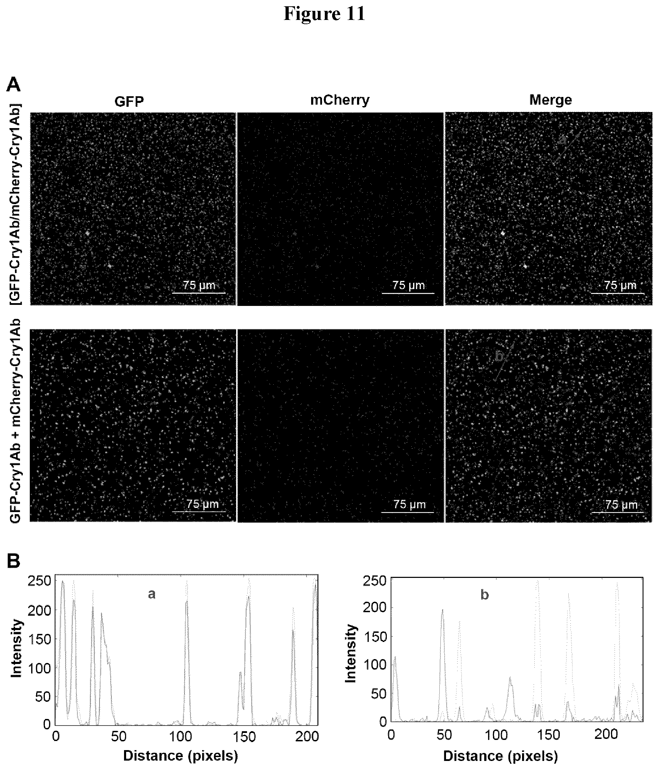

**Figures 11A-B** Characterization of Cry1Ab-mediated co-immobilization of GFP and mCherry. **Figure 11A:** Confocal microscopy performed to examine the flurosecence signal distribution of the co-expressed [GFP-Cry1Ab/mCherry-Cry1Ab] particles (upper panel) and the mixture of individually immobilized GFP-Cry1Ab and mCherry-Cry1Ab particles (lower panel). The yellow punctates indicated the overlapping of GFP and mCherry signals. **Figure 11B:** Corresponding fluorescence intensity profiles of the co-expressed [GFP-Cry1Ab/mCherry-Cry1Ab] particles (left) and the mixture of individually immobilized GFP-Cry1Ab and mCherry-Cry1Ab particles (right) measured along the solid magenta line a and line b in (A) using ImageJ software, respectively. The green curve and red curve represented GFP signal and mCherry signal, respectively. Green: GFP-Cry1Ab particles; red: mCherry-Cry1Ab particles.

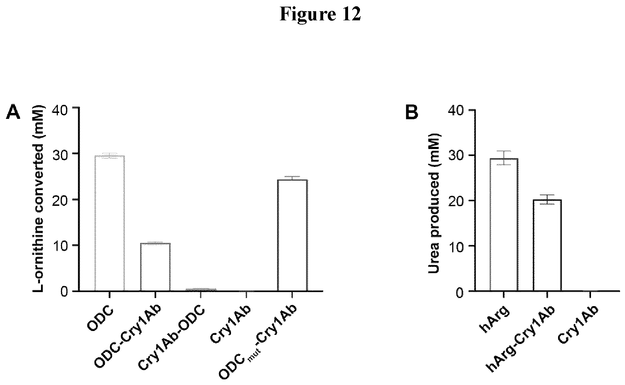

**Figure 12.** Activities of Cry 1Ab-mediated individually immobilized particles. **Figure 12A:** Comparison of the activities of ODC-Cry1Ab and Cry1Ab-ODC as well as $ODC_{mut}$-Cry1Ab particles. 1 μM of Cry1Ab ODC fusion particle (0.21 mg/mL) or ODC enzyme (0.079 mg/mL) was used for the reaction. The activity of ODC was based on the depletion of L-ornithine. The activities of free ODC and Cry1Ab were taken as positive and negative controls. $ODC_{mut}$-Cry1Ab was generated by introducing the double mutations (I163T/E165T) into wild-type ODC by site-directed mutagenesis.
**Figure 12B:** The activity of hArg-Cry1Ab particles. 1 μM of hArg-Cry1Ab fusion particle (0.165 mg/mL) or hArg enzyme (0.035 mg/mL) was used for the reaction. Their activities of hArg were evaluated based on the amount of urea produced. The activities of free hArg and Cry1Ab were taken as positive and negative controls. All experiments were performed in triplicate. Data are presented as mean ± standard deviation.

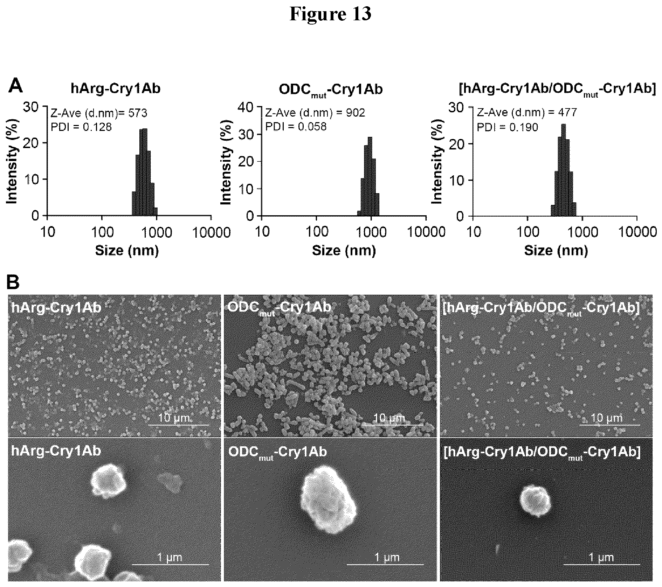

**Figure 13.** Characterization of Cry 1 Ab-immobilized enzymes. **Figure 13A:** DLS of Cry1Ab-immobilized particles. 25 μg/mL of particles resuspended in aqueous solution were used for measurement. **Figure 13B:** SEM of Cry1Ab-immobilized particles at 12,000 × (upper panel) and 80,000 × magnification (lower panel). The scale bars for the upper panel and lower panel are 10 μm and 1 μm, respectively.

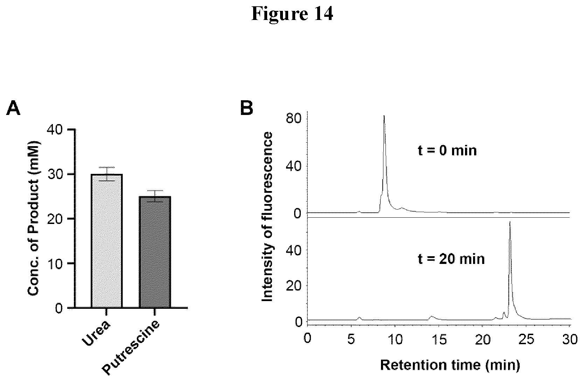

**Figure 14.** Activity of co-immobilized particles. **Figure 14A:** Determination of putrescine produced by indirect method. 0.5 mg/mL co-immobilized [hArg-Cry1Ab/ODC$_{mut}$-Cry1Ab] particles was incubated with 30 mM of L-arginine at 40°C for 20 min. The supernatant was used to determine the urea produced and L-ornithine remaining as described previously. The activities of hArg and ODC$_{mut}$ within the co-immobilized [hArg-Cry1Ab/ODC$_{mut}$-Cry1Ab] particles were based on the urea produced and putrescine produced, respectively. The amount of putrescine produced was indirectly determined by the subtraction of the L-ornithine remaining from the total amount of urea produced. **Figure 14B:** Direct detection of putrescine produced by HPLC method. The reaction solutions used in (A) were derivatized with OPA and then analyzed by HPLC. At 0 min and 20 min, the samples peaked at 8.8 min and 23.4 min, which corresponded to the elution profiles of standards of L-arginine and putrescine, respectively.

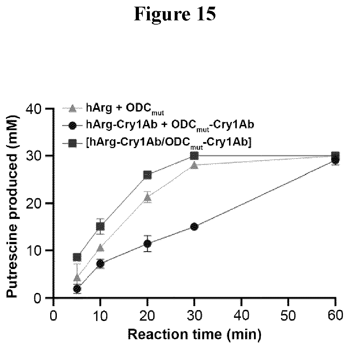

**Figure 15.** Comparison of enzyme activities of immobilized particles. The reactions of 0.5 mg/mL co-immoblized [hArg-Cry1Ab/ODC$_{mut}$-Cry1Ab] particles (red square), a mixture of 0.27 mg/mL hArg-Cry1Ab and 0.23 mg/mL ODC$_{mut}$-Cry1Ab particles (blue circle) and a mixture of 0.057 mg/mL hArg and 0.087 mg/mL ODC$_{mut}$ enzymes (green triangle) were conducted using 30 mM L-arginine as substrate at 40°C. The activity was based on the production of putrescine which was determined by indirect method as described previously. All experiments were performed in triplicate. Data are presented as mean $\pm$ standard deviation.

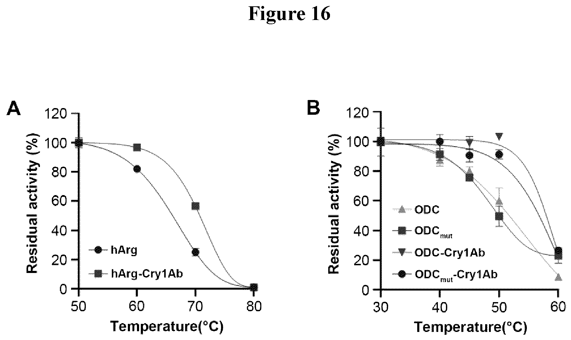

**Figure 16.** Thermostability of Cry1Ab immobilized particles and free enzymes. **Figure 16A:** Thermostability of hArg enzyme and hArg-Cry1Ab particles. 1 $\mu$M of hArg enzyme (0.035 mg/mL) and hArg-Cry1Ab fusion particle (0.165 mg/mL) were incubated at 50°C for 1 h before measuring the residual activity. The residual activity at 50°C was taken as 100%. **Figure 16B:** Thermostability of ODC and ODC$_{mut}$ enzymes, as well as their Cry1Ab-immobilized counterparts. 1 $\mu$M of ODC and ODC$_{mut}$ enzymes (0.079 mg/mL), as well as their Cry1 Ab-immobilized counterparts (0.21 mg/mL) were used in the studies. The residual activity at 30°C was taken as 100%. All the data were fitted to sigmoidal model using GraphPad Prism software. All experiments were performed in triplicate. Data are presented as mean $\pm$ standard deviation.

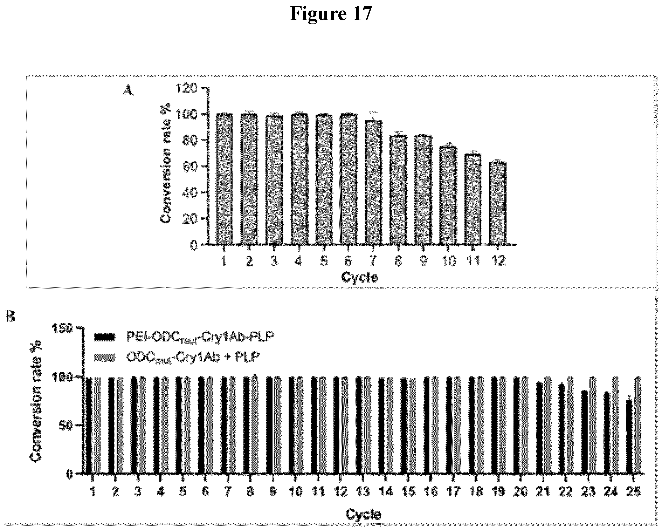

**Figure 17.** Recyclability of co-immobilized multienzymes or a single enzyme and its cofactor. **Fig. 17A:** Recyclability of Cry1Ab-mediated co-immobilized [hArg-Cry1Ab/ODC$_{mut}$-Cry1Ab] particles. 2 mg/mL of particles and 30 mM of L-arginine in 50 mM of HEPES buffer (pH 8.0) containing 0.1 mM PLP were incubated at 40°C and 750 rpm for 30 min in each cycle. After 12 cycles, the total turnover number (TTN) of the rate-limiting enzyme - ODC$_{mut}$ reached 73,284, suggesting that Cry1Ab-mediated co-immobilized particles were highly recyclable. **Fig. 17B:** Recyclability of PEI modified-ODC$_{mut}$-Cry1Ab. For PEI-ODC$_{mut}$-Cry1Ab particles, 0.14 $\mu$mol of PLP was bound prior to the recyclability studies, while for the control group (ODC$_{mut}$-Cry1Ab + PLP), 0.14 $\mu$mol of PLP was added to the reaction solution containing 4 mg/mL of particles and 10 mM of L-ornithine in 10 mM of HEPES buffer (pH 7.0) in each cycle. After 25 cycles, the TTN of PLP for the PEI-modified particles and the particles without modification were 1,736 and 71, respectively, indicating that the particles after PEI modification were cost-efficient and highly reusable. All experiments were performed in triplicate. Data are presented as mean $\pm$ standard deviation.

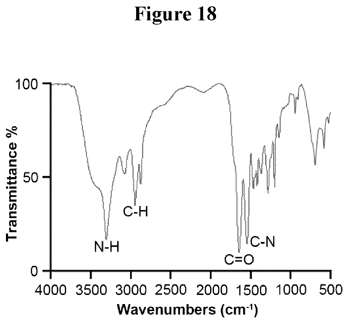

**Figure 18.** FTIR of nylon produced. Nylon produced *in situ* was washed and vacuum dried before FTIR analysis. Absorption peaks at 3300 cm$^{-1}$, 2940 cm$^{-1}$ and 2870 cm$^{-1}$, 1640 cm$^{-1}$ indicated the presence of N-H, C-H, C=O, respectively. The peak at 1540 cm$^{-1}$, corresponding to C-N stretching vibration, indicating that the chemical reaction had occurred between putrescine and adipoyl chloride.

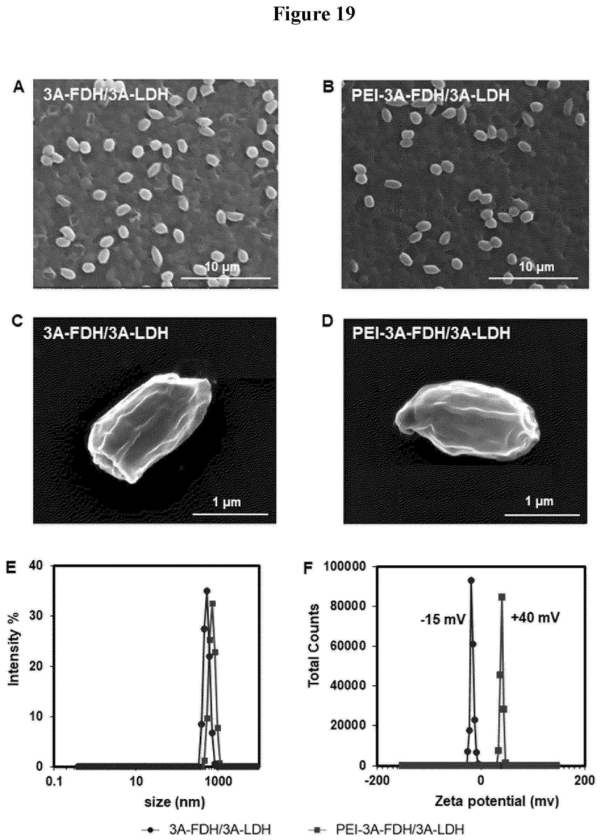

**Figure 19. Characterization of PEI-3A-FDH/3A-LDH particles.** SEM imaging of 3A-FDH/3A-LDH particles and PEI-3A-FDH/3A-LDH particles at 10,000$\times$ (A, B) and 150,000$\times$ (C, D) magnification, respectively. (E) Size distribution and (F) zeta potential of 3A-FDH/3A-LDH particles before (dark blue) and after PEI cationization (red).

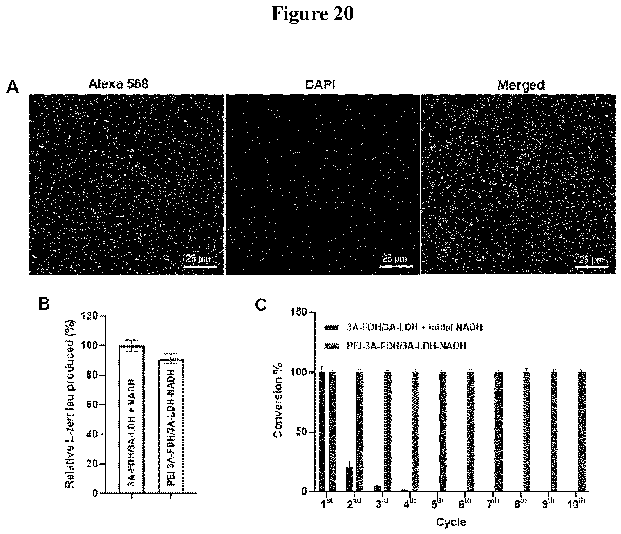

**Figure 20. The co-immobilization and retention of NADH within PEI-3A-FDH/3A-LDH. Fig. 20A:** Confocal images of fluorescent dye-labeled PEI-3A-FDH/3A-LDH-NADH particles. The red signals represent Alexa 568-labelled 3A-FDH/3A-LDH particles. NADH exhibited autofluorescence in the DAPI channel. **Fig. 20B:** L-*tert* Leu production by 3A-FDH/3A-LDH-NADH particles before and after modification with PEI. **Fig. 20C:** Recyclability of PEI-3A-FDH/3A-LDH particles and 3A-FDH/3A-LDH supplied with NADH at the start of cycle 1 only.

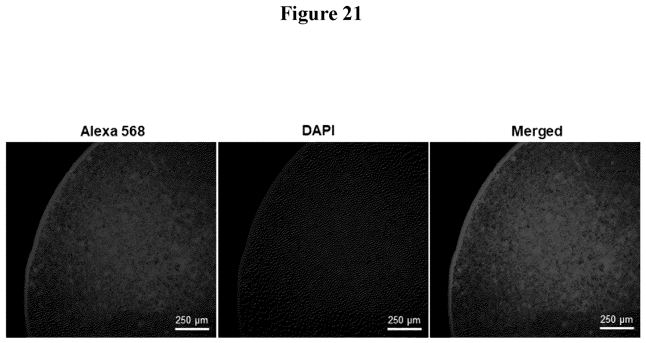

**Figure 21. Confocal microscopy analysis of fluorescence-labelled PEI-3A-FDH/3A-LDH-NADH entrapped agarose beads.** The Alexa 568-labeled PEI-3A-FDH/3A-LDH particles exhibit a uniform distribution throughout the beads. The NADH molecules observed in the DAPI channel overlap with the particle fluorescence showing that they are immobilized within the PEI-3A-FDH/3A-LDH particles.

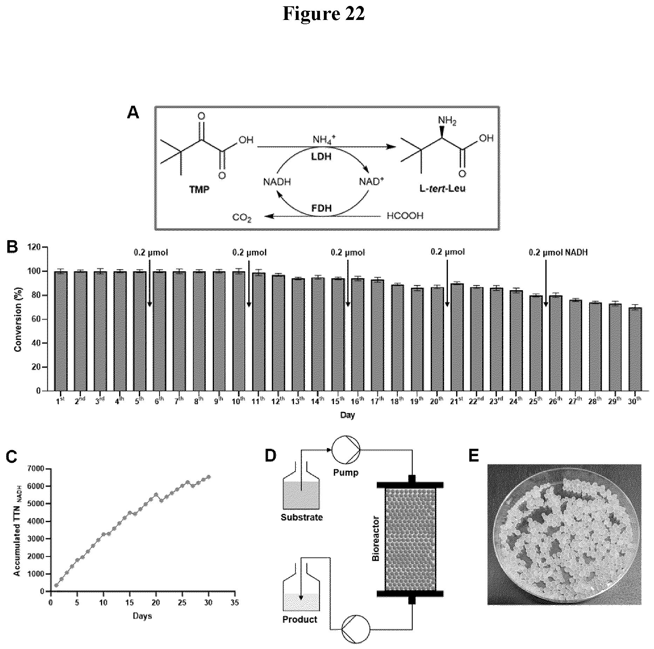

**Figure 22. Biosynthesis of *L-tert* Leucine in a continuous flow reactor. Fig. 22A:** The catalytic conversion of TMP to L-*tert* Leu is achieved using PEI-3A-FDH/3A-LDH particles embedded in agarose beads. The FDH component functions as an NADH regenerator. **Fig. 22B:** Change in conversion rates of PEI-3A-FDH/3A-LDH entrapped agarose beads over time. The beads were initially charged with 2 $\mu$mol of NADH, followed by the addition of 0.2 $\mu$mol of NADH every 5 days. **Fig. 22C:** The gradual accumulation of $TTN_{NADH}$ as a function of time. The $TTN_{NADH}$ values slightly went down on the 6th, 11th, 16th, 21st and 26th days due to the addition of exogenous NADH, but subsequently increased consistently over the next five days. **Fig. 22D:** Illustrative scheme of the continuous flow reaction system. **Fig. 22E:** PEI-3A-FDH/3A-LDH agarose beads used for continuous flow biosynthesis.

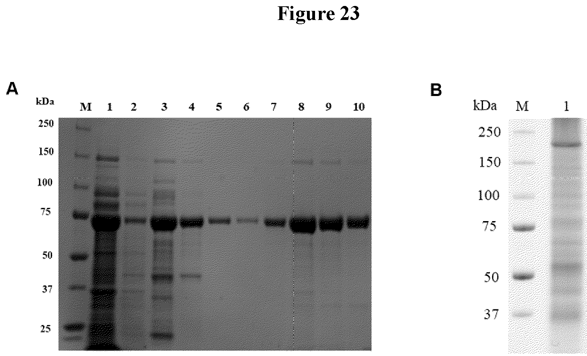

**Figure 23. Production of free CadA and CadA-Cry1Ab. Fig. 23A:** Nickel resin column purification of free CadA. Lane 1, flow though; lanes 2-6, wash with 0-50 mM imidazole; lane 7, elution with 100 mM imidazole; lanes 8-10, elution with 250 mM imidazole. **Fig. 23B:** SDS-PAGE of purified CadA-Cry1Ab particle.

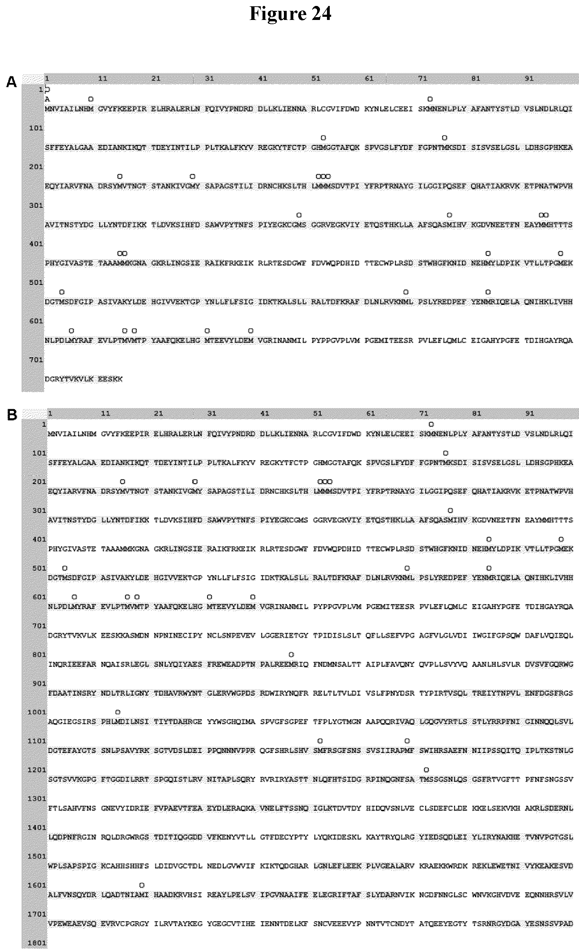

**Figure 24. Orbitrap mass spectrometry for CadA (Fig. 24A) and CadA-Cry1Ab (Fig. 24B).**

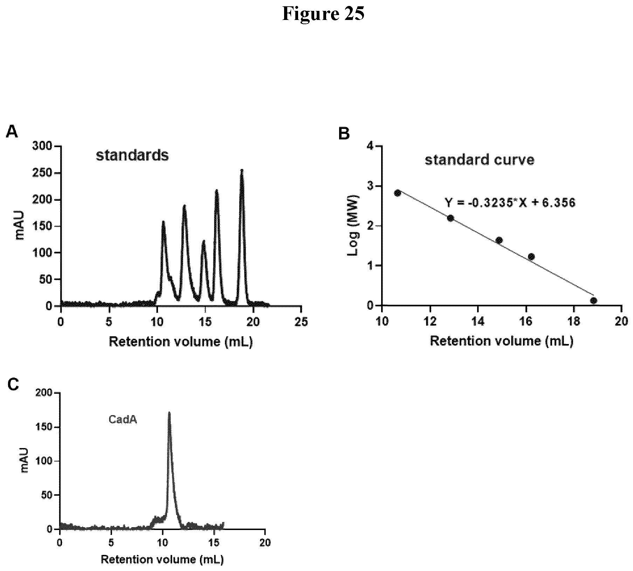

**Figure 25. Size exclusion chromatography of free CadA. Fig. 25A:** SEC for standards with different molecular weights. **Fig. 25B:** Standard curve of molecular weight against retention volume obtained from A. **Fig. 25C:** SEC for CadA.

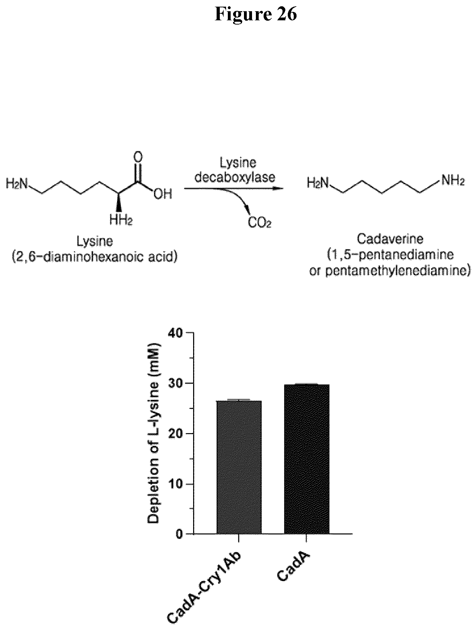

**Figure 26. Conversion of L-lysine by CadA-Cry1Ab.** The conversion of CadA was taken as positive control.

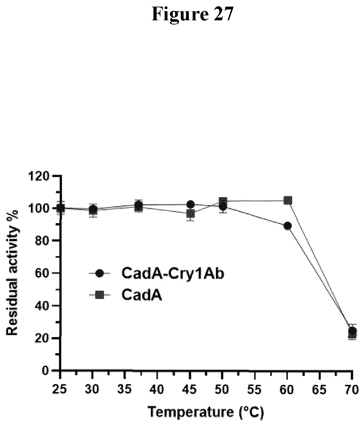

**Figure 27. Thermostability of CadA-Cry1Ab and free CadA.** The residual activity at 25°C was taken as 100%.

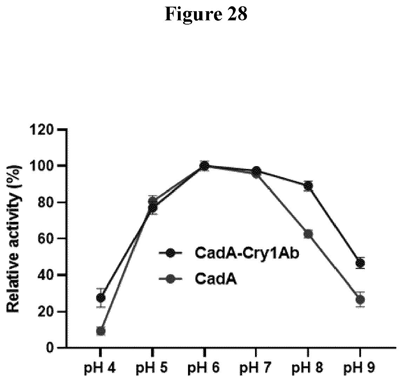

**Figure 28. Optimization of reaction pH for CadA and CadA-Cry1Ab.** The activity for both CadA and CadA-Cry1Ab reached the highest value at pH 6, which was considered as 100%.

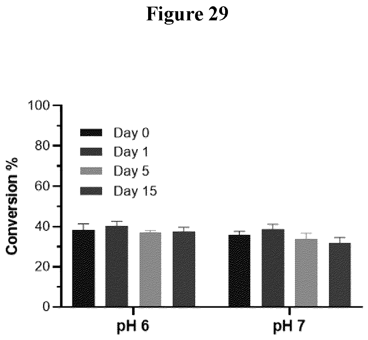

**Figure 29. Longevity of CadA-Cry1Ab particles at pH 6 and pH 7.**

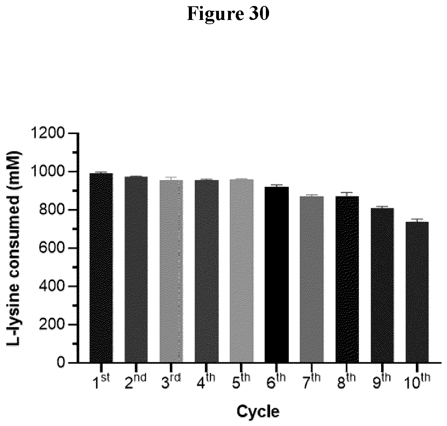

**Figure 30. Recyclability of CadA-Cry1Ab particles with high concentration of L-lysine substrate.** 45 mg of particles was reacted with 1 M L-lysine HCl in 10 mL of reaction buffer (1 M MES, 0.1 mM PLP, pH 6.0) at 37°C for 1 h in each cycle.

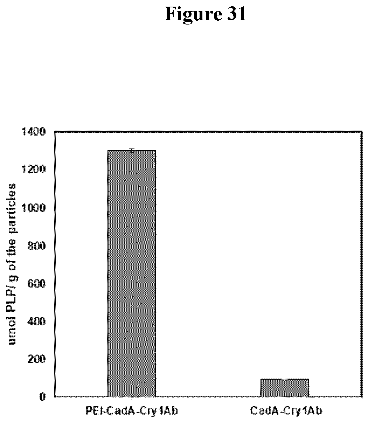

**Figure 31. Binding capacity of CadA-Cry1Ab before and after PEI modification.** The maximum PLP binding capacity of PEI-modified particles was estimated to be approximately 1,300 $\mu$mol/g, whereas the unmodified particles exhibited a binding capacity of about 100 $\mu$mol/g.

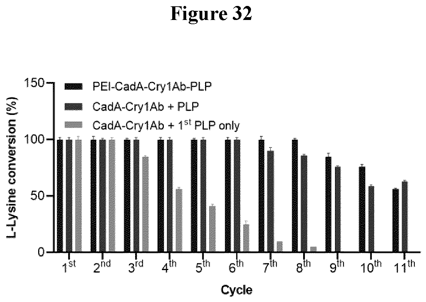

**Figure 32. Recyclability of PEI modified CadA-Cry1Ab particles.** PEI-CadA-Cry1Ab particles efficiently converted L-lysine to cadaverine with the addition of only 0.1 $\mu$mol of PLP prior to the experiment. In contrast, unmodified CadA-Cry1Ab particles required the continuous addition of 0.1 $\mu$mol of PLP before each cycle to achieve 100% conversion and could not function in the absence of PLP.

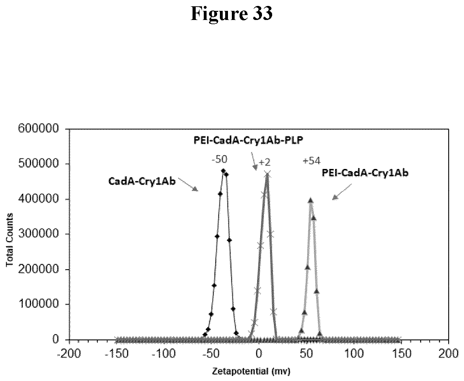

**Figure 33. Zeta potential of CadA-Cry1Ab before and after PEI modification as well as upon PLP binding.** The zeta potential of CadA-Cry1Ab particles shifted significantly from -50 mV to +54 mV after PEI modification. Upon PLP binding to the PEI-CadA-Cry1Ab particles, the zeta potential decreased to +2 mV, indicating electrostatic interactions between PLP and the PEI-modified particles.

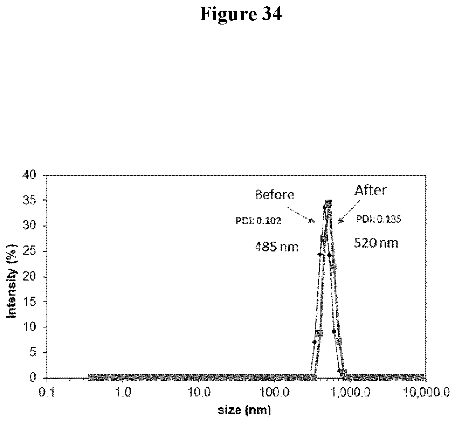

**Figure 34. Hydrodynamic size distribution of CadA-Cry1Ab particles before and after PEI modification.** The particle size increased slightly from 485 nm to 520 nm upon PEI binding, possibly due to the attachment of PEI molecules to the particle surface.

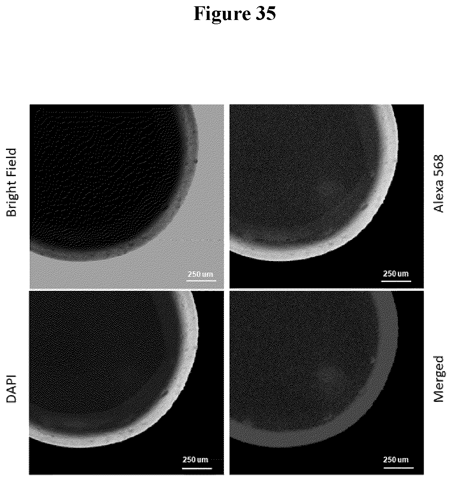

**Figure 35. Confocal microscopy analysis of agarose beads entrapped PEI-CadA-Cry1Ab-PLP.** The particles were labeled with Alexa 568, while PLP molecules exhibited intrinsic fluorescence in the DAPI channel. The imaging revealed uniform distribution of particles within the agarose beads and efficient PLP immobilization.

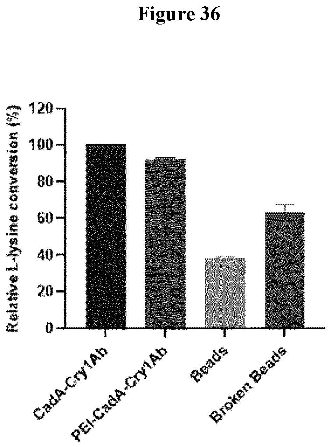

**Figure 36. Conversion comparison between CadA-Cry1Ab, PEI-CadA-Cry1Ab, agarose beads entrapped PEI-CadA-Cry1Ab, and physically fragmented** beads. The conversion of CadA-Cry1Ab was taken as 100%.

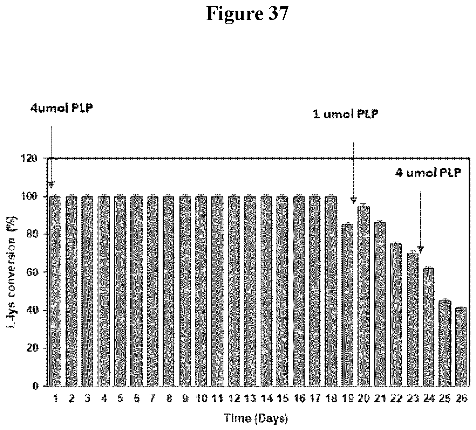

**Figure 37. Continuous-flow biosynthesis of cadaverine using PEI-CadA-Cry1Ab-agarose beads.** Cadaverine was continuously produced over 19 days without the need for exogenous PLP, using only 4 $\mu$mol of PLP at the beginning of the experiment. The flow rate was set to 50 $\mu$L/min, and the reaction buffer consisted of 50 mM L-lysine in 100 mM MES buffer (pH 6.0). The product was collected and measured every 24 hours. A total of 75 mg of particles were used. After 19 cycles, a ~15% reduction in activity was observed. Following the addition of 1 $\mu$mol PLP, enzymatic activity was not effectively restored and gradually declined, suggesting enzyme inactivation after approximately three weeks.

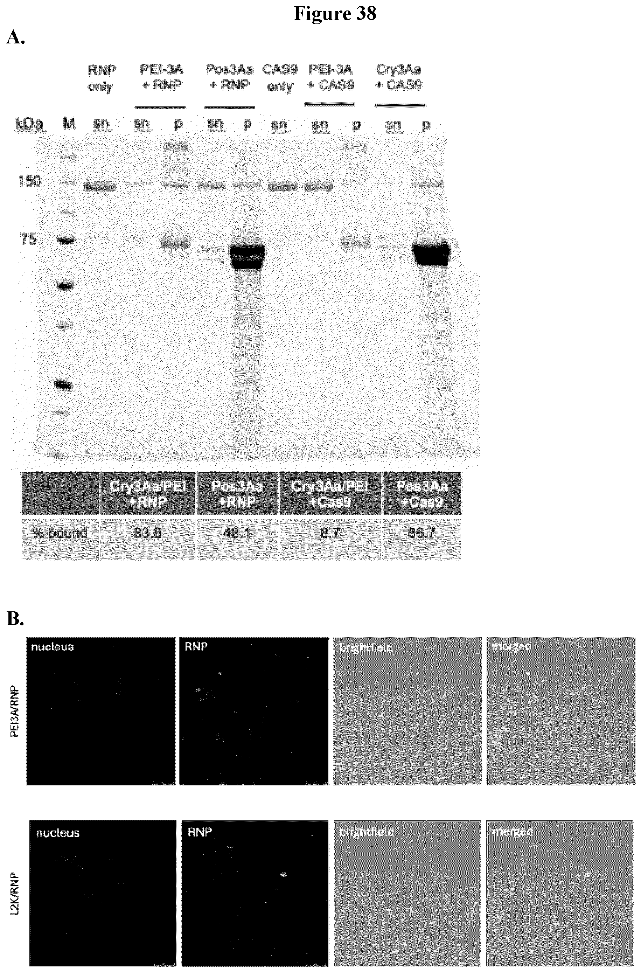

**Fig. 38A. Binding of RNP to PEI-modified Cry3Aa.** RNP was incubated with Cry3Aa or PEI- modified Cry3Aa (PEI-3A) or Pos3Aa crystal in serum free OptiMEM at 4oC. The reaction mixtures were centrifuged to separate the pellets comprising the RNP/crystal complexes (p) from the supernatants (sn). The pellets were washed once with OptiMEM and resuspended in 20$\mu$L OptiMEM for the subsequent SDS-PAGE analysis. Cas9 protein without complexed with gRNA was set up in parallel for comparison and as a control. **Fig. 38B. Intracellular delivery of RNP mediated by PEI3A in MDA-MB-231.** 37.5 nM equivalent of Cas9 in RNP (Cas9:sgRNA at 1:1.1 molar ratio) was used for all experimental groups. MDA- MB-231 cells in serum free DMEM supplemented with 1% P/S and 10 $\mu$g/mL gentamicin was treated with PEI3A/RNP, and RNP mixed in lipofectamine (L2K/RNP) for 4 h. Samples were then fixed with 4% PFA and blocked with 5% FBS, then incubated overnight with anti-Cas9 followed by secondary antibody Alexa-488 anti-MS IgG (H+L). Nucleus was stained using Hoechst 33342. L2K denotes lipofectamine 2000.

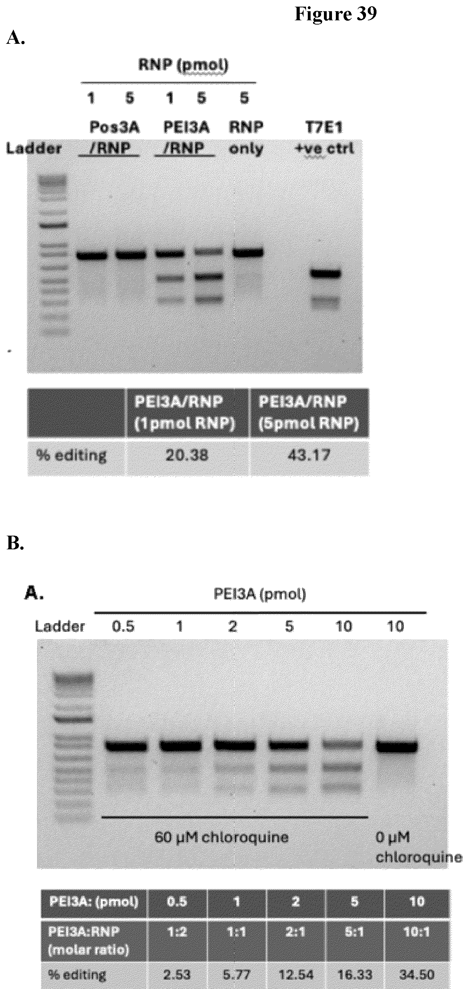

**Fig. 39A. Effect of chloroquine on gene editing efficiency of PEI3A/RNP in HEK293T.** Evaluation of the editing efficiency of PEI3A-mediated delivery of gene editing components in the presence of 60 $\mu$M chloroquine. After a brief 1-h incubation with 60 $\mu$M chloroquine. Culture medium was serially diluted using fresh serum-free DMEM, PEI/RNP was then added to the cells. FBS concentration was adjusted to 10% after 4-h incubation without removing PEI3A/RNP. Medium was further diluted with usual culturing media after 18-h. Editing efficiency was detected with T7E1 DNA mismatch cleavage assay. **Fig. 39B. Optimization of the gene editing efficiency of PEI3A/RNP combined with chloroquine in HEK293T.** Investigation of different molar ratios of PEI3A: RNP on gene editing efficiency in the presence of 60 $\mu$M chloroquine. RNP was fixed at 1 pmol and incubated with different concentrations of PEI3A. The resultant PEI3A/RNP was then added to HEK293T cells pretreated with 60$\mu$M chloroquine for 1 h. Editing efficiency was detected with T7E1 assay.

DEFINITIONS

The term **"Cry protein,"** as used herein, refers to any one protein among a class of crystalline proteins produced by strains of *Bacillus thuringiensis* (*Bt*). Some examples of "Cry proteins" include, but are not limited to, Cry1Aa, Cry1Ab, Cry2Aa, Cry3Aa, Cry4Aa, Cry4Ba, Cry11Aa, Cry11Ba, and Cry19Aa. Their amino acid sequences and polynucleotide coding sequences are known and can be found in publications such as U.S. Patent Application published as US2010/0322977. Their GenBank Accession Nos. are:

Cry1Aa AY197341.1
Cry1Ab AY847289.1
Cry2Aa AF273218.1
Cry3Aa AJ237900.1
Cry4Aa AB513706.1
Cry4Ba AB161456.1
Cry11Aa AL731825.1
Cry11Ba LC153032.1
Cry19Aa Y07603.1

In addition to the wild-type Cry proteins, the term "Cry protein" also encompasses functional variants, which (1) share an amino acid sequence identity of at least 80%, 81%, 82%, 83%, 84%, 88%, 89%, 90%, 91%, 92%, 93%, 94%, 95%, 96%, 97%, 98%, 99%, or 99.5% to the polypeptide sequence of any one of the Cry proteins listed in US2010/0322977; and (2) retain the ability to spontaneously form crystals within host cells as can be confirmed by known methods such as electron micrograph (see description in, *e.g.,* Park et al., Appl Environ Microbiol, 1998, 64, 3932-3938; Schnepf et al., Microbiol Mol Biol Rev, 1998, 62, 775-806; Whiteley and Schnepf, Annu Rev Microbiol, 1986, 40, 549-576; and Nair et al., PLoS One, 2015, 10, e0127669). For example, a "Cry protein" encompasses any variant that confers increased negative charges to the resultant protein by substitutions of at least 3, 4, 5, 6, 7, 8, 9, 10, 11, 12, 13, 14, 15, or more positively charged or neutral amino acids within the domain II of the wild-type Cry protein (*e.g.,* the 295 to 499 segment of SEQ ID NO:4, as well as its corresponding segment in other known Cry proteins, for example, those shown in Figure 27 of WO2020/216322) with negatively charged amino acids such as aspartate (D) and glutamate (E). One example of such mutant is NegCry3Aa, which is a Cry3Aa variant containing the following mutations: K384E, N391D, N395D, S425E, Q430E, TQ436437EE, KR442443EE, T461D, and K467E. See SEQ ID NO:6 in WO2020/216322. Further encompassed by the term "Cry protein" are modified Cry proteins containing amino acid insertions or deletions (for example, insertion of at least 1, 2, 3, 4, 5, 6, 7, 8, 9, or 10 amino acids, or a deletion of at least 1, 2, 3, 4, 5, 6, 7, 8, 9, 10, or more amino acids) at one or more of the 8 specific regions (for example, at least 1, 2, 3, 4, 5, 6, 7, or all 8 regions) shown in Figure 22 of WO2020/216322, as well as their corresponding regions in other Cry proteins as shown in Figure 18 of WO2020/211782, can effectively reduce or enlarge the protein's channel size, respectively, and can therefore make the resultant crystal-forming protein variant to more effectively entrap a target protein (such as an enzyme) with increased retention rate and reduced diffusion rate for the target protein. Typically, such a channel-size altering Cry protein mutant contains at least 3, 4, or 5 but no more than 10, 12, or 15 point mutations (deletions or insertions) in the entire protein. One example of a Cry3 Aa mutant of this nature is termed 3A2-2 (SEQ ID NO:7 in WO2020/216322). In addition, the term "Cry protein" also encompasses Cry protein mutants that have been modified at one or more residues to improve the stability of the resultant protein or its crystal structure, for example, introducing one or more cysteine residues by insertion or substitution to the wild-type Cry protein amino acid sequence so as to form additional disulfide bond or bonds to stabilize the protein or crystal structure. One such example is a double mutant of Cry3Aa (3AS145C, H161R), having the amino acid sequence of SEQ ID NO:9 set forth in WO2020/216322.

Similarly, a **"crystal-forming fragment"** of a Cry protein is a fragment of any of the known Cry proteins (*i.e.,* less than full length of the wild-type Cry protein) that still retains the ability of self-crystallization, which is demonstrated both by crystallization by the fragment alone and by causing a fusion protein to self-crystallize when the fragment is present in the fusion protein with another protein of interest (*e.g.*, an enzyme). In addition to being a truncated form of a Cry protein, a "crystal-forming fragment" may further contain one or more modifications to the native amino acid sequence such as insertions, deletions, or substitutions, especially conservative modifications, such that the resultant "crystal-forming fragment" shares an amino acid sequence identity of at least 80%, 81%, 82%, 83%, 84%, 88%, 89%, 90%, 91%, 92%, 93%, 94%, 95%, 96%, 97%, 98%, 99%, or 99.5% to the polypeptide sequence of the corresponding fragment of a wild-type Cry protein. Exemplary crystal-forming fragments of a Cry protein have been described in earlier disclosures, *e.g.*, WO2018/028371, WO2020/211782, and WO2020/216322. In this disclosure, the term **"protein crystal"** is used to describe insoluble protein aggregates, which may involve one or two or more different proteins, among which at least one comprising a crystal-forming protein or a crystal-forming fragment thereof, *e.g.,* as a part of a fusion protein. This term "protein crystal" does not require the presence of any particular crystal structure in an organized fashion.

The term **"nucleic acid" or "polynucleotide"** refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.*, degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98

(1994)). The term nucleic acid is used interchangeably with gene, cDNA, and mRNA encoded by a gene.

The term **"gene"** means the segment of DNA involved in producing a polypeptide chain. It may include regions preceding and following the coding region (leader and trailer) as well as intervening sequences (introns) between individual coding segments (exons).

The term **"amino acid"** refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.*, hydroxyproline, $\gamma$-carboxyglutamate, and O-phosphoserine. Amino acid analogs refers to compounds that have the same basic chemical structure as a naturally occurring amino acid, *i.e.,* an $\alpha$ carbon that is bound to a hydrogen, a carboxyl group, an amino group, and an R group, *e.g.*, homoserine, norleucine, methionine sulfoxide, methionine methyl sulfonium. Such analogs have modified R groups (*e.g.*, norleucine) or modified peptide backbones, but retain the same basic chemical structure as a naturally occurring amino acid. "Amino acid mimetics" refers to chemical compounds having a structure that is different from the general chemical structure of an amino acid, but that functions in a manner similar to a naturally occurring amino acid.

There are various known methods in the art that permit the incorporation of an unnatural amino acid derivative or analog into a polypeptide chain in a site-specific manner, *see, e.g.,* WO 02/086075.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, "conservatively modified variants" refers to those nucleic acids that encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein that encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another:

1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)

*(see, e.g.,* Creighton, Proteins, W. H. Freeman and Co., N. Y. (1984)).

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

In the present application, amino acid residues are numbered according to their relative positions from the left most residue, which is numbered 1, in an unmodified wild-type polypeptide sequence.

As used in herein, the terms **"identical"** or percent **"identity,"** in the context of describing two or more polynucleotide or amino acid sequences, refer to two or more sequences or subsequences that are the same or have a specified percentage of amino acid residues or nucleotides that are the same (for example, a Cry protein or a crystal-forming fragment of a Cry protein sequence comprised in the fusion protein of this invention has at least 80% identity, preferably 85%, 90%, 91%, 92%, 93, 94%, 95%, 96%, 97%, 98%, 99%, or 100% identity, to a reference sequence, *e.g.*, the amino acid sequence of a corresponding wild-type Cry protein or fragment), when compared and aligned for maximum correspondence over a comparison window, or designated region as measured using one of the following sequence comparison algorithms or by manual alignment and visual inspection. Such sequences are then said to be "substantially identical." With regard to polynucleotide sequences, this definition also refers to the complement of a test sequence. Preferably, the identity exists over a region that is at least about 50 amino acids or nucleotides in length, or more preferably over a region that is 75-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. For sequence comparison of nucleic acids and proteins, the BLAST and BLAST 2.0 algorithms and the default parameters discussed below are used.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, *e.g.,* by the local homology algorithm of Smith & Waterman, Adv. Appl. Math. 2:482 (1981), by the homology alignment algorithm of Needleman & Wunsch, J. Mol. Biol.

48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection, see, *e.g.,* Current Protocols in Molecular Biology (Ausubel et al., eds. 1995 supplement).

Examples of algorithms that are suitable for determining percent sequence identity and sequence similarity are the BLAST and BLAST 2.0 algorithms, which are described in Altschul et al., (1990) J. Mol. Biol. 215: 403-410 and Altschul et al. (1977) Nucleic Acids Res. 25: 3389-3402, respectively. Software for performing BLAST analyses is publicly available at the National Center for Biotechnology Information website, ncbi.nlm.nih.gov. The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.*, *supra*). These initial neighborhood word hits acts as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands. For amino acid sequences, the BLASTP program uses as defaults a word size (W) of 3, an expectation (E) of 10, and the BLOSUM62 scoring matrix (see Henikoff & Henikoff, Proc. Natl. Acad. Sci. USA 89:10915 (1989)).

The BLAST algorithm also performs a statistical analysis of the similarity between two sequences *(see, e.g.,* Karlin & Altschul, Proc. Nat'l. Acad. Sci. USA 90:5873-5787

(1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

**"Polypeptide," "peptide,"** and **"protein"** are used interchangeably herein to refer to a polymer of amino acid residues. All three terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins, wherein the amino acid residues are linked by covalent peptide bonds.

The term **"recombinant"** when used with reference, *e.g.,* to a cell, or a nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

An **"expression cassette"** is a nucleic acid construct, generated recombinantly or synthetically, with a series of specified nucleic acid elements that permit transcription of a particular polynucleotide sequence in a host cell. An expression cassette may be part of a plasmid, viral vector derived from a viral genome, or nucleic acid fragment/construct. Typically, an expression cassette includes a polynucleotide to be transcribed, operably linked to a promoter. Other elements that may be present in an expression cassette include those that enhance transcription (*e.g.,* enhancers) and terminate transcription (*e.g.,* terminators), as well as those that confer certain binding affinity or antigenicity to the recombinant protein produced from the expression cassette.

A **"promoter"** is defined as an array of nucleic acid control sequences that direct transcription of a polynucleotide sequence. As used herein, a promoter includes necessary polynucleotide sequences near the start site of transcription, such as, in the case of a polymerase II type promoter, a TATA element. A promoter also optionally includes distal enhancer or repressor elements, which can be located as much as several thousand base pairs from the start site of transcription. A "constitutive" promoter is a promoter that is active under most environmental and developmental conditions. An "inducible" promoter is a promoter that is active under environmental or developmental regulation. The term "operably linked" refers to a functional linkage between a polynucleotide expression control sequence (such as a promoter, or array of transcription factor binding sites) and a second polynucleotide sequence, wherein the expression control sequence directs transcription of the polynucleotide sequence corresponding to the second sequence.

The term **"heterologous"** as used in the context of describing the relative location of two elements, refers to the two elements such as polynucleotide sequences (e.g., a promoter or a protein/polypeptide-encoding sequence) or polypeptide sequences (e.g., a Cry protein sequence or another polypeptide sequence) that are not naturally found in the same relative positions. Thus, a "heterologous promoter" of a gene refers to a promoter that is not naturally operably linked to that gene. Similarly, a "heterologous protein," "heterologous polypeptide" or "heterologous polynucleotide" to a Cry protein or its encoding sequence or a fragment thereof is one derived from an origin other than the Cry protein or, in the case of a fragment of a Cry protein/coding sequence, may be derived from another part of the same Cry protein or coding sequence, but not naturally immediately connected to the fragment in the same fashion. The fusion of a fragment of a Cry protein (or its coding sequence) with a heterologous polypeptide (or polynucleotide sequence) does not result in a longer polypeptide or polynucleotide sequence that can be found naturally in the wild-type Cry protein.

By **"host cell"** is meant a cell that contains an expression vector and supports the replication or expression of one or more coding sequences harbored in the expression vector. Host cells may be prokaryotic cells such as *Bacillus thuringiensis* (*Bt*), *Bacillus subtilis* (*Bs*), or *E. coli,* or eukaryotic cells such as yeast, insect, amphibian, or mammalian cells such as CHO, HeLa and the like, *e.g.,* cultured cells, explants, and cells *in vivo.*

In this application, the term **"polyethylenimine-modified protein"** or **"PEI-modified protein"** refers to a protein that has been reacted with PEI in adequate quantity and under conditions that permit formation of a chemical bond between any one free carboxyl group (-COOH) on the protein with any one free amine (-NH$_2$) group on the PEI.

As used herein, a **"cofactor"** or "coenzyme" is a non-protein organic or inorganic compound that is required for an enzyme's proper function as a catalyst for a chemical reaction. An organic cofactor comprising at least one phosphate group (-PO$_4$) is also referred to as a "phosphorylated cofactor."

The term **"about"** as used herein denotes a range of +/- 10% of a reference value. For examples, "about 10" defines a range of 9 to 11.

DETAILED DESCRIPTION OF THE INVENTION

## I. INTRODUCTION

There has been growing interest in using enzymes to catalyze industrial reactions due to their high reactivity, excellent regio- and enantiospecificity, and low environmental toxicity. In order to financially compete with chemical catalysis, biocatalysts are optimized so they can be recycled multiple times. Additionally, biocatalysts are generally optimized so they can withstand high concentrations of organic solvents - conditions that can promote substrate solubility and enzyme activity. Enzyme immobilization can facilitate easy removal and subsequent reuse of enzymes during multiple rounds of catalysis, in addition to improving enzyme stability resistant to harsh industrial conditions. To this end, a first approach for immobilization of enzymes by way of recombinantly engineering and producing fusion proteins of an enzyme fused with a crystal-forming protein or a crystal-forming fragment of the protein has been successful in generating protein crystals with retained enzymatic activity, see, *e.g.*, WO2018/028371 and WO2020/211782. A second approach, immobilization of enzymes by entrapment of such enzymes, including entrapment of multiple enzymes at the same time in the same protein crystals, is particularly attractive since it does not involve any modifications to the enzyme structure, increasing the chance for high activity retention and native enzyme conformation, see, *e.g.*, WO2020/216322.

This disclosure provides a further improvement in this general methodology of enzyme immobilization and real-life applications. The present inventors have discovered that, upon modification with polyethylenimide (PEI), Cry proteins can tightly bind and therefore effectively immobilize cofactor NADH. This discovery supports a novel scheme of performing a chemical reaction where the enzyme or enzymes catalyzing the reaction and the requisite cofactor are both immobilized by the same protein crystals, the enzyme(s) by way of being a fusion partner with a Cry protein and the cofactor being adsorbed to the PEI-modified Cry protein. This important improvement allows for further minimizing time, processes, and materials for conducting a variety of chemical reactions, enabling greener and more cost-effective products.

## II. CONSTRUCTION OF RECOMBINANT PROTEINS

A. General Recombinant Technology

Basic texts disclosing general methods and techniques in the field of recombinant genetics include Sambrook and Russell, Molecular Cloning, A Laboratory Manual (3rd ed. 2001); Kriegler, Gene Transfer and Expression: A Laboratory Manual (1990); and Ausubel et al., eds., Current Protocols in Molecular Biology (1994).

For nucleic acids, sizes are given in either kilobases (kb) or base pairs (bp). These are estimates derived from agarose or acrylamide gel electrophoresis, from sequenced nucleic acids, or from published DNA sequences. For proteins, sizes are given in kilodaltons (kDa) or amino acid residue numbers. Proteins sizes are estimated from gel electrophoresis, from sequenced proteins, from derived amino acid sequences, or from published protein sequences.

Oligonucleotides that are not commercially available can be chemically synthesized, *e.g.*, according to the solid phase phosphoramidite triester method first described by Beaucage & Caruthers, Tetrahedron Lett. 22: 1859-1862 (1981), using an automated synthesizer, as described in Van Devanter et. al., Nucleic Acids Res. 12: 6159-6168 (1984). Purification of oligonucleotides is performed using any art-recognized strategy, *e.g.*, native acrylamide gel electrophoresis or anion-exchange HPLC as described in Pearson & Reanier, J. Chrom. 255: 137-149 (1983).

The sequence of a gene of interest, such as the polynucleotide sequence encoding an enzyme such as an alcohol dehydrogenase (ADH), formate dehydrogenase (FDH), leucine dehydrogenase (LDH), human arginase (hArg), or ornithine decarboxylase (ODC), a polynucleotide encoding a Cry protein or a crystal-forming fragment thereof, and synthetic oligonucleotides can be verified after cloning or subcloning using, *e.g.,* the chain termination method for sequencing double-stranded templates of Wallace et al., Gene 16: 21-26 (1981).

B. Coding Sequences

Polynucleotide sequences encoding Cry proteins, fragments, or fusion proteins for use in this invention can be readily constructed by using the corresponding coding sequences for the Cry proteins, fragments, or combining the coding sequences for the fusion partners, such as a Cry3Aa or Cry1Ab protein and hArg or ODC. The sequences for Cry proteins and enzymes are generally known and may be obtained from a commercial supplier.

In addition to the use of full-length wild-type Cry proteins for producing crystal-forming proteins for use in this invention, fragments of Cry proteins and/or variants of Cry proteins may also be useful. A DNA sequence encoding a Cry protein can be modified to generate fragments or variants of the Cry protein. So long as these fragments and variants retain the ability to spontaneously form crystals when expressed in a host cell, especially a *Bacillus* bacterial cell, they can be used for producing the protein crystals, either by themselves or by way of fusion proteins capable of undergoing spontaneous crystallization, and therefore producing protein crystals containing one or more recombinant proteins (*e.g.*, one or more enzymes) embedded within. Typically, the variants bear a high percentage of sequence identity (*e.g.,* at least 80, 85, 90, 95, 97, 98, 99% or higher) to the wild-type Cry protein sequence, whereas the fragments may be substantially shorter than the full length Cry protein, such as having some amino acids (*e.g.,* 10-300 or 20-200 or 50-100 amino acids) removed from the N- or C-terminus of the full length Cry protein. For example, a useful Cry3Aa fragment may be as short as the first 290 amino acids from the N-terminus, encompassing Domain I of the protein. Other examples of such fragments include a Cry protein fragment having its first 57 amino acids from N-terminus removed and a Cry protein fragment having its C-terminal 18 amino acids removed. The ability of a recombinantly produced Cry protein, a fragment thereof, or a fusion protein comprising a Cry protein or fragment to undergo spontaneous crystallization can be verified by electron micrograph, whereas the enzymatic activity of a recombinantly produced enzyme, including in the form of a fusion protein with a Cry protein or a fragment thereof, can be confirmed by established assays for each specific enzyme. Exemplary Cry protein fragments capable of self-crystalizing can be found in the inventors' earlier publications, *e.g.*, WO2018/028371.

In the case of a fusion protein, one or more peptide linkers or spacers may be used to connect the coding sequences for a Cry protein/fragment and one or more heterologous polypeptides. One purpose is to ensure the proper reading frame for the fusion protein such that the coding sequences for both Cry protein/fragment and the heterologous polypeptide(s) are in frame. Another purpose is to provide appropriate spatial relationship between the Cry protein/fragment and the heterologous polypeptide(s), such that each component of the fusion protein may retain its original functionality: the Cry protein/fragment is able to cause self-crystallization of the fusion protein, and the heterologous polypeptide such as an enzyme remains active in its catalytic capacity. Also, one or more linkers may be placed at the very beginning and/or the very end of the open reading frame, so as to facilitate proper start and termination of the coding sequence translation. Such linkage amino acid sequences are usually shorts and typically no longer than 100 or 50 amino acids, such as between 1 to 100, 1 or 2 to 50, 2 or 3 to 25, 3 or 4 to 10 amino acids.

C. Sequence Modification for Preferred Codon Usage in a Host Organism

The polynucleotide sequence encoding a recombinant protein to be expressed according to the method of this invention can be further altered to coincide with the preferred codon usage of a particular host. For example, the preferred codon usage of one strain of bacterial cells can be used to derive a polynucleotide that encodes a recombinant polypeptide of the invention and includes the codons favored by this strain. The frequency of preferred codon usage exhibited by a host cell can be calculated by averaging frequency of preferred codon usage in a large number of genes expressed by the host cell (*e.g.,* calculation service is available from web site of the Kazusa DNA Research Institute, Japan). This analysis is preferably limited to genes that are highly expressed by the host cell.

At the completion of modification, the coding sequences are verified by sequencing and are then subcloned into an appropriate expression vector for recombinant production of a protein (*e.g.,* an enzyme such as a lipase) along with a Cry protein, a crystal-forming fragment of the Cry protein, or a fusion protein comprising the Cry protein or crystal-forming fragment, such that the protein (*e.g.,* enzyme) is produced and embedded in the protein crystals formed by the Cry protein, fragment, or fusion protein.

## III. EXPRESSION AND ISOLATION OF RECOMBINANT PROTEINS

Following verification of the coding sequence, co-expression of a protein of interest (such as an enzyme) and a Cry protein, a crystal-forming fragment thereof, or a fusion protein comprising the Cry protein or fragment of this invention can be produced using routine techniques in the field of recombinant genetics, relying on the polynucleotide sequences encoding the Cry fusion protein disclosed herein.

### A. Expression Systems

To obtain high level expression of a polynucleotide sequence encoding a recombinant protein, one typically subclones a polynucleotide encoding the protein in the correct reading frame into an expression vector that contains a strong promoter to direct transcription, a transcription/translation terminator and a ribosome binding site for translational initiation. Suitable bacterial promoters are well known in the art and described, *e.g.,* in Sambrook and Russell, *supra,* and Ausubel *et al.*, *supra.* Bacterial expression systems for expressing the polypeptide are available in, *e.g., E. coli, Bacillus sp., Salmonella,* and *Caulobacter.* Kits for such expression systems are commercially available.

The promoter used to direct expression of a recombinant protein depends on the particular host cells used for the recombinant protein production. For instance, for effective expression in a *Bacillus* bacterial strain such as *Bacillus thuringiensis* (*Bt*) or *Bacillus subtilis* (*Bs*) cells, a promoter known to direct robust protein expression in these particular bacterial cells should be chosen. The promoter is optionally positioned about the same distance from an exogenous or recombinant protein transcription start site as it is from the transcription start site in its natural setting. As is known in the art, however, some variation in this distance can be accommodated without loss of promoter function. In some cases, a constitutive promoter is used, whereas in other cases an inducible promoter rather than a constitutive promoter is preferred. Further, the placement of a common promoter or multiple separate promoters (which may be the same or different kind of promoters) and/or the placement of one or more separate ribosome binding sites separating different segments of coding sequences (each encoding a recombinant protein or a crystal-forming protein) in a common expression cassette (*e.g.*, an expression vector such as a plasmid) can allow different expression ratios of the recombinant protein(s) to the crystal-forming protein so as to maximize the percentage of the recombinant protein(s) being entrapped or immobilized within the protein crystals formed by the crystal-forming protein such as a Cry protein, a crystal-forming fragment thereof, or a fusion protein comprising the Cry protein or the fragment.

In addition to the promoter, the expression vector typically includes a transcription unit or expression cassette containing all the additional elements that are required for the expression of the recombinant protein(s) and the crystal-forming protein in host cells. A typical expression cassette thus contains a promoter operably linked to the polynucleotide sequence encoding the recombinant protein and/or crystal-forming protein and signals required for efficient polyadenylation of the transcript, ribosome binding sites, and translation termination. The polynucleotide coding sequence may be linked to a cleavable signal peptide sequence to promote secretion of the polypeptide by the transformed cell. Such signal peptides include, among others, the signal peptides from tissue plasminogen activator, insulin, and neuron growth factor, and juvenile hormone esterase of *Heliothis virescens.* Additional elements of the cassette may include enhancers and, if genomic DNA is used as the structural gene, introns with functional splice donor and acceptor sites.

In addition to a promoter sequence, the expression cassette should also contain a transcription termination region downstream of the coding sequence to provide for efficient termination. The termination region may be obtained from the same gene as the promoter sequence or may be obtained from different genes. The placement of a commonly shared termination site in combination with the placement of multiple promoters directing transcription of multiple coding sequences can also be used to adjust the expression ratios of a recombinant protein to a crystal-forming protein.

The particular expression vector used to transport the genetic information into the cell is not particularly critical. Any of the conventional vectors used for expression in eukaryotic or prokaryotic cells may be used, especially those suitable for expression in cells of *Bacillus sp.* such as *Bt* and *Bs.* Standard bacterial expression vectors include plasmids such as pBR322 based plasmids, pSKF, pET23D, and fusion expression systems such as GST and LacZ.

The elements that are typically included in expression vectors also include a replicon that functions in bacteria such as *Bacillus sp.* and *E. coli,* a gene encoding antibiotic resistance to permit selection of bacteria that harbor recombinant plasmids, and unique restriction sites in nonessential regions of the plasmid to allow insertion of coding sequences. The particular antibiotic resistance gene chosen is not critical, any of the many resistance genes known in the art are suitable. Similar to antibiotic resistance selection markers, metabolic selection markers based on known metabolic pathways may also be used as a means for selecting transformed host cells.

B. Transfection Methods

Standard transfection methods are used to produce bacterial, mammalian, yeast, insect, or plant cell lines that express large quantities of a recombinant fusion protein of this invention, which are then purified using standard techniques (*see, e.g.,* Colley et al., J. Biol. Chem. 264: 17619-17622 (1989); Guide to Protein Purification, in Methods in Enzymology, vol. 182 (Deutscher, ed., 1990)). Transformation of eukaryotic and prokaryotic cells are performed according to standard techniques (*see, e.g.,* Morrison, J. Bact. 132: 349-351 (1977); Clark-Curtiss & Curtiss, Methods in Enzymology 101: 347-362 (Wu et al., eds, 1983).

Any of the well-known procedures for introducing foreign polynucleotide sequences into host cells may be used. These include the use of calcium phosphate transfection, polybrene, protoplast fusion, electroporation, liposomes, microinjection, plasma vectors, viral vectors and any of the other well-known methods for introducing cloned genomic DNA, cDNA, synthetic DNA, or other foreign genetic material into a host cell (*see, e.g.,* Sambrook and Russell, *supra*). It is only necessary that the particular genetic engineering procedure used be capable of successfully introducing at least one gene into the host cell capable of expressing the recombinant protein(s) along with a crystal-forming protein (*e.g.,* a Cry protein, a crystal-forming fragment thereof, or a fusion protein comprising a Cry protein or a crystal-forming fragment thereof) according to the method of this invention. As described above and herein, the recombinant protein or proteins (*e.g.,* enzyme or enzymes) and the crystal-forming protein may be contained within one single expression cassette, *e.g.,* in the same expression vector, where all coding sequences may be under the control of one single promoter, or each coding sequence may be under the control of a separate promoter (which optionally may differ from one another). In the alternative, each one of the coding sequences may be present in a separate expression cassette (*e.g.,* expression vector). In either alternative, different ratios of the recombinant protein(s) to the crystal-forming protein may be achieved by using single copy or multiple copies of any one coding sequence or by placing separate or commonly shared ribosome binding site(s) and/or termination site(s) in the expression cassette.

C. Isolation of Crystal-Entrapped Recombinant Proteins

Once the expression of the recombinant protein(s) along with a crystal-forming protein (*e.g.,* a Cry protein, a crystal-forming fragment thereof, or a fusion protein comprising a Cry protein or a crystal-forming fragment thereof) in transfected host cells is confirmed, *e.g.,* via electron micrograph for detecting protein crystals or an immunoassay such as Western blotting analysis, the host cells are then cultured in an appropriate scale for the purpose of purifying or isolating the recombinant protein entrapped within the protein crystals formed by the Cry protein, a crystal-forming fragment thereof, or a fusion protein comprising a Cry protein or a crystal-forming fragment thereof.

When the recombinant protein(s) and the crystal-forming protein are produced recombinantly by transformed bacteria in large amounts, for example after promoter induction, the recombinant protein(s) become entrapped within the crystals formed by the crystal-forming protein. In other words, the recombinantly produced proteins are present in crystalline form or insoluble aggregates within the host cells. Thus, one can readily isolate the crystals from the cell lysate based on their distinct density by utilizing techniques such as centrifugation and density gradient separation followed by one or more rinsing steps to further remove contaminants from the protein crystals.

There are several protocols that are suitable for purification of protein inclusion bodies. For example, purification of aggregate proteins (hereinafter referred to as inclusion bodies) typically involves the extraction, separation and/or purification of inclusion bodies by disruption of bacterial cells, *e.g.,* by incubation in a buffer of about 100-150 $\mu$g/ml lysozyme and 0.1% Nonidet P40, a non-ionic detergent. The cell suspension can be ground using a Polytron grinder (Brinkman Instruments, Westbury, NY). Alternatively, the cells can be sonicated on ice. Additional methods of lysing bacteria are described in Ausubel *et al.* and Sambrook and Russell, both *supra,* and will be apparent to those of skill in the art.

The cell suspension is generally centrifuged and the pellet containing the inclusion bodies resuspended in buffer which does not dissolve but washes the inclusion bodies, *e.g.,* 20 mM Tris-HCl (pH 7.2), l mM EDTA, 150 mM NaCl and 2% Triton-X 100, a non-ionic detergent. It may be necessary to repeat the wash step to remove as much cellular debris as possible. The remaining pellet of inclusion bodies may be resuspended in an appropriate buffer (*e.g.,* 20 mM sodium phosphate, pH 6.8, 150 mM NaCl). Other appropriate buffers will be apparent to those of skill in the art.

Upon isolation, the recombinant protein(s) recovered from host cells in the form of protein crystals, the protein or proteins may be directly used according to their inherent biological activity: for example, a lipase entrapped in Cry protein crystals may be used in a reaction to hydrolyze triglycerides. By virtue of being in an insoluble crystal form, the lipase has a heighted level of resistance to harsh environmental conditions such as high temperature, extreme pHs, organic solvents, *etc.,* thus allowing repeated cycles of cleaning and reuse.

In the alternative, following the washing step, the inclusion bodies are solubilized to release the entrapped recombinant protein(s) by the addition of a solvent that is both a strong hydrogen acceptor and a strong hydrogen donor (or a combination of solvents each having one of these properties). The protein(s) from the inclusion bodies may then be renatured by dilution or dialysis with a compatible buffer. Suitable solvents include, but are not limited to, urea (from about 4 M to about 8 M), formamide (at least about 80%, volume/volume basis), and guanidine hydrochloride (from about 4 M to about 8 M). Some solvents that are capable of solubilizing aggregate-forming proteins, such as SDS (sodium dodecyl sulfate) and 70% formic acid, may be inappropriate for use in this procedure due to the possibility of irreversible denaturation of the proteins, accompanied by a lack of immunogenicity and/or activity. Although guanidine hydrochloride and similar agents are denaturants, this denaturation is not irreversible and renaturation may occur upon removal (by dialysis, for example) or dilution of the denaturant, allowing re-formation of the immunologically and/or biologically active protein of interest. After solubilization, the protein(s) can be separated from other bacterial proteins by standard separation techniques. For further description of purifying recombinant polypeptides from bacterial inclusion body, *see, e.g.,* Patra et al., Protein Expression and Purification 18: 182-190 (2000).

While the protein crystals tend to remain insoluble at lower or neutral pHs, placing them in alkaline solutions with pH at or greater than 10 or 11 can often effectively dissolve the protein. Once dissolved, the protein can then be analyzed by gel separation (e.g., on an SDS gel) and immunoassays to confirm its identity based on the appropriate molecular weight and immunoreactivity.

### D. Polyethylenimine (PEI) modification of Crystal-forming Recombinant Proteins

Once the recombinant expression of a crystal-forming protein (*e.g.,* a Cry protein, a crystal-forming fragment thereof, or a fusion protein comprising a Cry protein or a crystal-forming fragment thereof) in transfected host cells is confirmed, *e.g.,* via electron micrograph for detecting protein crystals or an immunoassay such as Western blotting analysis, the host cells are then cultured in an appropriate scale for the purpose of producing and then purifying or isolating the protein crystals, before they are modified with polyethyleneimine (PEI).

PEI is a cationic polymer containing primary, secondary, and, if branched, tertiary amino groups with strong positive charges (Hernandez-Montelongo et al., Mater. Sci. Eng. C. 2017, 71, 718-724). PEI typically has a molecular weight range of 700 Da to 1000 kDa, depending on the degree of polymerization (Vicennati et al., A. Curr. Med. Chem. 2008, 15, 2826-2839). PEI can be covalently attached to proteins through reactions with the amino acid side chains using standard conjugation methods. Since PEI has a high cationic-charge density, limited coupling with PEI provides sufficient cationic charge to proteins without causing serious decline in protein functions or activities.

PEI conjugation to the protein crystals of this invention may be performed using methods known in the field or described herein. Briefly, protein crystals are recombinantly produced in a culture of transfected cells. Upon harvesting and lysing the host cells, the protein crystals are isolated via centrifugation of the cell lysate and a proper washing step. The protein crystals are then re-suspended in a reaction solution (*e.g.*, about 0.1-10 mg/ml of crystals) containing an appropriate amount of PEI (*e.g.,* 10-100 μg/mL, pH 6.5-7.5) and at least one coupling reagent that is capable of conjugating PEI to the protein crystals, *e.g.*, EDC/NHS coupling reagents at a molar ratio in the range of about 1:0.5-5. A further option is to use coupling reagent DCC in an organic solvent. Following incubation (optionally with stirring) for an adequate time period (*e.g.*, about 1 to 10 hours), the coupling reaction is terminated, and the PEI-coupled protein crystals are recovered from the solution (e.g., by centrifugation) and again washed (to remove any unbound PEI and other molecules) before the PEI-protein crystals are used in accordance with the present invention.

## IV. Immobilization of Cofactors

In another aspect, the present invention relates to the use of a cofactor or coenzyme, which is necessary for the enzymatic activity of an enzyme and has been co-immobilized with the enzyme contained within protein crystals, in the context of performing a biosynthetic reaction catalyzed by the enzyme. As described above, a recombinant protein, especially an enzyme, may be produced according to the method of this invention so that it is immobilized within protein crystals, either by way of entrapment when coexpressed with a crystal-forming protein or by way of participating in the protein crystal formation as a fusion partner with another polypeptide to yield the crystal-forming fusion protein. It is on this basis, the present inventors further discovered that PEI-modified protein crystals, with highly ordered natural scaffold of PEI, allow for the reversible binding of phosphorylated cofactors (such as NADH) that are essential for the catalytic activity of certain enzymes by applying or ceasing mechanical stirring of a solution containing PEI-modified protein crystals loaded with the cofactor. This discovery supports a reaction scheme where an enzyme and its cofactor are co-immobilized by protein crystals and therefore can be used in a recyclable and washable manner in multiple rounds of reactions catalyzed by the enzyme.

More specifically, the inventors discovered that PEI-modified protein crystals can bind cofactors like NADH at a high capacity, *e.g.*, about $550 \pm 5$ μmol/g, and retain over 50% of the bound NADH molecules even after 20 washing cycles.

These protein crystals thus serve as an ideal reservoir for storing cofactors (especially phosphorylated cofactors such NADH) in stirred batch reactions - the cofactors are released from the PEI-modified crystals under mechanical stirring, allowing the cofactors to participate in the cofactor-dependent reaction processes, whereas when the stirring stops, the cofactors are re-absorbed back onto the DEI-modified protein crystals. This reversible nature of the PEI-modified protein crystals in their releasing and reabsorption of cofactors, controlled by application or cessation of mechanical stirring, contributes to an important feature of the reaction scheme of this invention, where the step of cleaning/washing immobilized enzyme(s) and cofactor(s) after each round of reaction(s) can be expediently performed before the next round of reaction(s).

## V. Applications of Immobilized Proteins

A further aspect of the present invention relates to the use of a recombinant protein, such as an enzyme, entrapped and/or immobilized in protein crystals, especially PEI-modified protein crystals, produced according to the methods described herein to exert the protein's inherent biological activity, for example, to perform reactions typically catalyzed by the enzyme present within the protein crystals, such as hydrolysis, esterification, ligation, proteolysis, biosynthesis, and the like, when the protein crystals containing the enzyme are incubated in a reaction solution with at least one substrate of the enzyme, each in an effective amount, under conditions permissible for the reaction to take place to yield a desired product. In some serial reaction schemes, multiple immobilized enzymes may be placed in one single reaction mix with an initial substrate to be catalyzed by a first enzyme in a first reaction to yield a first product, which in turn serves as the substrate for a second enzyme to be catalyzed in a second reaction to yield a second product, and so on.

The presence of PEI, highly positively charged moieties, on the protein crystals, allows for the immobilization of one or more cofactors or coenzymes essential for the functions of the immobilized enzymes and therefore further secures the enzymatic activity in catalyzing the chemical reactions. As organic solvents are often able to facilitate such reactions and the immobilized recombinant protein produced by the method of this invention is highly tolerant to the presence of organic solvents, a reaction performed using the immobilized protein according to this invention often not only a water-based solvent but also one or more organic solvents, *e.g.*, ethanol, methanol, acetonitrile, and dimethylformamide.

The inventors discovered that immobilization of recombinant protein(s) within the crystalline Cry protein or fusion proteins leads the protein(s) to have a higher level of resistance to organic solvents and a higher level of thermostability, potentially can retain enzymatic activity for use in more cycles of reactions. The presence of PEI on the protein crystals provides a further option of immobilizing cofactor(s) or coenzyme(s) necessary for the reaction, which can be loaded, unloaded, or reloaded onto the PEI-modified protein crystals with relative ease. For example, incubating a phosphorylated cofactor (such as NADH) with PEI-modified protein crystals will result in the cofactor being bound or loaded onto the crystals and thus immobilized; whereas when the cofactor-loaded PEI-modified protein crystals are present in a fluid environment with mechanical stirring, the cofactor will be released or unloaded from the crystals, thus becoming available to facilitate an enzymatic reaction. Once the stirring ceases, the cofactor will again become bound or reloaded to the PEI-modified protein crystals.

In some cases, this reaction process includes a cleaning step, performed after the completion of one round of the reaction and removal of the reaction product(s) as well as any remaining substrate, during which the protein crystals containing recombinant protein(s) are rinsed or washed in preparation of being used again with fresh substrate in a subsequent round of reaction. Similarly, a cleaning step, comprising reloading cofactor(s) or coenzyme(s) onto the PEI moieties of the PEI-protein crystals upon completion of one round enzymatic reaction and then rinsing the crystals, can be performed in between any two rounds of the reaction in order to reuse, recycle, or replenish the cofactor(s) or coenzyme(s) for a consecutive reaction series.

The invention is summarized as follows:

1. A protein crystal formed by a Cry protein, a crystal-forming variant thereof, a crystal-forming fragment thereof, or a fusion polypeptide comprising the Cry protein, the variant, or the fragment fused to a heterologous protein, wherein the protein crystal is modified with polyethylenimine (PEI).

2. The protein crystal of item 1, wherein the Cry protein is Cry3Aa or Cry1Ab.

3. The protein crystal of item 1, which is formed by a fusion polypeptide comprising a Cry protein, a crystal-forming variant of the Cry protein, or a crystal-forming fragment of the Cry protein fused to a heterologous protein.

4. The protein crystal of item 1, which is formed by a Cry protein, a crystal-forming variant of the Cry protein, or a crystal-forming fragment of the Cry protein and comprises a heterologous protein entrapped within.

5. The protein crystal of item 3 or 4, wherein the heterologous protein is an enzyme.

6. The protein crystal of item 5, wherein enzyme is a formate dehydrogenase (FDH), leucine dehydrogenase (LDH), human arginase (hArg), ornithine decarboxylase (ODC), alcohol dehydrogenase (ADH), mandelate dehydrogenase (MDH), carbonyl reductase (CBR), reductive aminase (RedAm), or cytochrome P450 (CYP or P450).

7. The protein crystal of item 3, wherein the fusion polypeptide comprises a Cry protein, a crystal-forming variant of the Cry protein, or a crystal-forming fragment of the Cry protein fused to an enzyme.

8. The protein crystal of item 7, wherein the fusion polypeptide is a Cry3Aa-ADH fusion, Cry3Aa-FDH fusion, Cry3Aa-LDH fusion, Cry3Aa-MDH fusion, Cry3Aa-P450 fusion, Cry1Ab-hArg fusion, Cry1Ab-ODC fusion, or Cry1Ab-P450 fusion.

9. The protein crystal of any one of items 5-8, wherein the protein crystal is bound with a cofactor required for a chemical reaction catalyzed by the enzyme.

10. The protein crystal of item 9, wherein the cofactor is a phosphorylated cofactor.

11. The protein crystal of item 10, wherein the phosphorylated cofactor is nicotinamide adenine dinucleotide (NADH), flavin adenine dinucleotide (FADH), or pyridoxal phosphate (PLP).

12. The protein crystal of item 9, wherein the enzyme is an ADH, FDH, LDH, MDH, CBR, or RedAm and the phosphorylated cofactor is NADH.

13. The protein crystal of item 9, wherein the enzyme is an ODC and the phosphorylated cofactor is PLP.

14. The protein crystal of item 1, comprising two fusion polypeptides.

15. A composition comprising the protein crystal of any one of items 1-14.

16. The composition of item 15, wherein the protein crystal comprises an enzyme capable of catalyzing a substrate in a chemical reaction and a cofactor required for the reaction, and wherein composition further comprises the substrate.

17. The composition of item 15, wherein the protein crystal is formed by a fusion polypeptide comprising a Cry protein fused to an ADH, FDH, LDH, MDH, or P450.

18. The composition of item 15, wherein the protein crystal is formed by a fusion polypeptide comprising a Cry protein fused to an hArg or ODC.

19. The composition of item 15, wherein the protein crystal is formed by two fusion polypeptides.

20. The composition of item 17 or 19, wherein the protein crystal is bound with NADH.

21. The composition of item 18, wherein the protein crystal is bound with PLP.

22. A method for performing a chemical reaction, comprising the step of incubating the protein crystal of any one of items 5-12 with a substrate to the enzyme under conditions permissible for the substrate to be catalyzed by the enzyme in the chemical reaction.

23. The method of item 22, wherein the enzyme is a formate dehydrogenase (FDH), leucine dehydrogenase (LDH), human arginase (hArg), ornithine decarboxylase (ODC), alcohol dehydrogenase (ADH), mandelate dehydrogenase (MDH), carbonyl reductase (CBR), or reductive aminase (RedAm), or cytochrome P450 (CYP or P450).

24. The method of item 22, wherein the enzyme is an ADH, FDH, LDH, MDH, CBR, or RedAm and the protein crystal is bound with NADH.

25. The method of item 22, wherein the enzyme is an ODC and the protein crystal is bound with PLP.

26. The method of item 22, wherein the protein crystal comprises two fusion polypeptides.

27. The method of item 18-21, further comprising, after the reaction is completed, removing the reaction product and reusing the crystal in a second reaction.

28. An *in vitro* method of immobilizing a cofactor by incubating the cofactor with the protein crystal of any one of items 1-14 in a solution and allowing the cofactor to bind to the protein crystal.

29. The method of item 28, wherein the cofactor is NADH or PLP.

30. The method of item 28, further comprising, after the cofactor is bound to the protein crystal, stirring the solution to release the cofactor from the protein crystal.

31. The method of item 28, further comprising, after the cofactor is released from the protein crystal, discontinuing stirring the solution to allow the cofactor to again bind to the protein crystal.

32. A method of delivering a protein of interest to a target cell, comprising contacting the target cell with a pre-formed complex between the protein of interest and the protein crystal of any one of items 1-14.

33. The method of item 32, wherein the protein crystal is a PEI-Cry3Aa protein crystal, and wherein the protein of interest is a negatively charged protein or a protein comprising or linked to a negatively charged moiety.

34. The method of item 32, further comprising, prior to the contacting, incubating the protein of interest with the protein crystal to form a complex.

35. The method of item 32, wherein the protein of interest is a nuclear protein, and wherein the contacting is performed in the presence of an endosomolytic agent.

EXAMPLES

The following examples are provided by way of illustration only and not by way of limitation. Those of skill in the art will readily recognize a variety of non-critical parameters that could be changed or modified to yield essentially the same or similar results.

**Example 1. NADH-dependent Enzyme Crystals and Cry3Aa-PEI Crystals: A Novel Interface for Molecular Exchange under Mechanical Stirring**

NAD(H)-dependent enzymes play a crucial role in the biosynthesis of pharmaceutical and fine chemicals. However, their requirement for the NAD(H) cofactor is a significant barrier to their utilization in industrial biosynthesis due to the high cost of the cofactor. This study introduces polyethylenimine (PEI)-modified Cry3Aa (3A-PEI) crystals as a highly ordered natural scaffold recycling the NADH cofactor for multiple catalytic cycles via its controlled binding and release under mechanical stirring. The 3A-PEI crystals exhibit an impressive NADH binding capacity of $550\pm5$ $\mu$mol/g and retain over 50% of the bound NADH molecules even after 20 washing cycles. Thus, these 3A-PEI crystals can serve as an ideal reservoir for storing NAD(H) cofactor in stirred batch reactions - they release NADH molecules under mechanical stirring, allowing them to participate in the NADH-dependent processes, and rebind the NADH when the stirring is stopped. To demonstrate the utility of this NADH recycling technology in practical application, we have produced a second Cry3Aa protein particle comprised of genetically co-immobilized formate dehydrogenase (FDH) and leucine dehydrogenase (LDH) that can promote the synthesis of L-tert-leucine. Here, the FDH regenerates the NADH with concomitant oxidation of sacrificial formate, while the LDH performs the catalytic reaction of interest. Reaction solutions containing mixtures of NADH-loaded 3A-PEI and Cry3Aa-FDH/Cry3Aa-LDH crystals allow for efficient biosynthesis of L-*tert* leucine for more than 20 days with minimal addition of NADH (NADH turnover number: 284). This innovative approach provides an important step forward towards addressing the challenge of NAD(H) cofactor utilization in industrial NAD(H)-dependent biosynthesis by providing a sustainable and efficient means for maintaining a continuous NADH supply for multiple reaction cycles.

INTRODUCTION

NADH-dependent enzymes have demonstrated outstanding efficiency and enantioselectivity in drug biosynthesis, making them highly important for practical application [1]. Nevertheless, the high cost of NAD(H) production, 3000 USD per mol in bulk quantities [2], and the need for stoichiometric replenishment enzymatic catalysis are significant economic challenges which limit their large-scale utilization. Hence, the regeneration and recycling of NAD(H) are two important technical hurdles for the practical use of NAD(H) dependent enzymes to produce chiral pharmaceutical compounds.

While several techniques have been developed to regenerate NADH [1-3], enzymatic NADH regeneration is the primary approach utilized in the industrial sector due to its compatibility with other biosynthetic systems, mild reaction conditions, high selectivity and specificity toward NAD(H), and a low energy requirement [4]. In this regard, formate dehydrogenase (FDH) is an attractive enzyme for this purpose since it can catalyze the conversion of $NAD^+$ into NADH via the concomitant oxidation of formate to produce $CO_2$ - a byproduct that can be easily separated from the supernatants during workup [1, 4].

The recycling of the NAD(H) cofactor for multiple reaction cycles is an equally important challenge as it obviates the need to add NADH at the start of each reaction cycle, and thereby greatly reduces the amount of costly NADH cofactor required. Several approaches have been reported for NAD(H) recycling including ionic absorption [5], covalent immobilization [6], encapsulation [7, 8], and membrane filtration [8], however, these approaches each have different limitations including weak binding [9], support defects [9], diffusion problems [7], low support capacity for cofactor preservation [6], and the need for complex molecular assembly and design [10, 11], that hinder their general application. Thus, the development of a simple and practical solution for NADH recycling would be of great benefit.

We have been using Cry3Aa protein crystals produced in *Bacillus thuringiensis* (*Bt*) for multiple applications. The unique biological self-assembly properties of the Cry3Aa protein enable the entrapment of co-expressed protein targets within the Cry3Aa lattice, or direct immobilization of genetically-fused functional enzymes or proteins in a fashion that stabilized the fused protein against thermal and solvent denaturation[12, 13]. We successfully utilized the later strategy for the production of Cry3Aa-lipase A and Cry3Aa-PML fusion crystals, both of which showed high activity and recyclability for the conversion of vegetable oils to FAME biodiesel. The direct entrapment of PML within Cry3Aa crystals was demonstrated *in vitro* as well as in bacteria [12]. The entrapped PML was found to be even more active than for the Cry3Aa-PML fusion crystals, and shown to be suitable for the recyclable conversion of waste cooking oil to FAME biodiesel [14].

We became interested in cofactor entrapment within Cry3Aa crystals due to the initial observation of NADH binding affinity to Cry3Aa crystals. This observation was likely due to the long nanochannels within the Cry3Aa crystals, which presumably contribute to the absorption of NADH molecules. The level of NADH binding was suboptimal for practical application, and thus we directed our exploration to protein cationization as a prospective strategy to improve the binding capacity of the negatively charged cofactor for the crystals. Previous studies by our lab had generated a positively charged variant of Cry3Aa, Pos3Aa, by mutating multiple residues on the surface of domain II of Cry3Aa to lysines creating a positively charged surface [15]. While Pos3Aa crystals could bind more NADH that Cry3Aa, the levels were still too low to be of use for biocatalytic studies.

We therefore sought strategies to achieve greater levels of cationization, and decided to explore modifying the Cry3Aa crystals with polyethylenimine (PEI) due to its high positive charge and flexibility. Here, we demonstrate that cationization of Cry3Aa crystals using PEI was indeed effective in promoting high levels of NADH binding in amounts sufficient for use as a NAD⁺/NADH reservoir for facilitating NADH-dependent enzyme reactions. A simple strategy to facilitate the reversible release of the NADH from the Cry3Aa-PEI crystals involving mechanical stirring was subsequently developed to regulate the release of NADH molecules from Cry3A-PEI crystals into the reaction solution, and its rebinding. Finally, we couple this NADH capturing technology with an advanced version of our Cry3Aa enzyme immobilization technology to produce a biocatalyst comprised of co-immobilized formate dehydrogenase (FDH) that can regenerate the NAD⁺ released by the Cry3Aa-PEI crystals to NADH, and leucine dehydrogenase (LDH) which can then use the NADH for the biosynthesis of for L-*tert*-leucine, a chiral precursor that has been widely used in pharmaceutical industry to produce various type of therapeutic agents.

METHODS AND MATERIALS

3-aminopropyl-triethoxysilane (APTMS), O-phthalaldehyde (OPA), 1-fluoro-2,4-dinitrophenyl-5-L-alanine amide, nicotinamide adenine dinucleotide (NAD(H)), trimethylpyruvic acid (TMP), L-*tert*-leucine, and D-*tert*-leucine were obtained from the TCI company. Ammonia formate, polyethylenimine (PEI), and other buffer salts were also purchased from Sigma-Aldrich. Additional compounds and chemicals are given in parenthesis below.

**Production of 3A-PEI crystals**

Cry3Aa crystals were produced by transforming *Bacillus thuringiensis BT407G* cells with a previously prepared PHT-Cry3Aa plasmid and growing the cells for three days. The cells were harvested and lysed with lysozyme, and the Cry3Aa crystals were purified via low-speed centrifugation (6000 rpm) after washing with 10% hexane to remove the spores[16]. A solution of 0.5 mg/mL Cry3Aa crystals was mixed in PEI solution (50 μg/mL, pH:7.0) and then subjected to the gradual addition of EDC/NHS coupling reagents (1:2 molar ratio) under magnetic stirring. Following a 5-h reaction, the reaction was quenched using a solution of 100 mM Tri buffer at pH 7.0. Subsequently, the samples were centrifuged, washed multiple times with NaCl (100 mM), and then ddH₂O to eliminate any non-crosslinked or weakly bound PEI molecules.

**Quantification of PEI bound to Cry3Aa crystals**

To quantitate the PEI bound to Cry3Aa crystals, a PEI standard solution was prepared by dissolving 500 mg of PEI solution (50% v/v) in 50 ml of ddH₂O for 2 h, and then adjusting the pH to 7.0 by gradual addition of 5M HCl. Taking advantage of fact that PEI forms a blue colored complex with $Cu^{2+}$, 130 mM of copper (II) sulphate was fixed in the presence of different amount of PEI (0-50 μg/ml) to determine a standard curve for quantification of PEI in the solution. Subsequently, this standard curve was used for evaluation the PEI bound to the crystals using the residual amount of PEI after the cationization procedure [17, 18]. All assays were carried out at room temperature against copper (II) sulphate solution as a blank.

**Binding of NADH to 3A-PEI crystals**

The binding capacity of NADH to 3A-PEI crystals was determined by combining 50 μg of dried 3A-PEI crystals and NADH (0.25 mM) at 10°C for 30 min under gentle rotation. Then, the amount of NADH bound to the 3A-PEI crystals was quantified by measuring the absorbance of the supernatant at a wavelength of 340 nm using an Eppendorf BioSpectrometer (Eppendorf AG, Germany) after centrifugation at 15,000 rpm for 5 min. The NADH binding into the crystals was then defined as the number of moles of NADH bound per unit of dried weight of 3A-PEI crystals, which are shown in the 3A-PEI-NADH crystals (dried weight of 3A-PEI, moles of bound NADH) in this study. The binding of NADH molecules was further studied by using a Leica SP8 confocal microscope in DAPI channel. The excitation and emission wavelengths for NADH fluorescence were tuned to 335-350 nm and 440-470 nm, respectively [4]. To compare the binding capacity of 3A-PEI crystals with Cry3Aa and Pos3Aa crystals, the same experiment was conducted as the control.

***In vitro* growth of Cry3Aa crystals**

Cry3Aa protein crystals were dissolved in a solution containing 50 mM Na₂CO₃, 150 mM NaCl, and adjusted to pH 11. To solubilize and purify the Cry3Aa protein, a gel filtration technique was employed using a high-resolution size exclusion column (Bio-rad ENrichTM SEC 650). *In vitro* growth of Cry3Aa crystals was achieved at 10 °C using a sitting-drop vapor diffusion method. This involved combining 1 μL of protein solution (8.3 mg/mL) with 1 μL of the well solution (20% v/v 1, 4-butanediol, 100 mM HEPES/NaOH, 200 mM NaCl) in a 96-well crystallization plate (Hampton)[15]. The obtained Cry3Aa crystals were gently transferred to a solution containing 100 mM HEPES/PEI (50 $\mu$g/mL) and EDC/NHS coupling reagents (5 mM and 10 mM, respectively) at pH 7.5. The samples were incubated for 2 h at room temperature, followed by treatment with 100 mM Tri buffer (pH 7.5) for 30 min to deactivate any residual EDC/NHS cross-linkers. Subsequently, the samples were centrifuged and washed multiple times with HEPES (100 mM) and then ddH$_2$O to remove excess salts before being stored at 4°C for further use.

### Crystallization, data collection, phase determination and refinement

3A-PEI crystals were flash-frozen in liquid nitrogen, and 30% glycerol was used as a cryoprotectant. Subsequently, the datasets were collected on the microfocus beamline 05A at NSRRC, utilizing the RAYONIX MX-300 HS detector. Data processing and scaling were performed using the HKL2000 software[19]. To generate the initial structure, PHASER was employed under the CCP4i interface, with Cry3Aa structure (PDB ID: 1DLC) serving as the search model [20]. Iterative cycles of model building, and refinement were conducted using Coot, Rafmac5, and Phenix programs, respectively, to enhance the quality of the model [14, 21-23].

### Scanning electron microscope (SEM) of 3A-PEI crystals

Scanning electron microscopy (SEM) was used to study the morphology of *in vitro* and *in vivo* grown 3A and 3A-PEI (SEM). After incubating the samples in ddH$_2$O (0.2 mg/ml), 5 $\mu$L was placed on a carbon glass and allowed to dry gradually overnight before being coated with 80/20 Pd/Pt using a Q150T ES turbomolecular pumped coater (Quorum). The images were captured using a Hitachi SU8000 instrument.

### Distribution of PEI and NADH molecules within *in vitro* grown 3A-PEI crystals

The localization of PEI polymer within Cry3Aa crystals was assessed by first labeling of the *in vitro* grown crystals by mixing with Alexa Fluor™ 568 C5 Maleimide (Thermo Fisher Scientific) (1 mM) in 100 mM HEPES buffer (pH 7.5) for 1h under gentle rotation. Subsequently, the crystals were thoroughly washed multiple times with the same buffer to remove any unbound Alexa 568 molecules. In parallel, a solution of PEI (50 $\mu$g/mL) was incubated with Alexa Fluor™ 488 NHS Ester (Succinimidyl Ester) (Thermo Fisher Scientific) (1 mM) in 100 mM HEPES buffer (pH 7.5) for 1h under gentle rotation. Following this, the PEI solution was washed with 100 mM HEPES buffer using a centrifugal filter (3 kDa, Amicon® Ultra) multiple times to obtain the labeled PEI-Alexa488 polymer. Finally, the labeled Cry3Aa -Alexa568 and PEI-Alexa488 polymers were utilized for the cationization procedure to produce Cry3Aa-Alexa568-PEI-Alexa488 crystals. The obtained crystals were then incubated in the presence of NADH (0.25 mM) in ddH$_2$O under slow rotation at 10 °C for 30 min. After several ddH$_2$O washings to remove the unbound NADH molecules, the Cry3Aa-Alexa568-PEI-Alexa488-NADH crystals were analyzed by a Leica SP8 confocal microscope using DAPI, Alexa568, and Alexa488 channels.

### Dynamic Light Scattering (DLS)

The Zeta potential and hydrodynamic diameter of Cry3Aa and 3A-PEI crystals were measured at 25°C using a Malvern Zetasizer Nano ZS90 (Malvern Instruments, UK). 100 $\mu$g/mL crystals were individually prepared in ddH$_2$O solution for measurement of size distribution and PBS (10 mM) for zeta potential.

### Size distribution of PEI polymers bound in Cry3Aa crystals

The size distribution of PEI solution within Cry3Aa crystals were measured by immersing of Cry3Aa crystals (5 mg/ml) in a PEI solution (50 $\mu$g/ml, pH 7.0) while being gently stirred using a magnetic stirrer. This was followed by the gradual addition of EDC/NHS cross-linker. Afterward, the samples were subjected to centrifugation at a speed of 15,000 rpm for 10 min, allowing the crystals to separate from the surrounding solution. The resulting supernatants were then analyzed using Dynamic Light Scattering (DLS) to determine the size distribution of PEI molecules present. For comparison, a PEI solution (50 $\mu$g/ml, pH 7.0) was included as a control in this experiment.

### Retention of NADH on 3A-PEI crystals over multiple washing cycles

The capacity of 3A-PEI crystals to retain bound NADH was measured by incubation of NADH loaded 3A-PEI crystals (10mg, 216± 3 nmol NADH) in sodium phosphate buffer (Na-PB) (10 mM, pH 7.0) under slow rotation at 10°C. Afterwards, the samples were centrifuged at 15,000 rpm for 5 min, and the amount of released NADH in the supernatant was measured spectrophotometrically at 340 nm. This process was repeated for a total of 20 cycles. Subsequently, the samples were resuspended in 1 M Na-PB and vigorously vortexed to release all bound NADH.

### Effect of pH on NADH binding to 3A-PEI Crystals

To determine the effect of pH on the NADH binding capability of 3A-PEI crystals, 10 mg of 3A-PEI crystals were individually prepared in different pH (6.0, 7.0 and 8.0) of Na-PB (25 mM), then incubated in the presence of 2 mM NADH under slow rotation at 10°C. Then, the samples were centrifuged at 15,000 rpm for 5 min, and the amount of NADH bound to the 3A-PEI crystals were measured spectrophotometrically at 340 nm. NADH solution (2 mM) was treated as the control in all pHs.

### Impact of salt concentration on NADH binding to 3A-PEI crystals

3A-PEI-NADH crystals (10mg, 183$\pm$ 2 nmol NADH) was prepared in Na-PB (25 mM, pH:7.0), and then, incubated in the presence of gradual increase of NaCl (0-800 mM) separately for 10 min at 25°C. Then, the samples were centrifuged at 15,000 rpm for 5 min, and the amount of NADH released from the 3A-PEI crystals were measured spectrophotometrically at 340 nm.

### NADH release from 3A-PEI Crystals by mechanical stirring

The potential impact of mechanical agitation on the release rate of NADH molecules from 3A-PEI crystals was investigated by resuspending of 3A-PEI-NADH crystals (10 mg, 2 $\mu$mol) in sodium phosphate buffer(Na-PB) (10 mM, pH 7.0), and subjected to incubation at different shaking speeds (0, 400, 800,1200,1600 and 2000 rpm) (Eppendorf® ThermoMixer® C, Eppendorf AG, Germany) for 10 min at 25°C. Subsequently, the samples were immediately centrifuged at 15,000 rpm for 5 min, and the amount of released NADH was determined spectrophotometrically at 340 nm using a UV-visible spectrophotometer (Eppendorf BioSpectrometer, Eppendorf AG, Germany).

### The rebinding of NADH molecules into 3A-PEI crystals

The rebinding of NADH molecules into 3A-PEI crystals was investigated by first applying mechanical stirring to 3A-PEI-NADH crystals (10 mg, 2 $\mu$mol NADH) in Na-PB buffer (10 mM, pH: 7.0) for 10 min at 1000 rpm and 25 °C. Subsequently, the crystals were immediately centrifuged at 15,000 rpm for 5 min, and the amount of the released NADH was quantified. Then, the supernatant was returned to the original tube to resuspend the precipitated crystals. The suspension was then incubated for 30 min under gentle rotation at 10 °C. Next, the sample was centrifuged, and the amount of NADH bound to the crystals was measured.

### NADH binding to 3A-PEI crystals as a **function of mechanical agitation speed**

To determine the $K_d$ and $B_{max}$ constants of NADH binding into 3A-PEI crystals at different mechanical shaking conditions, a fixed amount of 3A-PEI crystal (0.1 mg) was incubated with different concentrations of NADH (0-1000 $\mu$M) at different mechanical shaking conditions (0, 400, 800, 1200, 1600, and 2000 rpm) for 5 min at 25°C. Then, the samples were subsequently centrifuged at 15000 rpm for 5 min to measure the amount of NADH bound to the crystal at 340 nm.

### Production of PEI-modified mesoporous silica beads

Different mesoporous silica beads Glantreo Company, Ireland) with diameters of either 1 $\mu$m or 150 nm, and pore sizes of either 40 Å or 100 Å were employed to produce PEI particles for comparison with 3A-PEI crystals. Each silica bead was individually treated with APTMS to expose amino groups by treating the surface hydroxyl groups [24, 25]. This treatment was achieved by immersing the beads in a 0.2% APTMS solution in anhydrous toluene under a vacuum for 15 hours. The beads were then washed with a mixture of toluene and methanol (50% v/v) to remove residual methanol. Afterward, the beads were dried at 80°C for 4 hours. Amino-functionalized silica beads were subjected to gentle rotation and immersed in a solution of glutaraldehyde (0.5% wt in PBS, 100 mM, pH:7.0) as a cross-linker for 30 minutes at 25°C. Subsequently, a PEI solution (0.6 mg/mL) was added to the functionalized beads in a PBS buffer (100 mM, pH: 7.0) and allowed to rotate gradually for 2 hours at 25°C. To block any remaining free amine groups, the beads were flushed with ethanolamine-HCl (0.5 M, pH 8.5). The quantity of PEI remaining in the solution was used to determine the amount of PEI bound to the beads. The samples were then washed multiple times with ddH$_2$O and incubated with a solution of 2.0 mM NADH in ddH$_2$O for 30 min at 10°C to determine the binding capacity. The quantity of bound NADH in the beads was determined at 340 nm. The immobilization of NADH on the beads was further analyzed using confocal fluorescence microscopy.

**Cry3Aa mediated co-immobilization of GFP and mCherry proteins**

The plasmids of pHT-Cry3 Aa-mCherry and pHT315-Cry3Aa-GFP were previously provided[26]. To prepare the pHT315-Cry3Aa-GFP-RBS-Cry3Aa-mCherry plasmid, the Cry3Aa-mCherry gene was amplified by Kappa HiFi DNA polymerase (Kappa Biosystems) using the primers that had a ribosome binding site (RBS) at the upstream **(Table** 1). Subsequently, pHT315-Cry3Aa-GFP plasmid was first amplified in XL10-Gold® ultracompetent cells and then digested by *KpnI* after purification by Takara MiniBEST kit 4.0. Using the Gibson Assembly Reaction (NEB), the amplified Cry3Aa-mCherry gene was fused to the downstream of the linearized pHT315-Cry3Aa-GFP. After the sequencing of the plasmid, it was transferred to ER2925 strain via heat shock for unmethylation. Then, the plasmid was purified, and used for transformation into *Bacillus thuringiensis BT407G* cells via electroporation. The cells were expressed in SSM medium (schaeffer's sporulation) in the presence of Erythromycin (50 μg/ml) at 25°C and 220 rpm for 72h, and then harvested by centrifugation at 8000 rpm. After incubation of the cells within chicken lysozyme (Sigma) (1mg/ml) for overnight, the samples were sonicated at 3 cycles of 2 seconds on, 2 seconds off, at 40% power in an ice/water pack. The samples were subsequently washed with 0.5 M NaCl, 0.25 M NaCl, and ddH$_2$O for fluorescence microscopy studies using Nikon TE300 microscope. The coefficient values of the Pearson correlation were acquired by ImageJ software.

**Cry3Aa NADH dependent enzyme fusion crystals**

To produce Cry3Aa fusion NADH dependent enzyme fusion crystals, the pHT315-Cry3Aa-FDH, pHT315-Cry3Aa-LDH,and pHT315-Cry3Aa-FDH/Cry3Aa-LDH plasmids were constructed as follows: to construct the expression plasmids for soluble formate dehydrogenase (FDH), leucine dehydrogenase (LDH), and alcohol dehydrogenase (ADH), The *fdh* gene from *Thiobacillus sp.* KNK65MA and the *adh* gene from *Bartonella apis* were synthesized Integrated DNA Technologies, while the LDH gene was amplified from the genome of *Bacillus thuringiensis 407G* using *Kappa HiFi DNA polymerase* (Kappa Biosystems) following the standard protocol. Subsequently, the corresponding genes were cloned into a pET28b vector downstream orientation using *XhoI* and *NdeI* restriction sites through the Gibson Assembly Reaction (NEB). This resulted in the insertion of C-terminal His tags for FDH and N-terminal His tags for LDH and ADH. To construct the plasmids for expression of Cry3A-FDH, Cry3A-LDH, and Cry3Aa-ADH, FDH, LDH and ADH genes were fist amplified by PCR reaction, and then inserted into the downstream of the Cry3A gene in pHT315 vector using Gibson Assembly at the *BamHI* and *KpnI* restriction sites. To provide the plasmids for co-expression of Cry3A-FDH and Cry3Aa-LDH or Cry3Aa-FDH and Cry3Aa-ADH, pHT315-Cry3Aa-FDH plasmid was first amplified in XL10-Gold® ultracompetent cells, and then digested by *KpnI* after purification by Takara MiniBEST kit 4.0. Then, Cry3A-LDH and Cry3A-ADH genes were individually amplified with primers containing the ribosome binding site (RBS). Finally, Gibson Assembly Reaction (NEB) was used to construct the co-expression plasmids of pHT315-Cry3A-FDH/Cry3A-LDH, pHT315-Cry3A-FDH/Cry3A-ADH and pHT315-Cry3A-FDH/Cry3Aa-FDH/Cry3A-ADH using the linearized pHT315-Cry3Aa-FDH at *KpnI* restriction site, and the amplified Cry3A-FDH and Cry3A-ADH DNA fragments. A detailed list of the primers employed for creating the distinct clones can be found in **Table 1.** All DNA modification experiments were conducted using XL10-Gold® ultracompetent cells. Plasmid purification was carried out using the Takara MiniBEST kit 4.0, and the successful plasmids were sequenced by BGI.

**Table 1.** The list of primers utilized for cloning (5' - 3'). The restriction sites are underlined.

| | |
|---|---|
| pET28- FDH Fwd | GAAGGAGATATACCATGGCCAAAATTCTGTGCGTGC (SEQ ID NO: 9) |
| pET28- FDH Rev | TCGACGGAGCTCGAATTCTTAATGATGATGATGATGATGGCCTGCTTTCTTAAATTTGGCTGCC (SEQ ID NO: 10) |
| pET28- LDH Fwd | CCGCGCGGCAGCCATATGACATTAGAAATCTTCG (SEQ ID NO: 11) |
| pET28- LDH Rev | GTGGTGGTGGTGCTCGAGTTAGCGACGGCTAATAATATCG (SEQ ID NO: 12) |
| pET28-BaADH Fwd | CCGCGCGGCAGCCATATGCGGGTAAAAGACAAGGTAGCC (SEQ ID NO: 13) |
| pET28-BaADH Rev | GTGGTGGTGGTGCTCGAGTTACTGAGCTGTGTACCCTCCG (SEQ ID NO: 14) |
| pHT-Cry3A-FDH Fwd | GAATTTATTCCAGTGAATGGATCCGCAGCTGCGATGGCTAAAATACTGTGTGTCCTG (SEQ ID NO: 15) |
| pHT-Cry3A-FDH Rev | CAGTGAATTCGAGCTCGGTACCTCATCCGGCTTTCTTGAACTTAGC (SEQ ID NO: 16) |
| pHT-Cry3A-LDH Fwd | TTTATTCCAGTGAATGGATCCGCGAGCTGCGATGACATTAGAAATCTTCG (SEQ ID NO: 17) |
| pHT-Cry3A-LDH Rev | CAGTGAATTCGAGCTCGGTACCTTAGCGACGACGGCTAATAATATCGTGACCG (SEQ ID NO: 18) |
| pHT-Cry3A-FDH-rbs-Cry3A-LDH Fwd | AAGAAAGCCGGATGAGGTACCAAAGGGAGGAAGAAAAATGAATCCGAACAATCGAAGTG (SEQ ID NO: 19) |
| pHT-Cry3A-FDH-rbs-Cry3A-LDH Rev | CAGTGAATTCGAGCTCGGTACCTTAACGACGGCTAATAATCGTGACCG (SEQ ID NO: 20) |
| pHT-Cry3Aa-FDH-rbs-Cry3Aa-ADH Fwd | AAGAAAGCCGGATAAGGTACCAAAGGGAGGAAGAAAAATGAATCCGAACAATCGAAGTG (SEQ ID NO: 21) |
| pHT-Cry3Aa-FDH-rbs-Cry3Aa-ADH Rev | AGTGAATTCGAGCTCGGTACCTTAC (SEQ ID NO: 22) |
| pHT-Cry3Aa-FDH-rbs-Cry3Aa-FDH-rbs-Cry3Aa-BaADH Fwd | GGAGGAAGAAAAACTCGAGATGAATCCGAACAATCGAAGTG (SEQ ID NO: 23) |
| pHT-Cry3Aa-FDH-rbs-Cry3Aa-FDH-rbs-Cry3Aa-BaADH Rev | GTTCGGGATTCATTTTTCTTCCTCCCTTTGGTACCTT TCCGGCTTTCTTGAACTTAGCG (SEQ ID NO: 24) |

The plasmids were then individually introduced into *Bacillus thuringiensis BT407G* cells via electroporation. The cells were then cultured in 500 mL SSM medium containing sporulation salt (schaeffer's sporulation), and 50 $\mu$g/ml Erythromycin at 25°C and 220 rpm for 72 h. Following the cultivation, the cells were collected by centrifugation at 8000 rpm for 8 min using an Avanti J25 Ultracentrifuge (Beckman Coulter). The cell pellets were then resuspended in ddH$_2$O in the presence of chicken lysozyme (Sigma) (1mg/ml) for overnight at room temperature, followed by sonication in ice-water pack for 30 min to achieve complete cell lysis. After that, the crystals were purified with 0.5 M NaCl, 0.25 M NaCl, and ddH$_2$O. The proteins were then characterized using a 10% SDS-PAGE gel. The concertation of the crystals was determined using Bradford assay (Bio-Rad).

## Combining 3A-PEI-NADH and Cry3Aa-FDH/Cry3Aa-LDH crystals for *L-tert* Leu biosynthesis

In this study, the aim was to explore the recyclability and retention of NADH in a coupled system with NADH-loaded 3A-PEI and Cry3Aa-FDH/Cry3Aa crystals for the biosynthesis of L-tert-leucine (L-*tert*-Leu). For this aim, 12 mg of 3A-PEI crystals were initially incubated in the presence NADH (0.5 mM ) in ddH$_2$O under slow rotation at 10 °C for 10 min. Then, the amount of bound NADH was quantified using the spectroscopic method at 340 nm. After several washings of the crystals with ddH$_2$O, the provided 3A-PEI crystals (0.5 $\mu$mol loaded NADH) were mixed with Cry3Aa-FDH/Cry3Aa-LDH crystals (12 mg/ml) in a reaction buffer containing 30 mM ammonia formte (pH 8.0) and TMP (25 mM). Then, the samples were subjected to mechanical stirring (1000 rpm) for 23.5 h at 25 °C. Then, the samples were taken and incubated at 10 °C for 30 min before starting the next cycle. The samples were centrifuged at 7,000 rpm for 7 min for 10 min before analyzing the supernatant by HPLC. Cry3Aa-FDH/Cry3Aa-LDH crystals were used as the control, with the addition of 0.5 $\mu$mol NADH at each cycle under the same condition. When the reduction in activity was seen relative to the control group, a partial amount of NADH was added to the 3A-PEI and Cry3Aa-FDH/Cry3Aa crystal mixture to keep the conversion at the highest rate. This process was repeated for 21 cycles.

RESULTS

## Production and Characterization of 3A-PEI Crystals

Cry3Aa is a self-crystallization protein produced naturally by certain strains of *Bt.* Our group has used crystals and particles of Cry3 Aa for entrapment of positively charged small peptides and proteins due to the presence of abundant negatively charged residues, aspartic acid (Asp) and glutamic acid (Glu) residues, on the exposed surface of its domain II, which forms the solvent channels **(Figure 1A).** We have engineered a protein called Pos3Aa, derived from the replacement of all these residues in domain II of Cry3Aa with positively charged arginine (Arg) and lysine (Lys) residues, which provide positively charged channels within the crystals. We hypothesized that these crystals would be able to entrap negatively charged molecules, such as phosphorylated cofactors, ionically inside the channels. Thus, initially, we tested the ability of Pos3Aa crystals to bind NADH. While the NADH binding was achievable, the quantity of NADH bound was deemed inadequate for practical implementation. Hence, we explored an alternative approach by considering the cationization of crystals with polyethyleneimine (PEI). Each molecular mass unit of this polymer (43 kDa) has 3 to 3.6-fold higher protonated nitrogen than poly-Arg and poly-Lys (one positive charge per 157 and 129 molecular masses), respectively. Therefore, it was expected that the cationization process would generate highly positively charged crystals.
To produce PEI-modified Cry3Aa crystals (3A-PEI), Cry3Aa crystals were purified using standard protocol. The purity of the crystals was then analyzed using a 10% SDS-PAGE gel. The distinct band at 73 kDa confirms the high purity of the obtained crystals. Cry3Aa crystals were then incubated in the presence of PEI polymer for 30 min under slow rotation at room temperature. It was speculated that this incubation allowing PEI molecules to penetrate into the channels of Cry3Aa crystals. Subsequent addition of EDC/NHS was used to permanently cross link the PEI to the crystal **(Figure 1B).**
The stable coordination of PEI with Cu$^{2+}$ ions makes a dark blue color, which can be used for visualization and quantification [27]. The obtained crystals were resuspended and washed with NaCl (100 mM) and ddH$_2$O several times to remove all non-covalently bound PEI or other molecules before being stored at 4 °C for further use. When 3A-PEI reacts with Cu$^{+2}$ ions, an immediate color change from white to a distinct dark blue hue occurs, confirming the binding of PEI within Cry3Aa crystals. In contrast, Cry3Aa crystals alone did not manifest any color change in the presence of Cu$^{+2}$ ions. The concentration of PEI in Cry3Aa crystals was determined to be approximately $12 \pm 0.3$ mg of PEI per g of Cry3Aa crystals. According to our calculations, the molar ratio should be 0.001 PEI:Cry3Aa monomer. Assuming the dimensions of Cry3Aa crystal are 1.6 $\mu$m $\times$ 0.80 $\mu$m $\times$ 0.80 $\mu$m [49], it is suggested that one single Cry3 Aa crystal should have $6425 \pm 2102$ PEI molecules when the binding and distribution are the same for all crystals.
Scanning electron microscopy (SEM) images showed that Cry3Aa crystals were rod-like crystals with an average length and width of 1.6 $\times$ 0.8 $\mu$m, which is similar to that of 3A-PEI crystals **(Fig. 2A-D**The size distribution determined by dynamic light scattering (DLS) for both the Cry3Aa and 3A-PEI crystals was coincident with their average hydronamic diameters of ~825 nm (PDI: 0.08) and ~ 914 nm (PDI: 0.102), respectively **(Fig. 2E).** We hypothesize that the slight increase observed in the DLS study for 3A-PEI crystals compared to Cry3Aa crystals could be attributed to the binding of some PEI molecules on the surface of the crystals. Overall, the results indicate that PEI binding to Cry3Aa crystals did not have a considerable effect on crystal size, crystallinity, and morphology. Zeta potential analysis of the Cry3Aa and 3A-PEI crystals, however, exhibited a dramatic change from a zeta potential of -35 mV for the original crystal to a positive value of +45 mV for 3A-PEI crystals **(Fig. 2F).** These results demonstrate the successful PEI mediated cationization of Cry3Aa crystals.

**Cofactor binding and retention with 3A-PEI Crystals**

NADH binding was assessed using spectroscopic method at 340 nm after incubation of NADH solution with the crystals. The NADH binding capacity of 3A-PEI crystals was determined to be $550\pm5$ $\mu$mol/g of 3A-PEI crystals. Although Cry3Aa, and its positive variant, Pos3Aa, crystals are capable of NADH binding ($6\pm2$ and $11.2\pm0.7$ $\mu$mol/g, respectively), their utilization is unsuitable for practical applications due to low binding capacity **(Figure 3A).** In other words, 3A-PEI crystals exhibited 91-fold and 50-fold higher NADH binding capacity than Cry3Aa and Pos3Aa crystals, respectively, demonstrating their superior ability to immobilize NADH. To evaluate the retention of NADH by 3A-PEI crystals, washing test was performed. As shown in **Fig. 3B** less than 50% of bound NADH to the 3A-PEI crystals was released after 9 cycles of washing with Na-PB (10 mM, pH:7.0), whereas the bound NADH remained stable up to twenty cycles of washing without significant lose. These results indicate that 3A-PEI crystals have high capacity for retention of NADH molecules. To confirm that the binding between NADH molecules and 3A-PEI crystals is electrostatic interactions, 1M Na-PB (pH:7.0) was used to release all NADH molecules from the crystals after 20 washing cycles. NaPB buffer consists of phosphate ($PO_4^{-3}$) ions that can compete with NADH molecules to bind PEI molecules.

To evaluate whether 3A-PEI crystals could binding to other phosphorylated cofactors, PLP, NAD$^+$, NADP$^+$, NADPH , FADH$_2$, CoA, Riboflavin, and TPP (each at a concentration of 0.25 mM) were incubated with 20 $\mu$g of 3A-PEI crystals for 30 min under slow rotation at 10°C. Subsequently, the crystals were centrifugated and the amount of bound cofactor within the crystals was quantified. As shown in Fig. 3C, 3A-PEI crystals are able to retain these other phosphorylated cofactors as well. Among the studied cofactors, PLP exhibits the strongest binding affinity ($650\pm2$ $\mu$mol/ g of support), whereas TPP demonstrates the weakest binding affinity ($160\pm1$ $\mu$mol/ g of support) toward 3A-PEI crystals. These data indicate that 3A-PEI crystals can be considered as a general platform for retention of cofactors in biocatalytic systems.

**Structural details of 3A-PEI crystals**

Cry3Aa crystals possess a consistent packing interface both *in vitro* and *in vivo,* thereby demonstrating an identical structure under both conditions. We used this advantage to explore the impact of PEI modification on the crystal structure. For this purpose, Cry3Aa crystals were purified and solubilized, then used to produce micrometer-sized crystals under *in vitro* conditions. The grown crystals were then modified using PEI with the same procedure as previously described. X-ray crystallography was subsequently used to determine the structure of these PEI-modified *in vitro*-grown Cry3Aa crystals. Analyses of the structures showed no evidence of any altered or specific interaction between the PEI polymer and the crystals **(Table 1).** These data indicate that the binding of PEI molecules within the Cry3Aa crystals occurred in a random manner.

While the confocal imaging of PEI modified *in vivo* grown Cry3Aa crystals was performable, these crystals are too small to determine the localization and distribution of NADH and PEI molecules within the crystals. Therefore, PEI-modified *in vitro*-grown Cry3Aa crystals were selected for this analysis. Here, we take advantage of the blue fluorescence of NADH in the DAPI channel to display the binding pattern of NADH molecules within the crystals using confocal fluorescence microscopy. For this aim, PEI-modified *in vitro*-grown Cry3Aa crystals were incubated in the presence of NADH solution and then used for confocal fluorescence microscopy studies after several washes with ddH$_2$O. NADH molecules are distributed and localized in the whole crystal. Z-stacking analysis confirmed that NADH molecules are localized inside the crystal.

The binding of PEI molecules in the Cry3Aa crystals is another important question that needs to be answered. In order to address this matter, the Cry3Aa crystals were labeled with Alexa 568 fluorophores (appearing red), while the PEI molecules were labeled independently with Alexa 488 fluorophores (green color). Subsequently, the PEI modification procedure was conducted as previously described. PEI molecules are localized throughout the crystal. Z-Stacking analysis indicated that the crystals were filled with PEI molecules, demonstrating that the PEI polymer can penetrate the crystals. Upon comparing the size distribution of PEI polymers before and after labeling with Cry3Aa crystals, it was recognized that those with a size distribution of less than 5 nm are more likely to attach to the crystals. These data support the concept that PEI molecules enter and label the interior of these channels. To obtain a 3D reconstruction of NADH loaded 3A-PEI crystal, PEI-Alexa488 modified Cry3Aa-Alexa568 crystals were incubated in the presence of NADH molecules, and then analyzed simultaneously in Alexa488, Alexa568 and DAPI channels **(Figure 4).**

**Stability of 3A-PEI-NADH crystals**

The interaction between NADH molecules and 3A-PEI crystals can be impacted by two main factors, including pH and ionic strength. pH is a key element that can affect the protonation state of the amine groups on PEI molecules (pKa value ~9.6). These amine groups become protonated under acidic circumstances, resulting in an increase in positive charge, while the positive charge decreases under basic conditions due to deprotonation. Na-PB buffer has a pH range between 5.8 and 8.0, which can be used for studying the effects of pH on NADH binding into 3A-PEI crystals. As shown in **Figure 5A,** the NADH binding capacity of 3A-PEI crystals was $412 \pm 4$ $\mu$mol/g in NaPB (25 mM, pH: 7.0). However, when the pH decreased to 6.0 in the same buffer, the NADH binding capacity reached $453.5 \pm 2.8$ $\mu$mol/g. On the other hand, the NADH binding capacity decreased to $350.2 \pm 3.6$ $\mu$mol/g when the pH was adjusted to 8.0. These results indicate that the stability of NADH molecules and 3A-PEI crystals is dependent on pH.

Ionic strength, the second key factor, is described as the concentration and charge of ions in a solution. In this study, the release of NADH molecules from 3A-PEI-NADH crystals was measured while employing a fixed concentration of NaPB (25 mM, pH: 7.0) and varying concentrations of NaCl in the solution (ranging from 0 to 800 mM). **Figure 5B** demonstrates that by increasing the concentrations of NaCl from 0-800 mM, the release rate of NADH was significantly increased from $5 \pm 2.4\%$ to $94 \pm 5.3\%$, respectively. These results indicate that heightened ionic strength results in diminished stability of NADH molecules bound within 3A-PEI crystals due to the increased competition from anion binding into the crystals.

**A strategy to release NADH from 3A-PEI crystals by mechanical stirring**

There have been several strategies developed to achieve molecular release by using the responsiveness of polymers to dynamic mechanical environments. Several studies have been used mechanical force as a stimulus for the molecular release from the polymer matrix, self-assembled polymer micelles, or phospholipid liposomes induced by mechanical perturbation [28-31]. A promising study conducted by Jen *et al.* (2016) showed that a fluorescent mechanophore that is created through a Michael addition reaction and stabilized by a protonated amine through electrostatic interactions can be reversibly released via external mechanical force[32]. In another similar study, Imato *et al.* (2020) reported that the local folding of fluorescent pyrene and neighboring naphthalene diimide linked polymer film via chargetransfer, which can be released via mechanical stretching[33]. In sufficiently soft materials such as polyurethanes, it was shown that pyrene was able to rebind and refolded by the polymer upon relaxation, indicating reversible behavior[34]. However, in amorphous polycaprolactone, it was irreversible probably due to strain-induced ordering and crystallization of the polymer matrix[33]. Intriguing advances in polymer technology have resulted in the creation of mechanocatalysts with outstanding responsiveness when subjected to mechanical stirring[35]. Although few mechanically activated catalysts have been reported, most of them are metal-ligand coordination complexes that are incorporated into a polymer backbone [36, 37]. A good example is ruthenium- silver-N-heterocyclic carbene complexes that can be activated by extensional forces due to their separation. Copper- silver-N-heterocyclic carbene complexes are also reported as promising catalyst that is controllable via ultrasound technique[38].

These encouraging findings prompted us to explore the possibility of regulating the release and binding of cofactor molecules from 3A-PEI crystals by applying mechanical stirring. To explore this hypothesis, we initially examined the stability of 3A-PEI-NADH crystals under various levels of orbital shaking (ranging from 0 to 2000 RPM) in NaPB buffer (10 mM, pH:7.0) using the Eppendorf ThermoMixer® C. As depicted in **Fig. 6A,** the release rate of NADH molecules from 3A-PEI crystals were remarkably increased from $5 \pm 2.4\%$ in the absence of orbital shaking to over $83 \pm 2.1\%$ at 2000 rpm. These release rates are considered as apparent NADH release rates due to the possibility of NADH rebinding during the centrifugation process before NADH quantification. It is suggested that applying mechanical shaking not only accelerates the competition of anions in the buffer with NADH molecules for 3A-PEI binding, but also it contributes to the dissociation of the NADH molecules within the 3A-PEI crystals via the shearing force.

To understand whether this strategy can be applied to other cofactors, we also investigated the release of other phosphorylated cofactors from 3A-PEI crystals. As shown in **Fig. 6B,** more than 80% of bound $NAD^+$, $NADP^+$, NADPH , $FADH_2$, CoA, and TPP cofactors within the crystals can be released into the solution after applying of orbital shaking (1000 rpm) for 2h at 25°C. However, only approximately $40 \pm 1\%$ and $65 \pm 3\%$ of PLP and riboflavin cofactors, respectively, could be released under the same conditions due to their higher binding affinity. These findings indicate that mechanical shaking can effectively release the bound cofactors within the 3A-PEI crystals, making them available for subsequent use by the catalyst in bioprocessing applications.

The kinetic study of NADH binding to 3A-PEI crystals indicated that when the orbital shaking speed increased from 0-2000 rpm, the maximum NADH binding capacity ($B_{max}$) of 3A-PEI crystals was decreased from $540 \pm 5.9$ to $152 \pm 4.8$ nmol/mg while the $K_d$ value (equilibrium dissociation constant), exhibited an increase from $111 \pm 0.6$ to $565 \pm 6.1$ $\mu$M, respectively (**Table 2**). These findings indicate that the majority of NADH molecules became dissociated under increased mechanical stirring. To determine the relative force needed for the release of NADH molecules from the 3A-PEI crystals, the following formula was used:

$$\text{Relative Force} = (\text{RPM})^2 \times 1.118 \times 10^{-5} \times r$$

where the relative centrifugal force (RCF) is the radial force generated by the spinning rotor. r is rotation distance (1.5 mm), and RPM is considered as speed of rotation per minute.

As shown in **Fig. 6C,** by raising the relative force from 0 to 1.8 mN, 50% of bound NADH molecules to 3A-PEI crystals were released into the solution. This relative force is indicative of minimum force required to release 50% of bound NADH molecules from 3APEI crystals.

The elimination of mechanical stirring is a trigger to NADH molecules to rebind into 3APEI crystals. As shown in **Fig. 6D,** after releasing NADH molecules from 3A-PEI crystals by using mechanical stirring, when the mechanical stirring removed, NADH molecules can be stabilized via electrostatic interactions into 3AP-EI crystals at low temperature. This strategy offers a simple dynamic for regulation of NADH binding and release in the reaction, ensuring continuous supply of free cofactor for the reaction under mechanical stirring, and subsequent recycling by elimination of mechanical stirring at low temperature. The rechargeability of the 3A-PEI crystals shown in **Fig. 6E** demonstrates that NADH can be replenished within the crystals even if all bound NADH molecules removed. Therefore, the crystals retain their usability and ability to be employed effectively.

**Table 2. The kinetic parameters of NADH binding into 3A-PEI crystals at different mechanical shaking.**

| RPM | $K_d$ ($\mu$M) | $B_{max}$ (nmol/mg) |
|---|---|---|
| 0 | 111$\pm$ 0.6 | 540$\pm$ 5.9 |
| 400 | 178 $\pm$ 1.4 | 495$\pm$ 2.3 |
| 800 | 263$\pm$ 0.9 | 445$\pm$ 3.1 |
| 1200 | 476$\pm$ 1.4 | 265$\pm$ 2.6 |
| 1600 | 555$\pm$ 4.8 | 180$\pm$3.9 |
| 2000 | 565$\pm$ 6.3 | 152$\pm$4.8 |

The results indicate that by rising the mechanical stirring, the $B_{max}$ value was significantly decreased (almost three-fold), while the $K_d$ value rose to approximately five times. This data represents the importance of mechanical stirring on NADH binding or release from 3APEI crystals.

**The importance of support for PEI modification**

To explore the significance of support for PEI modification for NADH binding, three types of PEI-modified silica beads were provided, including PEI-SB1 (1 $\mu$m size with a 4nm pore size), PEI-SB2 (1 $\mu$m size with a 10 nm pore size), and PEIS-B3 (150 nm size with a 4 nm pore size). Each type of bead was prepared with an equal amount of anchored PEI (52 $\pm$5 mg PEI/ g of silica beads). Then, the NADH binding capacity of the beads was assessed. The binding of NADH molecules into PEI-SB were analyzed with the fluorescence emissions of the bound NADH molecules by using confocal microscopy. PEI labeled with Alexa488 is shown successfully anchored into SB beads. This observation supports the successful modification of PEI polymers into SB to facilitate the absorption and capture of NADH.

Interestingly, when comparing beads with the same size but different pore sizes (PEI-SB1 and PEI-SB2), it was found that PEI-SB1 exhibited approximately two-fold higher NADH binding capacity (60$\pm$1.3 $\mu$mol per g) compared to PEI-SB2 (**Fig. 7A**). These data show that the increase in pore size leads to a reduced surface area of the silica beads, limiting the availability of binding sites for the interaction between NADH and PEI [39, 40]. Furthermore, the smaller pore size provides a more confined space for NADH molecules, facilitating a stronger interaction with PEI, however, the larger pore size creates a more open and less restricted environment, potentially losing NADH molecules after binding[41, 42]. Additionally, when comparing beads with the same pore size but different bead sizes (PEI-SB1 and PEI-SB2), it was observed that smaller beads exhibited a remarkably higher capacity for NADH immobilization (approximately a 1.9-fold increase) (114$\pm$0.9 $\mu$mol/g) (**Fig. 7A**). It is suggested that smaller beads have a higher surface area-to-volume ratio, providing more available binding sites for the interaction between NADH and PEI, resulting in a higher immobilization capacity [43-45].

To investigate the impact of PEI concentration on the NADH binding capacity of silica beads (SB), we labeled the SBs with varying amounts of PEI polymers. **Fig.7B** shows that increasing the quantity of PEI (0-60$\pm$0.6 mg PEI/ g of SB) linked to the beads resulted in a significant increase in the amount of NADH bound from 0 to 105$\pm$1.2 $\mu$mol per g. This indicates a positive correlation between the quantity of NADH bound and the number of cross-linked PEI macromolecules.

However, it is worth noting that a further increase in the amount of PEI can lead to a decrease in the NADH binding capacity. These findings indicate that the increased concentration of bound PEI on the SB leads to a greater availability of binding sites for NADH. This enhanced availability allows for more binding interactions between NADH molecules and the anchored PEI, resulting in a larger quantity of NADH being bound. However, beyond a certain point (specifically, when the amount of PEI-SB1 reaches more than 60 mg per g of the beads), further increasing the amount of PEI can decrease the capacity for NADH binding (**Fig. 7B**). This could be due to factors such as overcrowding of the PEI molecules, decreased accessible volume for NADH binding or possible conformational changes that limit the efficiency of binding.

Based on the provided data, it was observed that 3A crystals, when anchored to PEI ($12 \pm 0.3$ mg per g), exhibit a maximum binding capacity of $540 \pm 1.2$ $\mu$mol per g Cry3Aa-PEI crystals, which is much higher than the PEI modified SB beads. However, it is important to note that the density of SB beads and 3A crystals are different. The results obtained using the pycnometer method to measure the density of both 3A-PEI crystals and PEI-SBs indicate that 3A-PEI crystals have a lower density, approximately 60%, in comparison to the beads. Although PEI-SB beads have higher density than 3A-PEI crystals, it is evident that 3A-PEI crystals can achieve approximately a five-fold higher NADH binding capacity. It is proposed that the distribution and arrangement of PEI polymers in solvent channels may affected on this NADH binding capacity.

**Recyclability of 3APEI-NADH +3AFL mixture**

To explore the feasibility of 3A-PEI crystals in facilitating binding and release within enzymatic reactions, it is crucial to develop biocatalysts that can effectively utilize NADH for fine compound biosynthesis and regenerate NADH molecules. Our research group has previously utilized Cry3Aa technology to immobilize the enzyme through entrapment or genetically fusion approaches [12, 13, 46]. According to the crystallization of Cry3Aa fusion proteins in *Bt,* we assumed that co-expression of diverse mixtures of Cry3Aa fusions may contribute for comparable intermolecular interaction, yielding co-immobilized and co-localized proteins in a crystal. Therefore, to assess the feasibility of Cry3Aa fusion crystals for co-immobilization and co-localization of different proteins, we constructed pHT315-Cry3Aa-GFP-RBS-Cry3Aa-mCherry plasmid, where each Cry3Aa fusion protein had its independent RBS. Then, it was used for co-expression of Cry3Aa-GFP/Cry3Aa-mCherry crystals in *Bt.* The distribution and localization of GFP and mCherry fluorescent reporter proteins were evaluated by employing fluorescence microscopy. The co-localization of these fluorescent reporter proteins within the same crystals are evident by the dominance of the combined signals (yellowish color) from the two green (GFP) and red (mCherry) due to their close proximity. The image analysis indicated that the co-expressed crystals revealed a high Pearson correlation coefficient of 0.956, indicating a strong association between the distribution and intensity of the corresponding fluorescent signals. These findings indicate that Cry3Aa has the potential to be used as an effective platform for the direct co-immobilization of multifunctional enzymes.

In the present study, Cry3Aa-FDH/Cry3Aa-LDH (3AFL) crystals were expressed and produced in *Bt.* They were then used to catalyze the conversion of trimethylpyruvate (TMP) into *L-tert* Leucine (L-*tert* leu) by using ammonia and NADH as cofactor. These crystals can regenerate NADH molecules by oxidation of formic acid into $CO_2$ as shown in **Figure 8.**

Although the NADH regeneration system in 3AFL crystals eliminates the need for continuous NADH addition, the requirements for NADH replenishment in each reaction cycle pose a significant challenge for practical applications. To address this limitation, we utilized the capacity of 3APEI crystals to effectively minimize the costs associated with NADH supplementation. As shown in **Figure 9,** it is indicated that the 3AFL crystals can maintain a 100% conversion rate for over 15 cycles by adding free NADH in each cycle. The turnover numbers (TNN) of NADH and crystals were calculated to be 44 and 9271. However, the 3AFL+3APEI-NADH mixture demonstrates that the conversion rate can remain at 100% for over 6 reaction cycles without requiring further NADH addition, except for the first cycle (1 $\mu$mol of NADH). A decline in production rate to 70% at the 7th cycle suggested NADH lixiviation due to buffer ionic effect that is inevitable in this system. Therefore, 0.3 $\mu$mol of NADH was added to the mixture to sustain the highest conversion rate. This recharging step enabled the reaction to proceed for an additional 6 cycles without addition of NADH. A subsequent 30% decline in the crystal activity at the 14th cycle necessitated the recharge of 0.3 $\mu$mol to continue the reaction until the activity reduced to less than 65% at the 21st cycle. The TNN of NADH increased to 284, while the TNN of the crystals stayed at 8409. These results indicate that the capacity of 3APEI crystals in preserving NADH molecules over the reaction is more than 6 times greater than that of the control. Without the recharging steps, this number could reach over 659, but the conversion rate would drop to less than 50% after 12th cycle. The dual system of 3AFL+3APEI-NADH primarily requires mechanical stirring for catalytic reactions, as demonstrated by the release of NADH molecules during shaking. The NADH rebinding step at the end of each reaction cycle is crucial for the reattachment of released NADH molecules to the 3APEI crystals. In addition, in the absence of mechanical stirring, the reaction rate of 3AFL crystals was decreased approximately five-folds compared to when mechanical stirring is applied. It is understood that the use of mechanical stirring can accelerate reaction rates as well as reduce the reaction time and the number of catalysts required for highest conversion rate. Furthermore, this system allows for free access to NADH molecules through mechanical stirring, which is advantageous compared to co-immobilization systems where NADH molecules are usually restricted. Interestingly, as ammonia formate serves as the substrate in this system, its consumption leads to a decrease in the pH of the reaction from 8.0 to 7.75 $\pm$ 0.05. This decrease in pH contributes for NADH rebinding at the end of the reaction due to increased protonation of the PEI polymer. The assessment of NADH binding in the reaction buffer before and after the reaction indicated that the NADH binding rate is increased almost 30% when the reaction finished. This can be attributed due to the depletion of ammonia formate buffer, which reduce the buffering ionic effect, and slight acidification of the reaction at the end, which enhances the positive charge in the PEI polymer, facilitating NADH rebinding.

DISCUSSION

Immobilization of cofactor-dependent enzymes is an important technique in making fine chemicals because it permits enzymes bound to solids to improve reaction control and keep heterogeneous biocatalysts stable for long-term use[47]. However, the high cost of certain cofactors, such as NADH, is a significant obstacle to the implementation of these biocatalysts, which hinders their widespread application in industry[9]. One possible strategy to address this issue is the co-immobilization of enzymes and cofactors within porous carriers in close proximity. This approach has limitations, however, due to the internal diffusion of cofactors, which can hinder the productivity of the biocatalysts[48]. Studies have shown that enzymes exhibit reduced activity toward reversible or irreversible tethered cofactors on solid materials, highlighting the challenges associated with this methodology[49-51]. Furthermore, the design, and chemical construction of carriers can be complicate and costly, as it requires even and close localization of cofactors and enzymes inside the porous structure. This involves strict regulation of the intraparticle enzyme distribution by controlling the immobilization rates through adjustment of the chemical nature of the enzyme/cofactor-carrier interface. Ensuring the diffusion of cofactors within the pores and the complete utilization of the three-dimensional (3D) surface of carriers while simultaneously achieving rapid immobilization poses a significant challenge[26, 52, 53]. This challenge is amplified by the involvement of various spatiotemporal phenomena at multiple scales, ranging from the nanoscale to the macroscale, such as structural distortions of enzymes, limited substrate access, and inadequate reactor settings like insufficient agitation, thereby reducing catalytic efficiency[54, 55]. Several studies have explored the use of porous carriers with modified dimensions, chemical nature, and structures to improve the localization and effectiveness of cofactor and enzyme co-immobilization[53, 56, 57]. The use of hydrophobic porous materials, like hydrophobic porous methacrylate beads, however, still presents challenges related to the internal transport of cofactors[53]. Although PEI-modified hydrophilic porous carriers display enhanced association and dissociation equilibrium of phosphorylated cofactors, improving the access of cofactors to the active sites, the use of this system requires careful handling due to the instability of the complex in aqueous solutions[53]. In a study, phosphorylated cofactors ($NAD^+$, $FAD^+$, and PLP) and enzymes were successfully immobilized onto PEI-activated agarose microbeads. This method resulted in a catalyst with similar activity toward both soluble and co-immobilized $NAD^+$, which could be used up to four reaction cycles in batch mode without requiring additional $NAD^+$. In a flow reactor, they achieved a total turnover number (TTN) of $NAD^+$ 85 after 107h [9]. However, they faced challenges concerning the rapid loss of $NAD^+$ during the washing process, which limited the practicality of their approach. In another study, the same strategy was used to coimmobilized thermophilic (S)-3-hydroxybutyryl-CoA dehydrogenase from *Thermus thermophilus HB27* (Tt27-HBDH) and NADH PEI modified 6BCL agarose to perform the asymmetric reduction of ethyl acetoacetate (EAA). By increasing the amount of enzyme loading (32.3 mg enzyme/g of support), they achieved good TTNs of 145 and 5223 for NADH and Tt27-HBDH respectively, after 10 reaction cycles[58]. In another strategy, cofactors being covalently immobilized to polymeric particles, while enzymes are immobilized separately from the cofactor. The collision between these particles facilitates interaction between cofactor and enzymes, enabling desired biotransformation reactions. This catalytic system has been tested for the enzymatic production of methanol from carbon dioxide, which has been explored for greenhouse gas fixation and renewable fuel production. Efficient regeneration and reuse of cofactors are crucial for capitalizing on the potentials of enzymatic biotransformation of $CO_2$. However, this system represented low yield of product after five cycles of recycling due to the limited availability of cofactor NADH. The reactions using free cofactor for each reuse maintain a constant production rate[49].

Inspired by nature, this study utilized 3A crystals, which possess self-crystallization properties, to serve as a scaffold for enzymes and cofactors. The introduction of the 3A scaffold as a natural support offers different advantages, including low production cost, easy modification, elimination of complicated support synthesis requirements, non-toxic properties, and biocompatibility. By *in vivo* immobilization of NAD(H) dehydrogenase (FDH) within 3A crystals, an efficient NADH regeneration system was established, along with NADH-dependent catalysts (LDH or ADH) capable of utilizing this cofactor within the same crystal for the asymmetric reduction of alcohols or ketones. The system demonstrated high stability for batch mode reactions under mechanical agitation for nearly 20 days, achieving the highest conversion rate. The continuous demand for NADH replenishment was addressed by the introduction of 3APEI-NADH crystals, which generated a highly stable complex. The data represented that even after more than twenty washing cycles with different buffers (NaPB, PBS, HEPES or ammonia formate), over 50% of bound NADH was retained in the crystals, highlighting the high capacity of these crystals for NADH retention. This capacity ($540 \pm 5.9 \mu mol/g$) exceeded that of other reported systems, such as PEI-modified mesoporous agarose (18 $\mu$mol/g)[9] and PEI-modified hydrophilic porous 6% agarose beads (200 $\mu$mol/g)[53], with minimal use of PEI polymer (12 $\pm$ 0.3 mg /g of support). This difference can likely be attributed to the unique orientation and distribution of functional carboxyl groups (Asp and Glu residues) within the solvent channels, which facilitate PEI labeling and provide highly ordered cationized solvent channels. The study presented an innovative strategy using mechanical agitation as a trigger for releasing and rebinding of NADH molecules in NAD(H) dependent systems. This approach allows for the release of NADH when it is required for catalytic reactions by applying mechanical stirring, and the subsequent rebinding of cofactors is achieved by eliminating the mechanical stirring. This straightforward approach offers an efficient mean for controlling the availability of NADH in biocatalytic reactions, which can improve the outcome as well as retention of NADH molecules for next cycles. The kinetic study exploring the binding and release of NADH from 3APEI crystals under different mechanical stirring revealed that the $B_{max}$ value is significantly reduced as the mechanical stirring increased, while the $K_d$ value elevated, indicating the disassociation of NADH molecules from the crystals under mechanical stirring. The subsequent rebinding of NADH molecules into the 3APEI crystals upon the removal of mechanical stirring represents an intriguing mechano-responsivity phenomenon. The applicability of this method was tested for 3AFL crystals for efficient production of L-*tert* Leu with minimal amount of NADH. The study displayed the requirement of mechanical agitation for efficient diffusion of either substrate or cofactor molecules into the crystals, ultimately enhancing the production rate. Considering that we have also demonstrated the efficient binding and release of other phosphorylated cofactors by 3A-PEI crystals, this simple and no-fuss approach can be applicable to other cofactor-dependent catalytic systems.

CONCLUSION

This study presents an efficient strategy for controlling the accessibility of NADH molecules in aqueous environments, allowing them to participate in NAD(H) dependent reactions through the application of mechanical stirring. This approach enables 3AFL and 3AFFA joined 3APEI-NADH dual systems to catalyze the biosynthesis of chiral compounds such as L-*tert* Leu and S-EHB for multiple cycles with minimal amount of NADH. The recyclability and rechargeability of this dual system ensure its long-term usability for continuous reactions.

REFERENCES

1. Roche, J., et al., NADH regenerated using immobilized FDH in a continuously supplied reactor - Application to l-lactate synthesis. Chemical Engineering Journal, 2014. 239: p. 216-225.

2. Saba, T., et al., NADH Regeneration: A Case Study of Pt-Catalyzed NAD+ Reduction with H2. ACS Catalysis, 2021. 11(1): p. 283-289.

3. Ali, I., B. Soomro, and S. Omanovic, Electrochemical regeneration of NADH on a glassy carbon electrode surface: The influence of electrolysis potential. Electrochemistry Communications, 2011. 13(6): p. 562-565.

4. Wang, X., et al., Cofactor NAD(P)H Regeneration Inspired by Heterogeneous Pathways. Chem, 2017. 2(5): p. 621-654.

5. Velasco-Lozano, S., A.I. Benítez-Mateos, and F. López-Gallego, Co-immobilized Phosphorylated Cofactors and Enzymes as Self-Sufficient Heterogeneous Biocatalysts for Chemical Processes. Angew Chem Int Ed Engl, 2017. 56(3): p. 771-775.

6. Ji, X., et al., Tethering of Nicotinamide Adenine Dinucleotide Inside Hollow Nanofibers for High-Yield Synthesis of Methanol from Carbon Dioxide Catalyzed by Coencapsulated Multienzymes. ACS Nano, 2015. 9(4): p. 4600-4610.

7. Li, P., et al., Hierarchically Engineered Mesoporous Metal-Organic Frameworks toward Cell-free Immobilized Enzyme Systems. Chem, 2018. 4(5): p. 1022-1034.

8. Liang, J., et al., Hierarchically Porous Biocatalytic MOF Microreactor as a Versatile Platform towards Enhanced Multienzyme and Cofactor-Dependent Biocatalysis. 2021. 60(10): p. 5421-5428.

9. Velasco-Lozano, S., A.I. Benítez-Mateos, and F. López-Gallego, Co-immobilized Phosphorylated Cofactors and Enzymes as Self-Sufficient Heterogeneous Biocatalysts for Chemical Processes. 2017. 56(3): p. 771-775.

10. Fu, J., et al., Multi-enzyme complexes on DNA scaffolds capable of substrate channelling with an artificial swinging arm. Nat Nanotechnol, 2014. 9(7): p. 531-6.

11. Hartley, C.J., et al., Engineered enzymes that retain and regenerate their cofactors enable continuous-flow biocatalysis. Nature Catalysis, 2019. 2(11): p. 1006-1015.

12. Heater, B.S., et al., Directed evolution of a genetically encoded immobilized lipase for the efficient production of biodiesel from waste cooking oil. Biotechnol Biofuels, 2019. 12: p. 165.

13. Heater, B.S., et al., In Vivo Enzyme Entrapment in a Protein Crystal. Journal of the American Chemical Society, 2020. 142(22): p. 9879-9883.

14. Emsley, P. and K. Cowtan, Coot: model-building tools for molecular graphics. Acta Crystallogr D Biol Crystallogr, 2004. 60(Pt 12 Pt 1): p. 2126-32.

15. Yang, Z., M.M.M. Lee, and M.K. Chan, Efficient intracellular delivery of p53 protein by engineered protein crystals restores tumor suppressing function in vivo. Biomaterials, 2021. 271: p. 120759.

16. Mounsef, J.R., et al., A simple method for the separation of Bacillus thuringiensis spores and crystals. J Microbiol Methods, 2014. 107: p. 147-9.

17. Ungaro, F., et al., Spectrophotometric determination of polyethylenimine in the presence of an oligonucleotide for the characterization of controlled release formulations. Journal of Pharmaceutical and Biomedical Analysis, 2003. 31(1): p. 143-149.

18. Wen, T., et al., A facile, sensitive, and rapid spectrophotometric method for copper(II) ion detection in aqueous media using polyethyleneimine. Arabian Journal of Chemistry, 2017. 10: p. S1680-S1685.

19. Otwinowski, Z. and W. Minor, Processing of X-ray diffraction data collected in oscillation mode. Methods Enzymol, 1997. 276: p. 307-26.

20. McCoy, A.J., et al., Phaser crystallographic software. J Appl Crystallogr, 2007. 40(Pt 4): p. 658-674.

21. Winn, M.D., An overview of the CCP4 project in protein crystallography: an example of a collaborative project. J Synchrotron Radiat, 2003. 10(Pt 1): p. 23-5.

22. Murshudov, GN., et al., REFMAC5 for the refinement of macromolecular crystal structures. Acta Crystallogr D Biol Crystallogr, 2011. 67(Pt 4): p. 355-67.

23. Liebschner, D., et al., Macromolecular structure determination using X-rays, neutrons and electrons: recent developments in Phenix. Acta Crystallogr D Struct Biol, 2019. 75(Pt 10): p. 861-877.

24. Goscianska, J., A. Olejnik, and I. Nowak, APTES-functionalized mesoporous silica as a vehicle for antipyrine - adsorption and release studies. Colloids and Surfaces A: Physicochemical and Engineering Aspects, 2017. 533: p. 187-196.

25. Bourkaib, M.C., et al., APTES modified SBA15 and meso-macro silica materials for the immobilization of aminoacylases from Streptomyces ambofaciens. Microporous and Mesoporous Materials, 2021. 323: p. 111226.

26. Nair, M.S., et al., Cry Protein Crystals: A Novel Platform for Protein Delivery. PLOS ONE, 2015. 10(6): p. e0127669.

27. Semenova, A., et al., Copper-Binding Properties of Polyethylenimine-Silica Nanocomposite Particles. Langmuir, 2022. 38(34): p. 10585-10600.

28. Lee, K.Y, M.C. Peters, and D.J. Mooney, Controlled Drug Delivery from Polymers by Mechanical Signals. 2001. 13(11): p. 837-839.

29. Lin, H.-Y and J.L. Thomas, PEG-Lipids and Oligo(ethylene glycol) Surfactants Enhance the Ultrasonic Permeabilizability of Liposomes. Langmuir, 2003. 19(4): p. 1098-1105.

30. Küng, R., et al., Mechanochemical Release of Non-Covalently Bound Guests from a Polymer-Decorated Supramolecular Cage. 2021. 60(24): p. 13626-13630.

31. White, S.R., et al., Autonomic healing of polymer composites. Nature, 2001. 409(6822): p. 794-7.

32. Li, Z.a., et al., Highly Sensitive Built-In Strain Sensors for Polymer Composites: Fluorescence Turn-On Response through Mechanochemical Activation. 2016. 28(31): p. 6592-6597.

33. Imato, K., et al., Fluorescent supramolecular mechanophores based on charge-transfer interactions. Chemical Communications, 2020. 56(57): p. 7937-7940.

34. Aerts, A., et al., Pyranine Based Ion-Paired Complex as a Mechanophore in Polyurethanes. 2021. 42(1): p. 2000476.

35. Michael, P. and W.H. Binder, A Mechanochemically Triggered "Click" Catalyst. 2015. 54(47): p. 13918-13922.

36. Groote, R., et al., Unfolding and Mechanochemical Scission of Supramolecular Polymers Containing a Metal-Ligand Coordination Bond. Macromolecules, 2011. 44(23): p. 9187-9195.

37. Piermattei, A., S. Karthikeyan, and R.P. Sijbesma, Activating catalysts with mechanical force. Nature Chemistry, 2009. 1(2): p. 133-137.

38. Michael, P., S.K. Sheidaee Mehr, and W.H. Binder, Synthesis and characterization of polymer linked copper(I) bis(N-heterocyclic carbene) mechanocatalysts. 2017. 55(23): p. 3893-3907.

39. Shan, C.a., et al., Effects of nano-pore system characteristics on CH4 adsorption capacity in anthracite. Frontiers of Earth Science, 2019. 13(1): p. 75-91.

40. Liu, W., et al., Effect of Pore Size Distribution and Amination on Adsorption Capacities of Polymeric Adsorbents. Molecules, 2021. 26(17).

41. Suresh Kumar, P., et al., Effect of pore size distribution and particle size of porous metal oxides on phosphate adsorption capacity and kinetics. Chemical Engineering Journal, 2019. 358: p. 160-169.

42. Ferrati, S., et al., Leveraging nanochannels for universal, zero-order drug delivery in vivo. Journal of Controlled Release, 2013. 172(3): p. 1011-1019.

43. Zhang, B., et al., New insights into the effects of porosity, pore length, pore shape and pore alignment on drug release from extrusionbased additive manufactured pharmaceuticals. Additive Manufacturing, 2021. 46: p. 102196.

44. Gao, Y., et al., Effect of pore size of three-dimensionally ordered macroporous chitosan-silica matrix on solubility, drug release, and oral bioavailability of loaded-nimodipine. Drug Dev Ind Pharm, 2016. 42(3): p. 464-72.

45. Li, J., et al., Effects of pore size on in vitro and in vivo anticancer efficacies of mesoporous silica nanoparticles. RSC Advances, 2018. 8(43): p. 24633-24640.

46. Sun, Q., et al., Cry3Aa*SpyCatcher Fusion Crystals Produced in Bacteria as Scaffolds for Multienzyme Coimmobilization. Bioconjugate Chemistry, 2022. 33(2): p. 386-396.

47. Sheldon, R.A. and S. van Pelt, Enzyme immobilisation in biocatalysis: why, what and how. Chem Soc Rev, 2013. 42(15): p. 6223-35.

48. Rocha-Martín, J., et al., Rational Co-Immobilization of Bi-Enzyme Cascades on Porous Supports and their Applications in Bio-Redox Reactions with In Situ Recycling of Soluble Cofactors. 2012. 4(9): p. 1279-1288.

49. El-Zahab, B., D. Donnelly, and P. Wang, Particle-tethered NADH for production of methanol from CO(2) catalyzed by coimmobilized enzymes. Biotechnol Bioeng, 2008. 99(3): p. 508-14.

50. DiCosimo, R., et al., Industrial use of immobilized enzymes. Chem Soc Rev, 2013. 42(15): p. 6437-74.

51. Ji, X., et al., Enabling multi-enzyme biocatalysis using coaxial-electrospun hollow nanofibers: redesign of artificial cells. J Mater Chem B, 2014. 2(2): p. 181-190.

52. Bolivar, J.M., et al., Modulation of the distribution of small proteins within porous matrixes by smart-control of the immobilization rate. J Biotechnol, 2011. 155(4): p. 412-20.

53. Benítez-Mateos, A.I., et al., Design of the Enzyme-Carrier Interface to Overcome the O2 and NADH Mass Transfer Limitations of an Immobilized Flavin Oxidase. ACS Applied Materials & Interfaces, 2020. 12(50): p. 56027-56038.

54. Cantone, S., et al., Efficient immobilisation of industrial biocatalysts: criteria and constraints for the selection of organic polymeric carriers and immobilisation methods. Chemical Society Reviews, 2013. 42(15): p. 6262-6276.

55. Liese, A. and L. Hilterhaus, Evaluation of immobilized enzymes for industrial applications. Chemical Society Reviews, 2013. 42(15): p. 6236-6249.

56. Zhou, N., et al., Self-sufficient biocatalysts constructed using chitin-based microspheres. Chemical Engineering Journal, 2023. 459: p. 141660.

57. Wang, Z., et al., Durable cofactor immobilization in sol-gel bio-composite thin films for reagentless biosensors and bioreactors using dehydrogenases. Biosensors and Bioelectronics, 2012. 32(1): p. 111-117.

58. Orrego, A.H., et al., Self-sufficient asymmetric reduction of β-ketoesters catalysed by a novel and robust thermophilic alcohol dehydrogenase co-immobilised with NADH. Catal Sci Technol, 2021. 11(9): p. 3217-3230.

## Example 2. A Genetically Encoded Multienzyme Particle for Biosynthesis of Putrescine from L-Arginine

Immobilized enzyme biocatalysts have wide industrial applications, but the multiple steps involved in their preparation significantly increase the time, effort and cost to produce them. Our group has previously developed a platform for the direct immobilization of enzymes based on the crystal-forming Cry3Aa protein. The application of this platform has been demonstrated for the direct immobilization of individual enzymes, with molecular size limitation of 50 kDa. However, the biosynthesis of a large number of compounds is achieved by a cascade reaction of multiple enzymes of various sizes. Herein, we report the use of a larger Cry homolog, Cry1Ab, for the *in vivo* co-immobilization of two enzymes involved in the biosynthesis of putrescine - human arginase I (hArg, 35 kDa) and ornithine decarboxylase (ODC, 79 kDa). We show that Cry1Ab-mediated immobilization enhanced the thermostability of these enzymes, allowing for the efficient and recyclable conversion of arginine to putrescine - yielding 30 mM putrescine per cycle at nearly 100% conversion in 30 min in the first seven cycles, which can be directly converted to the thermostable polyamide nylon-4,6.

Further advancement of this system was accomplished by modifying the genetically immobilized ODCmut-Cry1Ab with PEI polymer, which enables the recycling of the cofactor PLP, and eliminates the need for its exogenous supplementation at each cycle. As a result, the PEI-modified ODCmut-Cry1Ab particles were able to achieve a 24-fold increase in TTN of PLP.

INTRODUCTION

Multienzyme cascade reaction systems possess enormous potential for a wide range of biomedical and chemical applications[1] - particularly for industrial applications where the chemical syntheses takes place under harsh and environmentally harmful conditions.[2] In this regard, enzymes not only offer a much greener solution but also a more sustainable and economical production means, given their intrinsic specificity and catalytic nature, resulting in less toxic and unwanted side products.[3,4] More importantly, the coupling of multiple enzymes in cascades in a confined microenvironment, such as co-immobilization of enzymes onto a single support, not only enhances total reaction efficiency by bringing active sites spatially close together thus leading to increased local concentration of intermediate substrates,[5-7] but also by enabling the use of reactions that involve unstable intermediates, since they can be readily turned over by the subsequent enzyme in the enzymatic pathway.[6,8]

Putrescine is a polyamine found in many organisms and its biosynthesis can occur via several different enzymatic pathways.[9-11] Commercially, putrescine has many industrial applications, including as a precursor for the production of polyamide polymers,[12] such as nylon-4,6, which is produced by the polycondensation of putrescine and adipic acid,[13] and is commonly used in the production of biomedical instruments and textiles as well as automobile components.[14,15] Most putrescine used today, however, is chemically produced by the hydrogenation of succinonitrile, which is neither sustainable nor environmental-friendly, due to its use of non-renewable propene as raw material and its required *in situ* production of the toxic intermediate, hydrogen cyanide.[16,17] In an effort to minimize the negative impact on environment, the biosynthesis of putrescine has been extensively explored, with nearly all of the focus on microbial engineering, with *E. coli* being the most commonly employed system. In many of these whole cell systems, extensive genetic engineering was performed, to overexpress the enzymes associated with two key putrescine synthetic pathways - (1) the ornithine decarboxylase (ODC) pathway that utilizes ODC to directly convert L-ornithine to putrescine, and (2) the arginine decarboxylase (ADC) pathway that employs ADC and agmatinase to convert L-arginine to putrescine.[13,16,18-23] Additional genetic modifications, such as deletion of putrescine degradation pathways and disruption of competing pathways of the precursor L-ornithine utilization, have also been carried out in order to enhance the production of putrescine.[16,18-20,23,24] Nevertheless, the amounts produced are generally low, in part because high concentrations of putrescine are toxic to the organisms. [16,23]

One strategy that bypasses the limitations of the aforementioned approaches is the cell-free biosynthesis of putrescine using immobilized enzymes. In industry, enzyme immobilization is commonly used to achieve higher thermal, mechanical, and solvent stability, as well as better recyclability.[4,25-30] Furthermore, the enhancement of enzyme selectivity towards non-natural substrates may occur by the immobilization of enzymes, which may induce a conformational change that consequently alters the interaction between the substrate and the active site of the enzyme.[27,28] However, the cost of purification of the free enzyme and its subsequent immobilization limits its general use.[4,25,31]

Our group has developed a novel enzyme immobilization platform based on Cry proteins, a class of crystal-forming proteins that naturally form crystals during expression in the bacterium *Bacillus thuringiensis* (*Bt*).[32-34] We subsequently demonstrated that the fusion of lipases to Cry3Aa, a member of the Cry protein family, did not disrupt its crystal-forming ability and resulted in the production of immobilized lipases with high activity and high thermostability, thus enabling their use for the recyclable production of biodiesel.[35,36] Notably, this approach abrogates the need for column purification of the target enzyme and subsequent enzyme immobilization on solid supports, and reduces these steps to simple purification of the fusion protein crystals by density gradient centrifugation.

Recently, we have further developed this system for the co-immobilization of enzymes. In the proof-of-concept study in which we genetically fused Cry3Aa protein with SpyCatcher to produce Cry3Aa-SpyCatcher crystals, we demonstrated the successful co-immobilization of multiple soluble enzyme-SpyTag proteins onto these crystals and ultimately their enhanced thermal stability and recyclability using the menaquinone biosynthetic enzymes as a model system.[37] While the co-immobilized crystals had higher reaction rates and were more thermostable than their free enzyme counterparts, the additional steps for the purification of the enzyme-SpyTag were required, and the efficiency of the SpyTag-SpyCatcher conjugation was limited by the size of the target protein.[37] The latter being consistent with our observation thus far, that the maximum size of subunits that have been successfully fused to Cry3Aa protein to produce active fusion crystals *in vivo* was less than 50 kDa.[35,38,39]

As one approach to directly immobilize larger enzymes, we decided to switch from Cry3Aa to different crystal protein, Cry1Ab, as the immobilization platform. While Cry1Ab contains the same three domain core (the N-terminal $\alpha$-helical domain, the variable domain and the conserved domain) as Cry3Aa, it is much larger (130-140 kDa) due to the presence of a C-terminal protoxin crystallization domain.[40] Further motivation comes from the fact that previous studies have shown that Cry1Ab expressed in *E. coli* could form nicely shaped particles,[41,42] while those of Cry3Aa were irregular aggregates.[35] Given that *E. coli* is the most widely studied bacteria, we surmised that its utilization for biocatalyst production would enhance the general applicability of our immobilization technology. Herein, we describe our effort in the development of an *in vivo* multienzyme immobilization approach for the production of immobilized multienzyme biocatalysts in one step. We reported the successful co-immobilization of two key enzymes in the putrescine synthetic pathway, hArg and ODC, to Cry1Ab resulting in the generation of a biocatalytic system (**Figure 10**) that enables cell-free synthesis of putrescine in high yield. We further demonstrated that the putrescine produced *in situ* could be directly used for the synthesis of nylon-4,6, a heat resistant polyamide used in multiple commercial applications.[43]

MATERIALS AND METHODS

**Construction of Expression Plasmids**

To construct the plasmids for the expression of ODC and hArg, the *speC* gene (Accession Number: NP_417440.4) encoding ODC and the *hArg* gene (Accession Number: NM_000045.4) were each amplified by PCR and inserted into a pET28 vector via Gibson Assembly (NEB). The resulting recombinant plasmids were transformed into *E. coli* XL10. All positive colonies were verified by DNA sequencing (BGI).

The plasmids for the expression of Cry3Aa fusions and Cry1Ab fusions were prepared by inserting the amplified *speC, hArg, gfp* (Accession Number: OQ870305.1) or *mCherry* gene (Accession Number: MK160997.1) into a pHT315-Cry3Aa vector or pET28-Cry1Ab vector, respectively.

The double mutants (I163T/E165T) of ODC and ODC-Cry1Ab were generated by introducing the corresponding mutations into pET28-ODC and pET28-ODC-Cry1Ab via site-directed mutagenesis using the mutagenic primers listed in **Table 3.** The resultant plasmids were digested with *DpnI* and then transformed into *E. coli* XL10 as described above.

**Table 3.** Primers for construction of expression plasmids.

| Primer name | Primer sequence |
|---|---|
| pET28-Ndel-Arg-Fwd: | CCGCGCGGCAGCCATATGAGCGCCAAGTCCAGAAC CATAG (SEQ ID NO: 25) |
| Arg-Xhol-pET28-Rev: | GTGGTGGTGCTCGAGTTACTTAGGTGGGTTAAGGTA GTCAATAGGC (SEQ ID NO: 26) |
| pET28-Ndel-ODC-Fwd: | CCGCGCGGCAGCCATATGAAATCAATGAATATTGCC GCCAG (SEQ ID NO: 27) |
| ODC-Xhol-pET28-Rev: | GTGGTGGTGCTCGAGTTACTTCAACACATAACCGTAC AACC (SEQ ID NO: 28) |
| Arg-Nhel-Cry1Ab-Rev | CGGATTGTTATCCATGCTAGCCTTAGGTGGGTTAAGG TAGTCAATAGGC (SEQ ID NO: 29) |
| ODC-Nhel-Cry1Ab-Rev | CGGATTGTTATCCATGCTAGCCTTCAACACATAACCG TACAACC (SEQ ID NO: 30) |
| Cry1Ab-Xhol-<u>RBS</u>-ODC-Fwd: | GAAGAATAATGACTCGAG<u>AAGGAG</u>ATGAAATCAATGA ATATTGCCGCCAG (SEQ ID NO: 31) |
| Cry1Ab-Xhol-pET28-Rev: | GTGGTGGTGCTCGAGTCATTATTCTTCCATGAGGAGT AGTTCCACGC (SEQ ID NO: 32) |
| pET28-Ndel-GFP-Fwd | CCGCGCGGCAGCCATATGAGTAAAGGAGAAGAACTT TTCACTGG (SEQ ID NO: 33) |
| GFP-Nhel-Cry1Ab-Rev | CGGATTGTTATCCATGCTAGCTTTGTATAGTTCATCC ATGCCATGTGTAATCC (SEQ ID NO: 34) |
| pET28-Ndel-mCherry-Fwd | CCGCGCGGCAGCCATATGGTGAGCAAGGGCGAGGA GGATAAC (SEQ ID NO: 35) |
| mCherry-Nhel-Cry1Ab-Rev | CGGATTGTTATCCATGCTAGCCTTGTACAGCTCGTCC ATGCCG (SEQ ID NO: 36) |
| Cry1Ab-Xhol-<u>RBS</u>-mCherry-Fwd | GAAGAATAATGACTCGAG<u>AAGGAG</u>ATGGTGAGCAAG GGCGAGGAGGATAAC (SEQ ID NO: 37) |
| ODC I163T/E165T Fwd | TAAAATTGGGCGATCTGCTTACCCATACCGGATCGG CGAAAGATGC (SEQ ID NO: 38) |
| ODC I163T/E165T Rev | GCATCTTTCGCCGATCCGGTATGGGTAAGCAGATCG CCCAATTTTA (SEQ ID NO: 39) |

To construct the plasmid for co-expression of GFP-Cry1Ab and mCherry-Cry1Ab, the *mCherry-crylAb* gene was amplified with primers containing the ribosome binding site (RBS) (AAGGAG) and then inserted into the linearized pET28-GFP-Cry1Ab to produce the plasmid pET28-GFP-Cry1Ab-RBS-mCherry-Cry1Ab. Similarly, for co-expression of hArg-

Cry1Ab and ODC$_{mut}$-Cry1Ab, the *speC$_{mut}$-cry1Ab* was amplified with primers containing the RBS (AAGGAG) and then inserted into the linearized pET28-hArg-Cry1Ab to produce the plasmid pET28-hArg-Cry1Ab-RBS-ODC$_{mut}$-Cry1Ab.

All primers used for gene cloning and the amino acid sequences of proteins used in this study were listed in **Table 3** and sequence listing.

**Expression and Purification of Cry3Aa Fusion Crystals**

For production of Cry3Aa-ODC crystals, the pHT315-Cry3Aa-ODC plasmid was transformed into *Bt* 407G cells by electroporation and the transformed cells were cultured at 25°C for 72 h. Cell pellets were lysed with lysozyme in aqueous solution overnight followed by sonication and washed with NaCl and ddH$_2$O. The expression and purity of the Cry3Aa-ODC crystals were verified by SDS-PAGE. Multiple independent trials for the expression and purification of Cry3Aa-ODC crystals at the same conditions had been conducted, and no significant variations were observed in terms of the expression level and purity.

**Expression and Purification of Cry1Ab Fusion Particles**

Expression of individual Cry1Ab fusion, such as GFP-Cry1Ab or mCherry-Cry1Ab, as well as co-expression of Cry1Ab fusion pairs, such as GFP-Cry1Ab and mCherry-Cry1Ab ([GFP-Cry1Ab/mCherry-Cry1Ab]) or hArg-Cry1Ab and ODCmut-Cry1Ab ([hArg-Cry1Ab/ ODC$_{mut}$-Cry1Ab]), was achieved by transforming their encoding plasmids into *E. coli* BL21(DE3) by heat shock and the resultant colonies were inoculated into 500 mL Terrific Broth (TB, IBI Scientific) supplemented with 4 mL glycerol and 50 μg/mL kanamycin. Cells were then grown at 37°C until the OD$_{600}$ reached 0.6-0.8, at which point, 2 mM Isopropyl β-D-1-thiogalactopyranoside (IPTG, IBI Scientific) was added and the cells were further cultured for 48 h at 30°C. The cell pellets were resuspended in lysis buffer (50 mM Tris-HCl, 50 mM EDTA, 15% sucrose, pH 8.0) and incubated with lysozyme overnight, followed by sonication and centrifugation the next day. The particles collected were washed with Crystal Wash I solution (0.5 M NaCl, 2 % Triton X-100), followed by 0.5 M NaCl and ddH$_2$O. The expression and purity of the target fusion protein particles were confirmed by SDS-PAGE. Multiple independent trials for the expression and purification of Cry1Ab fusion particles at the same conditions had been conducted, and no significant variations were observed in terms of their expression level and purity.

**Expression and Purification of ODC and hArg Free Enzymes**

For the expression of free enzymes, the corresponding plasmids were transformed into *E. coli* BL21(DE3) via heat shock. The resultant colonies were cultured in 500 mL Lennox Broth (LB, IBI Scientific) with 50 μg/mL kanamycin at 37°C until the OD$_{600}$ reached 0.6-0.8. Then 0.2 mM IPTG was added to induce the expression of soluble enzymes at 18°C for 20 h. Cell pellets were resuspended in buffer containing 20 mM Tris (pH 7.5), 300 mM NaCl, 1 mM benzamidine hydrochloride (TCI Chemicals) and 1 mM phenylmethylsulfonyl fluoride (PMSF, Cayman Chemical) and lysed by sonication. The cell lysates were clarified by centrifugation and the resultant supernatants were filtered and applied to a nickel resin column (BioRad) for affinity purification. The protein of interest was eluted with 250 mM imidazole and the purity of the eluted fractions was analyzed by SDS-PAGE.

**Quantification of Enzymes in Cry1Ab Immobilized Particles**

All protein concentrations were obtained using the Bradford protein assay (BioRad).[44] To quantify the amount of enzymes in the Cry1Ab fusion particles, the total protein concentration (mg/mL) of the particle was converted to its molar concentration by dividing the corresponding combined molecular weight of the enzyme and Cry1Ab. For the fusion particle, the molar concentration of the enzyme component was equal to that of the total particle. Meanwhile, the mass/volume concentration (mg/mL) of enzyme component could be calculated by multiple the molar concentration with its molecular weight.

**Confocal Microscopy**

The fluorescence images of the co-expressed [GFP-Cry1Ab/mCherry-Cry1Ab] particles resuspended in ddH$_2$O as well as the mixture of individually expressed GFP-Cry1Ab particles and mCherry-Cry1Ab particles were acquired using a Leica TCS SP8 Confocal Microscope. The fluorescence intensity profiles and Pearson correlation coefficient values were generated using ImageJ software.

**hArg Activity Assay**

The activity of hArg was determined based on the production of urea following the method of Rahmatullah *et al.*[45] with slight modifications. brief, 1 $\mu$M of hArg-Cry1Ab fusion particle (0.165 mg/mL) or hArg enzyme (0.035 mg/mL) was added to a reaction solution containing 30 mM L-arginine dissolved in 50 mM HEPES (pH 8.0), 0.1 mM $Mn^{2+}$ buffer and incubated at 40°C with shaking at 750 rpm for 10 min. The reaction was stopped by 2 M $HClO_4$ and the urea produced was measured using Diacetylmonoxime (DAMO) - thiosemicarbazide (TSC) reagent. The reaction between urea and DAMO-TSC reagent under boiling water for 5 min resulted in the formation of a pink-red colored product with an absorbance peak at 525 nm. The protein concentrations for all constructs used in the assay were determined using the Bradford protein method (BioRad), and the quantification of hArg in hArg-Cry1Ab fusion particle was carried out as described previously. All experiments were performed in triplicate. Multiple independent trials for the activity assay of freshly expressed and purified hArg-Cry1Ab fusion particles had been conducted, and no significant variations were observed in terms of the activity.

**ODC Activity Assay**

The activity of ODC was determined based on the L-ornithine converted following the ninhydrin method of Chinard *et al.*[46] The reaction solution containing 1 $\mu$M of Cry1Ab ODC fusion particles (0.21 mg/mL) or Cry3Aa ODC fusion crystals (0.152 mg/mL) or ODC enzyme (0.079 mg/mL) and 30 mM L-ornithine in 50 mM HEPES (pH 8.0), 0.1 mM PLP was incubated at 40°C with shaking at 750 rpm for 30 min. The reaction was stopped by the addition of 2 M $HClO_4$. The L-ornithine remaining was determined by further reacting the reaction solution with 20 mg/mL ninhydrin dissolved in 2-methoxyethanol at 90°C with shaking at 750 rpm for 30 min. The L-ornithine converted was obtained by subtracting the L-ornithine remaining from its input amount. Assuming a 1:1 reaction stoichiometry for the conversion of L-ornithine to putrescine, the amount of putrescine produced equals the L-ornithine converted. The protein concentrations for all constructs used in the assay were determined using the Bradford protein assay, and the quantification of ODC in Cry ODC fusion particle was carried out as described previously. All experiments were performed in triplicate. Multiple independent trials for the activity assay of freshly expressed and purified Cry1Ab ODC fusion particles had been conducted, and no significant variations were observed in terms of their activities.

**Quantitative Analysis of Putrescine Produced**

The amount of putrescine produced by the co-immobilized [hArg-Cry1Ab/$ODC_{mut}$-Cry1Ab] particles could be quantitated using two approaches, the indirect method and HPLC.

For the indirect method, the amount of putrescine was determined by the subtraction of the L-ornithine remaining at the end of the reaction, which could be conveniently measured by the ninhydrin method, from the total amount of urea produced at the first reaction step catalyzed by hArg, which could be measured by the DAMO-TSC method. Here, the assumption was based on the fact that the first reaction yielded 1:1 molar ratio of urea and L-ornithine. The calibration curves of urea and L-ornithine were obtained by plotting the absorbance at 525 nm and 515 nm respectively against its corresponding concentrations.

To verify the validity of using the indirect method to quantify the putrescine produced, a reaction solution containing 0.5 mg/mL co-immobilized particles and 30 mM L-arginine was incubated at 40°C for 20 min, and then analyzed by both the indirect method and HPLC.

For HPLC measurement, the supernatant of the resultant reaction solution was diluted 25-fold with 0.1 M HCl and then analyzed by HPLC using a SUPELCO RP-$C_{18}$ column (250 mm $\times$ 4.6 mm, 5 $\mu$m). Prior to injection, the sample was derivatized with o-phthalaldehyde (OPA). The OPA-derivatized product was detected by monitoring the fluorescence of its eluted band (excitation wavelength: 335 nm; emission wavelength: 440 nm).[47] The retention times of derivatized L-arginine, L-ornithine and putrescine were obtained by HPLC measurement of their corresponding standards. And the calibration curve of putrescine was obtained by plotting the peak area against its concentration and then used for quantification.

**Kinetic Studies**

0.1 mg/mL Cry1Ab-immobilized particles, 3.5 $\mu$g/mL hArg and 7.9 $\mu$g/mL of $ODC_{mut}$ were used for kinetic studies. The kinetic parameters of hArg enzyme, immobilized hArg-Cry1Ab particles, and co-immobilized [hArg-Cry1Ab/$ODC_{mut}$-Cry1Ab] particles were measured at 40°C using 0 - 500 mM L-arginine in 50 mM HEPES (pH 8.0), 0.1 mM $Mn^{2+}$ buffer as substrate (for the particles, the kinetic parameters are based on the concentration of hArg), while those of $ODC_{mut}$ enzyme, immobilized $ODC_{mut}$-Cry1Ab particles, and co-immobilized [hArg-Cry1Ab/$ODC_{mut}$-Cry1Ab] particles were determined at 40°C using 0-100 mM L-ornithine in 50 mM HEPES (pH 8.0), 0.1 mM PLP buffer as substrate (for the particles, the kinetic parameters are based on the concentration of $ODC_{mut}$). The urea produced was determined by the

DAMO-TSC method[45] as described previously. The production of putrescine was measured by HPLC. One enzyme activity unit is defined as the amount of the total particles or enzymes required to produce 1 $\mu$mol of urea (for hArg) or putrescine (for $ODC_{mut}$) per min at 40°C. The protein concentrations for all constructs used in the assay were determined using the Bradford protein assay (BioRad), and the quantification of hArg or $ODC_{mut}$ in the Cry 1Ab fusion particles was carried out as described previously. All experiments were performed in triplicate.

**Reaction Rates Comparison of Co-immobilized [hArg-Cry1Ab/$ODC_{mut}$-Cry1Ab] Particles, Mixture of hArg-Cry1Ab + ODCmut-Cry1Ab Particles and Mixture of hArg + $ODC_{mut}$ Enzymes**

The calibration curves (concentration against band intensity of SDS-PAGE) of individually immobilized hArg-Cry1Ab or $ODC_{mut}$-Cry1Ab particles were separately plotted to quantify the two proteins within the co-immobilized particles. Based on the same molar concentrations of free enzymes used, reaction of 30 mM L-arginine with either (1) 0.5 mg/mL co-immobilized [hArg-Cry1Ab/$ODC_{mut}$-Cry1Ab] particles, (2) a mixture of 0.27 mg/mL hArg-Cry1Ab and 0.23 mg/mL $ODC_{mut}$-Cry1Ab particles, or (3) a mixture of 0.057 mg/mL hArg and 0.087 mg/mL $ODC_{mut}$ enzymes were conducted at 40°C and 750 rpm. The reaction was sampled at specific time points (5, 10, 20, 30 and 60 min) to allow for the determination of the putrescine produced using indirect method as described previously. The concentration of the particles and free enzymes was measured using the Bradford protein assay. All experiments were performed in triplicate.

**Effect of Urea on the Activity of hArg and $ODC_{mut}$**

1 $\mu$M of hArg (0.035 mg/mL) or $ODC_{mut}$ (0.079 mg/mL) was incubated in different concentrations (0, 250, 500 and 1000 mM) of urea solution at 40°C with shaking at 750 rpm for 6 h. The residual activities were measured and compared as described previously. The protein concentrations were obtained using the Bradford protein assay. All experiments were performed in triplicate.

**Orbitrap Mass Spectrometry**

To confirm the identities of the two dominant bands obtained from SDS-PAGE of the co-expressed [hArg-Cry1Ab/$ODC_{mut}$-Cry1Ab] particles are indeed hArg-Cry1Ab and $ODC_{mut}$-Cry1Ab, the bands were separately excised from the Coomassie blue-stained gel and cut into tiny pieces. The gel pieces were destained and dehydrated, and then digested with trypsin. The digested samples were analyzed by Orbitrap Fusion Lumos Tribrid Mass Spectrometer (Thermo Fisher Scientific).

**Scanning Electron Microscopy (SEM)**

Samples were prepared by resuspending 20 $\mu$g of each particle in 1 mL ddH$_2$O, followed by sonication for 1 min. 1 $\mu$L of the sonicated sample was loaded to a copper stub and air-dried overnight. After that, the samples were coated with conductive Au/Pd using a S150B sputter coater (Edwards) before imaging on a Prisma E (Thermo Scientific) scanning electron microscope at 20 kV and at a working distance of 8.0 mm to 8.1 mm. Multiple independent trials for the SEM of freshly expressed and purified Cry1Ab-immobilized particles had been conducted, and no significant variations were observed in terms of their size and morphology.

**Dynamic Light Scattering (DLS)**

The size and dispersity of immobilized hArg-Cry1Ab particles, $ODC_{mut}$-Cry1Ab particles, and co-immobilized [hArg-Cry1Ab/$ODC_{mut}$-Cry1Ab] particles were measured using a Malvern Zetasizer Nano ZS90 at 25 °C. Samples were prepared by resuspending 50 $\mu$g of each particle in 2 mL of ddH$_2$O and then sonicating for 1 min before measurement. Multiple independent trials for the DLS of freshly expressed and purified Cry1Ab-immobilized particles had been conducted, and no significant variations were observed in terms of their size.

**Thermal Stability**

1 $\mu$M of hArg enzyme (0.035 mg/mL), hArg-Cry1Ab fusion particle (0.165 mg/mL), ODC or $ODC_{mut}$ enzyme (0.079 mg/mL), and ODC-Cry1Ab or ODCmut-Cry1Ab fusion particle (0.21 mg/mL) were used in thermal stability studies. For hArg-Cry1Ab fusion particles and hArg enzyme, they were separately incubated in a reaction buffer consisting of 50 mM HEPES (pH 8.0), 0.1 mM Mn$^{2+}$ at 50°C with shaking at 750 rpm for 1 h. Then aliquots of 100 $\mu$L of the reaction mixture were transferred to individual tubes and incubated at different temperatures with shaking at 750 rpm for 1 h and then cooled on ice before measuring the residual activity at 40°C as described previously. The residual activity at 50°C incubation was taken as 100%.

The thermal stability of ODC and $ODC_{mut}$ proteins as well as the corresponding Cry1Ab fusion particles were conducted in the same reaction buffer as that for the activity assays, and the procedure was similar to that described above for hArg and its Cry1Ab fusion counterparts, except here the residual activity at 30°C was taken as 100%.

The data was fitted to a sigmoidal curve and the $T_{50}$ values were calculated using GraphPad Prism software. $T_{50}$ is defined as the temperature at which 50% activity remains after 1 h incubation. The protein concentrations were measured using Bradford protein assay. All measurements were performed in triplicate.

**Optimization of Catalytic Conditions**

Optimization of the temperature and pH for the catalytic reaction was performed using 0.5 mg/mL co-immobilized [hArg-Cry1Ab/$ODC_{mut}$-Cry1Ab] particles and 30 mM L-arginine substrate and 10 min reaction time. To identify the optimal reaction temperature, the particles and substrates were resuspended in pH 8 buffer and subjected to a series of incubation temperatures for the indicated 10 min. The amount of putrescine produced was determined as described previously. The optimal pH was identified by performing the reaction in different pH buffers (pH 6: 0.1 M BIS-Tris buffer; pH 7-8: 0.1 M Tris buffer; pH 9: 0.1 M CAPSO buffer; pH 10: 0.1 M CAPS buffer) and incubated at 40°C for the indicated time.

To ascertain whether the pH impacts the solubility of the fusion protein particles and thereby affects their recyclability, 2 mg/mL co-immobilized particles were incubated in the same buffers as listed above in the pH optimization experiment, and an additional basic buffer: 0.1 M CAPS pH 11 known to dissolve Cry crystals[35] at 40°C for 24 h in a thermomixer shaking at 1500 rpm. At the end of incubation, the suspensions were centrifuged at 15,000 rpm for 5 min and the supernatant was transferred for subsequent protein concentration measurement by Bradford method.

All experiments were performed in triplicate.

**Recyclability of Co-immobilized Particles for the Conversion of L-Arginine to Putrescine**

The recyclability of the co-immobilized particles was evaluated by incubating 2 mg/mL [hArg-Cry1Ab/$ODC_{mut}$-Cry1Ab] particles with 30 mM L-arginine substrate in a reaction buffer containing 50 mM HEPES (pH 8.0), 0.1 mM $Mn^{2+}$, 0.1 mM PLP at 40°C for 30 min. At the end of the incubation, the reaction solution was centrifuged at 15,000 rpm for 5 min, and the supernatant was removed for subsequent testing of the putrescine produced as described previously. The pellet containing the [hArg-Cry1Ab/$ODC_{mut}$-Cry1Ab] particles was resuspended in fresh reaction buffer and then sonicated for 10 s. Then, fresh L-arginine was added to start the next reaction cycle. The concentration of the particles was measured using Bradford protein assay. All experiments were performed in triplicate.

**_In Situ_ Production of Nylon-4,6 from Putrescine**

To demonstrate the _in situ_ production of nylon-4,6, 4 mg/mL of co-immobilized [hArg-Cry1Ab/$ODC_{mut}$-Cry1Ab] particles were incubated with 500 mM of substrate L-arginine under optimal reaction conditions for 12 h to convert the L-arginine to putrescine. The supernatant was then mixed with NaOH and then reacted with adipoyl chloride in hexane. The nylon film was retrieved by continuously pulling it out in strands from the interface of the reaction mixture and then washed with 50 % MeOH and deionized water before vacuum dried overnight. The dehydrated nylon was grounded into pieces prior to analysis by FTIR.

**Fourier-transform Infrared Spectroscopy (FTIR)**

Samples were prepared by mixing the nylon pieces with potassium bromide (KBr) to make a KBr pellet for FTIR analysis. Infrared spectra were collected by recording the transmittance at different wavenumbers between 4000 and 400 $cm^{-1}$ on a Nicolet iS10 FTIR Spectrometer (Thermo Scientific).

RESULTS AND DISCUSSION

**Cry1Ab-mediated Co-immobilization of GFP and mCherry**

We had previously demonstrated that Cry3Aa-hArg crystals were active[48] and thus our initial plan was to co-immobilize hArg and ODC on Cry3Aa. However, full-length Cry3Aa-ODC crystals were found to be poorly expressed and inactive. We speculated that this was due to the large size of ODC making it difficult for Cry3Aa to form the stabilizing interactions present in Cry3Aa crystals.[32,49] Indeed, over the course of developing the Cry3Aa immobilization technology, we have observed the general trend that only proteins of less than 50 kDa could be successfully expressed as highly active Cry3Aa fusion crystals _in vivo_.[35,36,39,49] We thus sought another strategy to promote its immobilization.

Since Cry1Ab is much larger than Cry3Aa, we wondered whether it might be better suited for the immobilization of large proteins such as ODC with respect to preserving the enzyme activity. Moreover, we speculated that since Cry1Ab forms crystals in *Bt,* in the particles generated from Cry1Ab-fusions should have the propensity to form similar interactions, indicating that mixtures of Cry1Ab-fusions might lead to co-immobilized particles. Towards this end, the suitability of Cry1Ab as an immobilization platform for multiple proteins was first investigated. To facilitate the analysis of colocalization of distinct immobilized proteins, the fluorescent reporter proteins, GFP and mCherry, were chosen as the fusion partners to Cry1Ab to generate GFP-Cry1Ab and mCherry-Cry1Ab fusions respectively. Individually expressed GFP-Cry1Ab and mCherry-Cry1Ab particles as well as the co-expressed [GFP-Cry1Ab/mCherry-Cry1Ab] particles were produced in *E. coli* and characterized by confocal microscopy (**Figure 11A**). The colocalization of the two Cry1Ab-reporter proteins within the same particles was obvious for the co-expressed [GFP-Cry1Ab/mCherry-Cry1Ab] samples as evidenced by the abundant yellow punctate signals in the merged image as opposed to the separate green and red punctate dots shown in the merged image of the mixture of GFP-Cry1Ab and mCherry-Cry1Ab particles (**Figure 11A**). Moreover, the fluorescence intensity profiles of the co-expressed [GFP-Cry1Ab/mCherry-Cry1Ab] particles demonstrated a strong correlation between distribution and intensity of the two fluorescent signals (Pearson correlation coefficient = 0.874) whereas those of mixture of individually immobilized GFP-Cry1Ab and mCherry-Cry1Ab displayed distinct distribution patterns and unrelated intensities (Pearson correlation coefficient = 0.162) (**Figure 11B**).

## Production of Active ODC-Cry1Ab Particles

Having verified the feasibility of producing Cry1Ab-fusion particles in *E. coli* containing co-immobilized two proteins, GFP and mCherry, our focus shifted towards generating the relevant constructs involved in the biosynthesis pathway of putrescine from L-arginine (**Figure 10**). Given the problem observed with Cry3Aa-ODC, we prioritized investigating the activity of ODC fused with Cry1Ab. To ascertain whether the position of ODC in the Cry1Ab fusion would impact its enzymatic activity, ODC was genetically fused to the C- or N-terminus of Cry1Ab to produce Cry1Ab-ODC and ODC-Cry1Ab particles, respectively, and their activities were evaluated. The expression of Cry1Ab-ODC and ODC-Cry1Ab in *E. coli* BL21 (DE3) were confirmed by SDS-PAGE. Measurement of their ODC activities revealed that while Cry1Ab-ODC particles were inactive, ODC-Cry1Ab particles exhibited activity, albeit less than that of the free enzyme (**Figure 12A**). Thus N-terminal fusions of ODC to Cry1Ab were used in the subsequent experiments.

Previous studies had shown that ODC is the rate-limiting enzyme in the two-step enzymatic conversion of L-arginine to putrescine by hArg and ODC,[11,50] and the double mutant ODC (I163T/E165T) that exhibited 60-fold increase in catalytic efficiency was generated by Choi *et. al* to overcome this limitation.[11] We thus introduced similar mutations into ODC-Cry1Ab to produce $ODC_{mut}$-Cry1Ab, with the goal of attaining a more active particle. The expression of $ODC_{mut}$-Cry1Ab in *E. coli* BL21 (DE3) was verified by SDS-PAGE, and the activity of the isolated $ODC_{mut}$-Cry1Ab particles was evaluated and compared to that of ODC-Cry1Ab particles. As expected, the $ODC_{mut}$-Cry1Ab particles exhibited much improved conversion, showing a >2.5-fold enhancement (**Figure 12A**).

## Production of Active hArg-Cry1Ab Particles

We then proceeded to produce and characterize the partner enzyme hArg in the multistep reaction. hArg was genetically fused to the N-terminus of Cry1Ab, and hArg-Cry1Ab particles were successfully expressed based on SDS-PAGE. The resultant hArg-Cry1Ab particles were also determined to be active (**Figure 12C**).

## Production and Characterization of Co-immobilized [hArg-Cry1Ab/ODC$_{mu}$t-Cry1Ab] Particles

Having confirmed that the individually immobilized particles were active, we moved to generate the co-immobilized particles of $ODC_{mut}$-Cry1Ab and hArg-Cry1Ab. The co-expression of hArg-Cry1Ab and $ODC_{mut}$-Cry1Ab was verified by SDS-PAGE based on the two dominant bands corresponding to the molecular weights of hArg-Cry1Ab (165 kDa, lower band) and $ODC_{mut}$-Cry1Ab (210 kDa, upper band), which were subsequently excised and trypsin-digested for mass spectroscopic analysis. Their respective identities were confirmed with identified peptides covering >40% of the corresponding sequences.

The size and morphology of the immobilized particles were then investigated by DLS and SEM. The DLS data indicated that the average size of hArg-Cry1Ab, $ODC_{mut}$-Cry1Ab and [hArg-Cry1Ab/$ODC_{mut}$-Cry1Ab] particles were 573 nm, 902 nm, and 477 nm, respectively (**Figure 13A**). SEM images showed that the naturally immobilized particles possessed a spherical shape and exhibited uniform size distribution, which is an attribute important to biocatalytic reactions (**Figure 13B**).

## Activity of Co-immobilized [hArg-Cry1Ab/ODC$_{mut}$-Cry1Ab] Particles

The activity of the co-immobilized [hArg-Cry1Ab/ODC$_{mut}$-Cry1Ab] was then investigated based on their ability to produce putrescine and quantitated using indirect method as described in Methods and validated by HPLC. As shown in **Figure 14,** the co-immobilized [hArg-Cry1Ab/ODC$_{mut}$-Cry1Ab] was able to convert 30 mM of L-arginine substrate to 25.1 mM (based on the indirect method) or 26.1 mM (based on HPLC) of putrescine at 40°C in 20 min, corresponding to ~ 84% conversion. The agreement of the two results supports the use of the indirect approach for determining the putrescine produced for the subsequent experiments. Notably, both methods demonstrated that the [hArg-Cry1Ab/ODC$_{mut}$-Cry1Ab] particles are active and can promote the facile conversion of L-arginine to putrescine (**Figure 14**).

## Kinetic Studies of Cry1Ab-immobilized Particles and Free Enzymes

To better understand the influence of Cry1Ab-mediated co-immobilization on the activities of hArg and ODC$_{mut}$, their kinetic parameters were determined. The apparent $K_m$ (app$K_m$) values for the immobilized enzymes and their corresponding free enzyme counterparts displayed a close resemblance (**Table 4**), indicating that the immobilization did not significantly impact the substrate binding affinity to the enzymes. However, the $k_{cat}$ values for [hArg-Cry1Ab/ODC$_{mut}$-Cry1Ab] particles were found to be approximately 50% and 60% of those for free hArg and ODC$_{mut}$ (**Table 4**), respectively. This observation suggested that the enzyme activity was reduced, presumably due to conformational change induced by the immobilization process, or a slower rate of exchange of the substrate and products within the confines of the particle.

**Table 4.** Kinetic parameters of Cry1Ab-mediated immobilized enzymes and the corresponding free enzymes.

| Construct | Specific Activity (U/mg of total particles)* | Specific Activity (U/mg of enzyme)* | appKm (mM) | $k_{cat}$ (s$^{-1}$) | $k_{cat}$/appK$_m$ (mM$^{-1}$ s$^{-1}$) |
|---|---|---|---|---|---|
| hArg | - | 171.2 ± 2.9 | 20.0 ± 1.3 | 99.9 ± 1.7 | 5.0 ± 0.4 |
| hArg-Cry1Ab | 21.5 ± 0.3 | 101.4 ± 1.4 | 18.5 ± 0.3 | 59.7 ± 0.8 | 3.2 ± 0.1 |
| [hArg-Cry1Ab/ODC$_{mut}$-Cry1Ab] † | 12.3 ± 0.4 | 82.8 ± 2.7 | 22.1 ± 0.5 | 48.3 ± 1.6 | 2.2 ± 0.1 |
| ODC$_{mut}$ | - | 29.1 ± 1.3 | 2.7 ± 0.4 | 38.3 ± 1.7 | 14.5 ± 2.6 |
| ODC$_{mut}$-Cry1Ab | 3.4 ± 0.1 | 9.0 ± 0.3 | 2.7 ± 0.1 | 11.9 ± 0.4 | 4.4 ± 0.3 |
| [hArg-Cry1Ab/ODC$_{mut}$-Cry1Ab] ‡ | 4.0 ± 0.1 | 17.7 ± 0.4 | 4.1 ± 0.3 | 23.3 ± 0.6 | 5.7 ± 0.5 |

†The activity and kinetic parameters were based on the concentration of hArg within the co-immobilized particles. ‡The activity and kinetic parameters were based on the concentration of ODC$_{mut}$ within the co-immobilized particles. *1U is defined as the amount of the total particles or free enzyme required to produce 1 μmol of urea or putrescine per min at 40°C. 0.1 mg/mL Cry1Ab-immobilized particles, 3.5 μg/mL hArg and 7.9 μg/mL ODC$_{mut}$ were used for the kinetic studies. All protein concentrations were determined using the Bradford method. The quantitation of the amount of enzyme in the particles was carried out as described in **Materials and Methods.** The $k_{cat}$ values were derived by dividing the V$_{max}$ values with the corresponding concentrations of enzymes, assuming a steady state and excess of the substrate. All experiments were performed in triplicate. Data are presented as mean ± standard deviation.

## Reaction Rates of Co-immobilized Particles in Comparison with the Mixture of Individually Immobilized Particles and the Mixture of Free Enzymes

The previous confocal microscopy studies on [GFP-Cry1Ab/mCherry-Cry1Ab] particles (**Figure 11**) had shown that Cry1Ab-mediated co-immobilization resulted in co-localization of multiple proteins. By inference, the enzymes within the co-immobilized [hArg-Cry1Ab/ODC$_{mut}$-Cry1Ab] particles should have a similar distribution, which should bring their active sites into close proximity and in turn lead to improved reaction rates. To verify this conjecture, the activity of co-immobilized [hArg-Cry1Ab/ODC$_{mut}$-Cry1Ab] particles was compared to the activities of the mixture of individually immobilized hArg-Cry1Ab and ODC$_{mut}$-Cry1Ab particles, as well as the mixture of free hArg and ODC$_{mut}$ proteins. Since urea is known to inactivate enzymes at high concentration,[51,52] our first task was to determine whether urea produced by arginase in the first step of the cascade reaction would affect the reaction rates of the enzymes. At the tested concentrations ranging from 0 to 1 M, the activities of both free hArg and ODC$_{mut}$ were unaffected even when incubating at 1 M urea for 6 h.

Having ruled out urea as a factor affecting enzymatic activities for the conditions used, we proceeded to carry out the enzymatic conversion of L-arginine to putrescine. For the mixture of individually immobilized particles and the mixture of free enzymes, the molar ratio of 1.5:1 of hArg and $ODC_{mut}$ was used. This ratio was based on the stoichiometry of the [hArg-Cry1Ab/$ODC_{mut}$-Cry1Ab] particles derived from densitometric analysis of the band intensities of SDS-PAGE of co-expressed [hArg-Cry1Ab/$ODC_{mut}$-Cry1Ab] and the calibration curves of individually immobilized hArg-Cry1Ab and $ODC_{mut}$-Cry1Ab (concentrations against band intensities). Based on our previous work with Cry3Aa-mediated co-immobilized enzyme systems,[37] our expectation was that co-immobilized enzymes would exhibit a higher reaction rate due to the higher local substrate concentrations, which in this case was L-ornithine for the second enzyme $ODC_{mut}$. As expected, despite the slower rates of the individual reactions, after 20 minutes of reaction, the co-immobilized particles produced 1.25-fold more putrescine than the mixture of free hArg and $ODC_{mut}$, respectively (**Figure 15**). These data indicate that Cry1Ab-mediated co-immobilization of multienzymes leads to improved reaction rates of multistep reactions.

Our previous work on Cry3Aa-mediated co-immobilized enzyme systems had found that a mixture of individually immobilized particles was much slower than the co-immobilized enzyme system.[37] Similarly, compared to the co-immobilized [hArg-Cry1Ab/$ODC_{mut}$-Cry1Ab] particles, the individually immobilized hArg-Cry1Ab and $ODC_{mut}$-Cry1Ab particles exhibited lower activity - 2.5-fold less putrescine produced after 20 min (**Figure 15**). We speculate that this slower rate stems from the inefficient escape of the intermediate L-ornithine from the particles, which is in contrast to the mixture of hArg and $ODC_{mut}$ enzymes where the L-ornithine is free to move in solution, and the co-immobilized particles where the L-ornithine need only react within the same particle.

## Thermal Stability of Cry1Ab-immobilized Particles

Previous studies had shown that Cry3Aa-mediated immobilization could lead to increased thermostability,[35,37] we therefore investigated the influence of Cry1Ab protein on the stability of the enzymes. hArg is an $Mn^{2+}$- dependent metalloenzyme that catalyzes the hydrolysis of L-arginine to L-ornithine and urea,[53] and had been shown to coordinate strongly with $Mn^{2+}$ by incubating at 50°C for a specific period.[54] Thus, prior to studying the thermal stability of hArg and hArg-Cry1Ab, we first identified the incubation period needed for optimal integration of $Mn^{2+}$ into the enzymes by incubating the particles and free proteins with $Mn^{2+}$ at 50°C for different lengths of time and then determined their residual activities. The activities of both free hArg and its immobilized form reached the peak and remained almost unchanged after 60 minutes' incubation, indicating that $Mn^{2+}$ was strongly integrated into the enzyme. The thermal stability of hArg and hArg-Cry1Ab was then evaluated, by first incubating them in the $Mn^{2+}$-containing reaction buffer at 50°C for 60 min, followed by incubation at different temperatures for 1 h before measuring their residual activities. For the thermal stability studies of ODC and $ODC_{mut}$ as well as their corresponding Cry1Ab-immobilized particles, no such preincubation was required, and the enzymes were directly incubated at different temperatures.

The $T_{50}$ values obtained from the thermal stability studies for hArg-Cry1Ab, ODC-Cry1Ab and ODCmut-Cry1Ab were 70.68°C $\pm$ 0.05, 57.98°C $\pm$ 0.42 and 56.43°C $\pm$ 0.26, respectively (**Figure 16**). In contrast, the data for the free enzyme counterparts, hArg, ODC and $ODC_{mut}$, were 65.89°C $\pm$ 0.11, 51.81°C $\pm$ 1.86 and 48.85°C $\pm$ 0.91, respectively (**Figure 16**). These results indicate that the immobilized particles were more thermostable than their free enzyme counterparts, and that Cry1Ab-mediated immobilization led to enzyme stabilization - possibly due to the partial confinement of the protein within the particle hindering unfolding and aggregation. Meanwhile, it was observed that $ODC_{mut}$-Cry1Ab was less stable than hArg-Cry1Ab, with the stable range with minimal loss of activity being between 30°C and 40°C (**Figure 16B**). This indicates that for the recyclable use of the co-immobilized [hArg-Cry1Ab/$ODC_{mut}$-Cry1Ab] particles, it would be best to not exceed this temperature range.

## Optimization of Reaction Conditions

Determination of the optimal temperature for maximal activity of the co-immobilized [hArg-Cry1Ab/$ODC_{mut}$-Cry1Ab] particles indicated that 45°C gave the highest production of putrescine, but considering that the thermostable range of $ODC_{mut}$-Cry1Ab was between 30°C and 40°C, 40°C was selected as the optimal reaction temperature to prolong the lifetime of biocatalyst during recyclability studies. Investigations of the activity of the co-immobilized particles as a function of pH suggested that both pH 8 and pH 9 yielded the maximum production of putrescine. Given that Cry crystals, including Cry1Ab, have been shown to solubilize in alkaline pHs,[15] and that the recyclability of enzyme can be negatively impacted by enzyme loss including from solubilization,[4] the stability of Cry 1Ab was thus investigated by determining the amount of protein released into the supernatant when the co-immobilized enzymes were incubated at different pHs. In agreement with previous studies on the stability of most Cry crystals, the co-immobilized [hArg-Cry1Ab/$ODC_{mut}$-Cry1Ab] particles were stable to solubilization from pH 6.0 to pH 8.0. Collectively, these data indicate that pH 8 is the optimal pH at which the enzymes and the immobilization platform are minimally affected.

**Recyclability of Co-immobilized Particles**

The recyclability of a biocatalyst is one of the most important features in evaluating its usability for industrial application[55] given that its lifetime directly impacts the production costs. We thus tested the ability of 2 mg/mL of co-immobilized [hArg-Cry1Ab/ODC$_{mut}$-Cry1Ab] particles for the recyclable conversion of 30 mM L-arginine to putrescine under the optimized conditions (40°C and pH 8.0). A conversion rate of more than 80% was maintained even after nine reaction cycles (**Figure 17A**), resulting in a total amount of 321 mM putrescine produced, and a yield of 0.451 g/g L-arginine, across twelve cycles. By calculation, the TTN of ODC$_{mut}$ within the co-immobilized particles was 73,284. In this case, however, exogenous PLP was required in each cycle, which significantly increased the overall cost of the biosynthetic process. To make the cofactor PLP more cost-effective and reusable, the genetically immobilized ODC$_{mut}$-Cry1Ab was modified with positively charged PEI polymer, which allowed the phosphorylated PLP to be tethered and thus recycled. As a result, the PEI-ODC$_{mut}$-Cry1Ab-PLP particles were able to promote the recyclable biosynthesis of putrescine without the addition of the exogenous cofactor, with a TTN$_{PLP}$ of 1,736 after 25 cycles (**Figure 17B**). In contrast, without PEI modification towards ODC$_{mut}$-Cry1Ab particles, PLP was required in each cycle, with a TTN$_{PLP}$ of 71 only.

**Production of Putrescine and Its Use for Synthesis of Nylon-4,6**

The conversion of putrescine to nylon-4,6 is of significant interest due to its superior mechanical properties and thermal stability compared to other nylons, making it an attractive material for use in various industries.[14,56,57] Biobased putrescine would be an attractive starting material for nylon-4,6 production because it can offer sustainable and environmentally friendly alternatives to traditional petrochemical-based processes[17] - particularly if nylon-4,6 could be produced directly from the reaction solutions for putrescine biosynthesis. To this end, putrescine was biosynthesized in a single cycle reaction using 500 mM L-arginine as substrate. After 12 h, approximately 430 mM (37.9 g/L) of putrescine was produced, with a productivity of 3.16 g/L/h and an overall product yield of 0.435 g/g L-arginine. This performance was comparable to that reported for whole cells, as in the case of the *Bacillus amyloliquefaciens* engineered with a synthetic ODC pathway in the study by Li *et al.,* which produced 5.51 g/L putrescine after 48 h, with a productivity of 0.11 g/L/h and an overall product yield of 0.14 g/g xylose,[19] or the engineered *E. coli* cells overexpressing the enzymes in the ADC pathway in the study by Yang *et al.,* which attained a productivity of 8.56 g/L/h and an overall product yield of 0.41 g/g L-arginine after 2 h.[22] Of note is the use of high concentration of cofactor PLP in the latter case (1 mM versus 0.1 mM in our system).[22]

The resulting putrescine (approximately 430 mM) was retrieved after centrifugation of the reaction mixture and removal of the co-immobilized [hArg-Cry1Ab/ODC$_{mut}$-Cry1Ab] biocatalysts (which could be reused in subsequent reaction cycles), and directly mixed with adipoyl chloride to synthesize nylon-4,6 polymer. The resulting nylon was characterized by FTIR (**Figure 18**). Consistent with previous reports,[56,58] absorption peaks at 3300 cm$^{-1}$, 2940 cm$^{-1}$ and 2870 cm$^{-1}$, 1640 cm$^{-1}$ attributed to the stretching vibrations of N-H, C-H, C=O, respectively and the peak at 1540 cm$^{-1}$ corresponded to N-H deformation and C-N stretching (Amide II) were observed. These data support the synthesis of nylon-4,6 from reaction of adipoyl chloride with the putrescine directly produced from the [hArg-Cry1Ab/ODC$_{mut}$-Cry1Ab] biocatalyst.

CONCLUSIONS

We have developed a Cry1Ab-based platform and demonstrated its use for direct co-immobilization of hArg and ODC$_{mut}$ *in vivo.* Notably, this multienzyme catalyst exhibits enhanced thermostability and higher reaction rates than a mixture of its free enzyme counterparts and a mixture of the individually immobilized particles, and enables the recyclable biosynthesis of putrescine. Moreover, the reaction solutions produced containing putrescine can be directly used for the subsequent reaction with adipoyl chloride to produce nylon-4,6. As far as we know, this was the first demonstration of using cell-free method to biosynthesize putrescine from L-arginine, and both the productivity and the yield are comparable to the whole cell synthesis by engineered bacteria. It is noteworthy to mention that these Cry1Ab-mediated co-immobilized multienzyme particles can be cheaply and easily obtained by the co-expression of hArg-Cry1Ab and ODC$_{mut}$-Cry1Ab in *E. coli* cells and the subsequent isolation of the particles via sonication and centrifugation, with high yield (~ 100 mg of co-immobilized enzyme particles per L of TB medium). More broadly, these results highlight the potential use of Cry1Ab protein particles as a cost-efficient and robust platform to directly co-immobilize multienzymes involved in multi-step cascade reactions for productive biosynthesis of useful chemicals.

We further advanced our technology for the recycling of the expensive cofactor PLP by the cationization of ODC$_{mut}$-Cry1Ab with PEI polymer. We demonstrated that the self-sufficient PEI-ODC$_{mut}$-Cry1Ab-PLP could promote the biosynthesis of putrescine for many cycles without adding exogenous PLP, with a significant increase in TTN$_{PLP}$ (1,736 vs. 71 for the ODC$_{mut}$-Cry1Ab particles without PEI modification).

REFERENCES

1. Li, D.; Xiong, Q.; Liang, L.; Duan, H. Multienzyme nanoassemblies: from rational design to biomedical applications. Biomater. Sci. 2021, 9, 7323-7342

2. Bugada, L. F.; Smith, M. R.; Wen, F. Engineering spatially organized multienzyme assemblies for complex chemical transformation. ACS Catal. 2018, 8, 7898-7906

3. Adrio, J. L.; Demain, A. L. Microbial enzymes: tools for biotechnological processes. Biomolecules 2014, 4, 117-139

4. Sheldon, R. A.; Basso, A.; Brady, D. New frontiers in enzyme immobilisation: robust biocatalysts for a circular bio-based economy. Chem. Soc. Rev. 2021, 50, 5850-5862

5. Arana-Peña, S.; Carballares, D.; Morellon-Sterlling, R.; Berenguer-Murcia, Á.; Alcántara, A. R.; Rodrigues, R. C.; Fernandez-Lafuente, R. Enzyme co-immobilization: Always the biocatalyst designers' choice... or not? Biotechnol. Adv. 2021, 51, 107584

6. Santiago-Arcos, J.; Velasco-Lozano, S.; López-Gallego, F. Multienzyme coimmobilization on triheterofunctional supports. Biomacromolecules 2023, 24, 929-942

7. Zhang, G; Quin, M. B.; Schmidt-Dannert, C. Self-assembling protein scaffold system for easy in vitro coimmobilization of biocatalytic cascade enzymes. ACS Catal. 2018, 8, 5611-5620

8. Ricca, E.; Brucher, B.; Schrittwieser, J. H. Multi- enzymatic cascade reactions: overview and perspectives. Adv. Synth. Catal. 2011, 353, 2239-2262

9. Schneider, J.; Wendisch, V. F. Putrescine production by engineered Corynebacterium glutamicum. Appl. Microbiol. Biotechnol. 2010, 88, 859-868

10. Handa, A. K.; Fatima, T.; Mattoo, A. K. Polyamines: bio-molecules with diverse functions in plant and human health and disease. Front. Chem. 2018, 6

11. Choi, H.; Kyeong, H.-H.; Choi, J. M.; Kim, H.-S. Rational design of ornithine decarboxylase with high catalytic activity for the production of putrescine. Appl. Microbiol. Biotechnol. 2014, 98, 7483-7490

12. Chung, H.; Yang, J. E.; Ha, J. Y; Chae, T. U.; Shin, J. H.; Gustavsson, M.; Lee, S. Y. Bio-based production of monomers and polymers by metabolically engineered microorganisms. Curr. Opin. Biotechnol. 2015, 36, 73-84

13. Li, G H.; Huang, D. X.; Wang, L.; Deng, Y. Highly efficient whole-cell biosynthesis of putrescine by recombinant Escherichia coli. Biochem. Eng. J. 2021, 166

14. Roerdink, E.; Warnier, J. M. M. Preparation and properties of high molar mass nylon-4, 6: a new development in nylon polymers. Polymer 1985, 26, 1582-1588

15. Shakiba, M.; Rezvani, G; Khosravi, F.; Jouybar, S.; Bigham, A.; Zare, M.; Abdouss, M.; Moaref, R.; Ramakrishna, S. Nylon-A material introduction and overview for biomedical applications. Polym. Adv. Technol. 2021, 32, 16

16. Qian, Z. G; Xia, X. X.; Lee, S. Y. Metabolic engineering of Escherichia coli for the production of putrescine: a four carbon diamine. Biotechnol. Bioeng. 2009, 104, 651-662

17. Sanders, J.; Scott, E.; Weusthuis, R.; Mooibroek, H. Bio- refinery as the bio- inspired process to bulk chemicals. Macromol. Biosci. 2007, 7, 105-117

18. Kong, C.-C.; Wei, X.; Liu, G-L.; Chi, Z.-M.; Chi, Z. Metabolic engineering of Aureobasidium melanogenum for the overproduction of putrescine by improved L-ornithine biosynthesis. Microbiol. Res. 2022, 260, 127041

19. Li, L.; Zou, D.; Ji, A.; He, Y.; Liu, Y.; Deng, Y.; Chen, S.; Wei, X. Multilevel metabolic engineering of Bacillus amyloliquefaciens for production of the platform chemical putrescine from sustainable biomass hydrolysates. ACS Sustain. Chem. Eng. 2020, 8, 2147-2157

20. Thongbhubate, K.; Irie, K.; Sakai, Y; Itoh, A.; Suzuki, H. Improvement of putrescine production through the arginine decarboxylase pathway in Escherichia coli K-12. AMB Express 2021, 11

21. Hui, H.; Bai, Y.; Fan, T.; Zheng, X.; Cai, Y. Biosynthesis of putrescine from L-arginine using engineered Escherichia coli whole cells. Catalysts 2020, 10, 947

22. Yang, S. C.; Ting, W. W.; Ng, I. S. Effective whole cell biotransformation of arginine to a four-carbon diamine putrescine using engineered Escherichia coli. Biochem. Eng. J. 2022, 185

23. Yang, F. Y; Qiu, K. L.; Zhu, Y. C.; Zhang, X.; Yang, T. W.; Yi, G F.; Xu, M. J.; Rao, Z. M. Production of putrescine in metabolic engineering Corynebacterium crenatum by mixed sugar fermentation. ACS Sustain. Chem. Eng. 2022, 10, 14407-14416

24. Schneider, J.; Eberhardt, D.; Wendisch, V. F. Improving putrescine production by Corynebacterium glutamicum by fine-tuning ornithine transcarbamoylase activity using a plasmid addiction system. Appl. Microbiol. Biotechnol. 2012, 95, 169-178

25. Bolivar, J. M.; Woodley, J. M.; Fernandez-Lafuente, R. Is enzyme immobilization a mature discipline? Some critical considerations to capitalize on the benefits of immobilization. Chem. Soc. Rev. 2022

26. Rodrigues, R. C.; Berenguer-Murcia, Á.; Carballares, D.; Morellon-Sterling, R.; Fernandez-Lafuente, R. Stabilization of enzymes via immobilization: Multipoint covalent attachment and other stabilization strategies. Biotechnol. Adv. 2021, 52, 107821

27. Mateo, C.; Palomo, J. M.; Fernandez-Lorente, G; Guisan, J. M.; Fernandez-Lafuente, R. Improvement of enzyme activity, stability and selectivity via immobilization techniques. Enzyme Microb. Technol. 2007, 40, 1451-1463

28. Rodrigues, R. C.; Ortiz, C.; Berenguer-Murcia, Á.; Torres, R.; Fernández-Lafuente, R. Modifying enzyme activity and selectivity by immobilization. Chem. Soc. Rev. 2013, 42, 6290-6307

29. Guisan, J. M.; Fernandez-Lorente, G; Rocha-Martin, J.; Moreno-Gamero, D. Enzyme immobilization strategies for the design of robust and efficient biocatalysts. Curr. Opin. Green Sustain. Chem. 2022, 35, 100593

30. Maghraby, Y. R.; El-Shabasy, R. M.; Ibrahim, A. H.; Azzazy, H. M. E.-S. Enzyme immobilization technologies and industrial applications. ACS Omega 2023, 8, 5184-5196

31. Olcucu, G; Klaus, O.; Jaeger, K.-E.; Drepper, T.; Krauss, U. Emerging solutions for in vivo biocatalyst immobilization: tailor-made catalysts for industrial biocatalysis. ACS Sustain. Chem. Eng. 2021, 9, 8919-8945

32. Li, J.; Carroll, J.; Ellar, D. J. Crystal structure of insecticidal $\delta$-endotoxin from Bacillus thuringiensis at 2.5 Å resolution. Nature 1991, 353, 815-821

33. Schnepf, E.; Crickmore, N.; Van Rie, J.; Lereclus, D.; Baum, J.; Feitelson, J.; Zeigler, D. R.; Dean, D. Bacillus thuringiensis and its pesticidal crystal proteins. Microbiol. Mol. Biol. Rev. 1998, 62, 775-806

34. Loutfi, H.; Fayad, N.; Pellen, F.; Le Jeune, B.; Chakroun, M.; Benfarhat, D.; Lteif, R.; Kallassy, M.; Le Brun, G; Abboud, M. Morphological study of Bacillus thuringiensis crystals and spores. Appl. Sci. 2021, 11, 155

35. Heater, B. S.; Lee, M. M.; Chan, M. K. Direct production of a genetically-encoded immobilized biodiesel catalyst. Sci. Rep. 2018, 8, 1-10

36. Heater, B. S.; Chan, W. S.; Lee, M. M.; Chan, M. K. Directed evolution of a genetically encoded immobilized lipase for the efficient production of biodiesel from waste cooking oil. Biotechnol. Biofuels 2019, 12, 1-14

37. Sun, Q.; Heater, B. S.; Li, T. L.; Ye, W.; Guo, Z.; Chan, M. K. Cry3Aa* SpyCatcher fusion crystals produced in bacteria as scaffolds for multienzyme coimmobilization. Bioconjug. Chem. 2022, 33, 386-396

38. Nair, M. S.; Lee, M. M.; Bonnegarde-Bernard, A.; Wallace, J. A.; Dean, D. H.; Ostrowski, M. C.; Burry, R. W.; Boyaka, P. N.; Chan, M. K. Cry protein crystals: a novel platform for protein delivery. PLoS ONE 2015, 10, e0127669

39. Sun, Q.; Cheng, S. W.; Cheung, K.; Lee, M. M.; Chan, M. K. Cry protein crystal-immobilized metallothioneins for bioremediation of heavy metals from water. Crystals 2019, 9

40. Peña-Cardeña, A.; Grande, R.; Sánchez, J.; Tabashnik, B. E.; Bravo, A.; Soberón, M.; Gómez, I. The C-terminal protoxin region of Bacillus thuringiensis Cry1Ab toxin has a functional role in binding to GPI-anchored receptors in the insect midgut. J. Biol. Chem. 2018, 293, 20263-20272

41. Oeda, K.; Inouye, K.; Ibuchi, Y.; Oshie, K.; Shimizu, M.; Nakamura, K.; Nishioka, R.; Takada, Y.; Ohkawa, H. Formation of crystals of the insecticidal proteins of Bacillus thuringiensis subsp. aizawai IPL7 in Escherichia coli. J. Bacteriol. 1989, 171, 3568-3571

42. Lee, T. F. Study on Escherichia coli-expressed Cry1Ab towards the development of a targeted protein delivery system. Master's thesis, The Chinese University of Hong Kong, 2017.

43. Kudo, K.; Mochizuki, M.; Kiriyama, S.; Watanabe, M.; Hirami, M. Studies on the structure and properties of nylon 46 fiber. I. Dimensional stability. J. Appl. Polym. Sci. 1994, 52, 861-867

44. Bradford, M. M. A rapid and sensitive method for the quantitation of microgram quantities of protein utilizing the principle of protein-dye binding. Anal. Biochem. 1976, 72, 248-254

45. Rahmatullah, M.; Boyde, T. R. C. Improvements in the determination of urea using diacetyl monoxime; methods with and without deproteinisation. Clin. Chim. Acta 1980, 107, 3-9

46. Chinard, F. P. Photometric estimation of proline and ornithine. J. Biol. Chem. 1952, 199, 91-95

47. Pereira, V.; Pontes, M.; Câmara, J.; Marques, J. C. Simultaneous analysis of free amino acids and biogenic amines in honey and wine samples using in loop orthophthalaldeyde derivatization procedure. J. Chromatogr. A 2008, 1189, 435-443

48. Heater, B. S. Cry3Aa-inspired enzyme immobilization: from biocatalysis to therapeutics. Doctoral thesis, The Chinese University of Hong Kong, 2018.

49. Heater, B. S.; Yang, Z.; Lee, M. M.; Chan, M. K. In Vivo Enzyme Entrapment in a Protein Crystal. J. Am. Chem. Soc. 2020, 142, 9879-9883

50. Alarcón, R.; Orellana, M. S.; Neira, B.; Uribe, E.; Garcia, J. R.; Carvajal, N. Mutational analysis of substrate recognition by human arginase type I- agmatinase activity of the N130D variant. FEBS J. 2006, 273, 5625-5631

51. Mashino, T.; Fridovich, I. Effects of urea and trimethylamine-N-oxide on enzyme activity and stability. Arch. Biochem. Biophys. 1987, 258, 356-360

52. Chilaka, F. C.; Nwamba, C. O. Kinetic analysis of urea-inactivation of $\beta$-galactosidase in the presence of galactose. J. Enzyme Inhib. Med. Chem. 2008, 23, 7-15

53. Grobben, Y.; Uitdehaag, J. C. M.; Willemsen-Seegers, N.; Tabak, W. W. A.; de Man, J.; Buijsman, R. C.; Zaman, G J. R. Structural insights into human Arginase-1 pH dependence and its inhibition by the small molecule inhibitor CB-1158. J. Struct. Biol.: X 2020, 4

54. Stone, E. M.; Glazer, E. S.; Chantranupong, L.; Cherukuri, P.; Breece, R. M.; Tierney, D. L.; Curley, S. A.; Iverson, B. L.; Georgiou, G Replacing Mn2+ with Co2+ in human arginase I enhances cytotoxicity toward L-arginine auxotrophic cancer cell lines. ACS Chem. Biol. 2010, 5, 333-342

55. Basso, A.; Serban, S. Industrial applications of immobilized enzyme-A review. Mol. Catal. 2019, 479, 110607

56. Zhang, Z.; Huang, K.; Liu, Z. Synthesis of high molecular weight nylon 46 in supercritical carbon dioxide. Macromolecules 2011, 44, 820-825

57. Yamanobe, T.; Kurihara, Y.; Uehara, H.; Komoto, T. Structure and characterization of nylon 46. J. Mol. Struct. 2007, 829, 80-87

58. Cong, P.; Xiang, F.; Liu, X.; Li, T. Morphology and microstructure of polyamide 46 wear debris and transfer film: In relation to wear mechanisms. Wear 2008, 265, 1100-1105

**Example 3. A Simple Strategy for Co-immobilization of NADH-Dependent Enzymes and NADH for Efficient Biosynthesis in Continuous Flow Reactors**

The application of NADH-dependent enzymes in continuous flow reactors poses significant challenges due to the high cost of NADH and its inevitable loss during reactor operation. To overcome these limitations, we have developed a novel platform comprised of Cry3Aa-enzyme fusion particles modified with polyethylenimine (PEI) and demonstrate its application for two catalytic processes. In the first system, two NAD(H)-dependent enzymes, formate dehydrogenase (FDH) and leucine dehydrogenase (LDH), were genetically co-immobilized within Cry3Aa particles and then modified with PEI to facilitate NADH co-immobilization. To adapt the system for continuous flow reactors, PEI-modified particles were entrapped within agarose beads, loaded with NADH, and then used to catalyze L-tert leucine biosynthesis under continuous flow conditions for 30 days with minimal NADH recharging achieving LDH and NADH turnover numbers of 21,348 and 6,544, respectively. This platform was then validated for another NADH-dependent multienzyme system comprised of FDH and alcohol dehydrogenase (ADH) and used for the continuous conversion of ethyl acetoacetate to ethyl-(R)-3-hydroxybutyrate. ADH and NADH turnover numbers of 15,074 and 3,256 were obtained after 30 days. These examples illustrate the significant potential of this technology for facilitating NADH-dependent enzyme-mediated biosynthesis in continuous flow reactors.

METHODS AND RESULTS

NAD(H)-dependent enzymes are promising biocatalysts for the production of pharmaceutical precursors due to their high selectivity and enantiomeric specificity. However, their application has been limited due to the high cost of NADH and the necessity for its continuous regeneration and replenishment. Many strategies have been implemented to address these issues for continuous flow systems. Common solutions to achieve NADH regeneration include the use of coupling enzymes such as formate dehydrogenase (FDH),[1] while multiple strategies have been explored for NADH retention, including covalent binding to the support,[2, 3] entrapment in polymer materials,[4, 5] adsorption onto solid supports,[6] and NADH encapsulation in nanostructures.[7]

For industrial synthesis, continuous flow reactors offer multiple advantages improve efficiency and catalytic performance for high-throughput reactions including superior control over reaction conditions, easy scalability to larger scale reactors, and continuous product removal which minimizes product inhibition.[8] However, the implementation of NADH-dependent enzymes in such reactors faces significant challenges, most notably the need to maintain a continuous supply of NADH in the reactor.[9] Arguably one of the simplest strategies to address this issue for continuous flow systems involves co-immobilizing the NADH-dependent enzymes with NADH on solid supports coated with cationic polymers, such as polyethylenimine (PEI).[10, 11] This strategy relies on electrostatic interactions to bind the cofactor without altering its chemical structure, thereby maintaining its natural functionality. The free mobility of the cofactor enables seamless shuttling between the enzyme's active site and the immobilized support, thereby ensuring sustained enzymatic activity while preserving the enzyme's functionality.[12, 13] Moreover, the NADH cofactor can be replenished should it become lost or inactivated. Nevertheless, previous reported systems exhibited several limitations such as low NADH binding capacity, low total turnover number of NADH ($TTN_{NADH}$), unproven NADH rechargeability, and the need for labor-intensive enzyme purification prior to cofactor co-immobilization. For example, in the asymmetric reduction of acetophenone, co-immobilizing $NAD^+$, alcohol dehydrogenase (ADH), and formate dehydrogenase (FDH) onto PEI-coated agarose beads results in a low TTN of NADH ($TTN_{NADH}$: 85), operating for only 107 h.[14] Other systems, such as one used for the asymmetric reduction of trifluoro-acetaldehyde, exhibited a better TTN for NADH ($TTN_{NADH}$: 1,076), but the operation time was still only 120 h.[11] These limitations demonstrate the need and opportunity for new technologies for promoting NADH-dependent enzyme reactions in continuous flow systems.

Previously, we reported the production of *in vivo* immobilized Cry3Aa-enzyme fusion particles for the efficient and multicycle biosynthesis of L-*tert*-leucine using two distinct Cry3 Aa-particles in batch mode.[15] Here, one particle was comprised of two co-immobilized NADH-dependent enzymes - a Cry3 Aa-leucine dehydrogenase (3A-LDH) component which drives the L-*tert* Leucine (L-*tert* Leu) biosynthesis, and a Cry3Aa-formate dehydrogenase (3A-FDH) component which facilitates NADH regeneration. The second particle consisted of PEI-modified Cry3Aa (PEI-3A) crystals which could be used as a mechano-responsive NADH sponge for NADH recycling. Under high stirring, the NADH is released from the PEI-3A crystal where it can mix with the enzyme crystal and reactants to promote L-*tert* Leu biosynthesis. Between cycles, stirring is stopped, and the NADH binds to the insoluble PEI-3A crystals, allow for exchange of reactant solution and without loss of the NADH.

While this strategy is ideal for batch reactions, it is ineffective for continuous flow reactors due to the transition of NADH between the particles, which may lead to the cofactor passing out alongside the product. Therefore, we sought a way to make it suitable for continuous flow reactions. We hypothesized that one simple way to achieve our goal could be to modify the co-immobilized 3A-LDH/3A-FDH particles with PEI at a level that would enable the co-immobilization of NADH, without loss in enzyme activity. Our hypothesis was that this dual enzyme and NADH co-immobilization would permit the cofactor to migrate between enzymes in the microenvironment of the particle without being released to the aqueous media.

To test this hypothesis, *in vivo* co-immobilized 3A-FDH/3A-LDH particles were prepared as reported previously[15] and then modified with PEI. At low concentrations of EDC/NHS or PEI, the activity of 3A-FDH/3A-LDH particles remained almost unchanged. After PEI-modification, the particles maintained their ovoid shape and were of only slightly larger size - presumably due to labeling of some PEI on the surface of the particles (**Figure 19A-E**), the zeta potential shifted from -15 mV to + 40 mV (**Figure 19F**), strongly supporting its PEI-mediated cationization.

Confocal microscopy was used to monitor the attachment of dye-modified PEI to 3A-FDH/3A-LDH particles, while the co-immobilization of NADH with these particles was confirmed by taking advantage of the NADH's intrinsic fluorescence (**Figure 20A**). The NADH binding capacity of the PEI-3A-FDH/3A-LDH particles was $226 \pm 5$ $\mu$mol NADH per g of particle, while the particles without PEI retained only ~$4.2 \pm 0.3$ $\mu$mol NADH per g, as determined spectroscopically by measuring NADH absorbance at 340 nm.

The impact of PEI modification on the conversion efficiency of 3A-FDH/3A-LDH was determined for the production of L-*tert* Leu and showed that modification led to a small ~10% reduction in the product produced (**Figure 20B**). To gain further insight, the kinetic parameters of 3A-FDH/3A-LDH and PEI-3A-FDH/3A-LDH particles were determined and showed that the main impact of PEI modification was a slight increase in the $K_m$ values of both FDH and LDH, thus slightly reducing catalytic efficiencies. Overall, these data showed that PEI-modification had only a minimal impact on Cry3Aa-immobilized enzyme function.

To evaluate the ability of PEI-3A-FDH/3A-LDH particles to retain the NADH cofactor for use in catalysis, we tested its use in multicycle batch reactions under slow shaking (80 rpm) for the production of L-*tert* Leu. PEI-3A-FDH/3A-LDH-NADH particles were able to promote the complete conversion of substrate to product over 10 cycles without addition of exogenous NADH, while unmodified 3A-FDH/3A-LDH-NADH particles exhibited a significant (~80%) decrease in product yield after the first cycle (**Figure 20C**). These data demonstrated that PEI modification significantly enhances NADH retention in 3A-FDH/3A-LDH particles.

The next step was to optimize the use of PEI-3A-FDH/3A-LDH-NADH particles for continuous flow systems. Given the small size of the co-immobilized enzyme particles, two concerns were their retention in the column under flow conditions and their potential to induce clogging. To overcome these issues, we embedded the PEI-modified particles within 0.5% (w/v) agarose beads following procedures[16] reported to produce pores of less than 200 nm[17-19] that would thus be suitable for entrapping the micrometer sized PEI-3A-FDH/3A-LDH-NADH particles while allowing for moderate access of substrates and products. The produced beads were spherical with a 20 mm diameter (wet) (~2 mm diameter (air-dried)) with approximately $7.1 \pm 0.2$ mg of PEI-3A-FDH/3A-LDH particles per g of wet bead. Confocal microscopy of PEI-3A-FDH/3A-LDH-Alexa 568-agarose beads showed that the particles were evenly distributed within the agarose beads (**Figure 21**).

To confirm that the NADH binding ability of the particles remained intact after the entrapment in agarose beads, the presence of NADH molecules within the beads was examined by confocal microscopy, taking advantage of the cofactor's autofluorescence. The images showed that the NADH colocalized to the PEI-3A-FDH/3A-LDH particles within the agarose beads (**Figure 21**), indicating the effectiveness of this system for co-entrapping both the cofactor and enzyme particles.[14]

To assess the impact of entrapment of the particles in agarose beads on the activity of the co-immobilized enzymes, the production efficiency of the particles for L-*tert* Leu production was evaluated before and after bead formation. These studies showed that entrapment in agarose resulted in a significant decrease in the production of L-*tert* Leu to 23% of that of the original PEI-3A-FDH/3A-LDH particles under the conditions used. Much of this change was deduced to be due to substrate diffusion limitations imparted by the beads given that crushing the agarose beads into fragments doubled the amount of L-*tert* Leu produced compared to the intact beads. These findings were consistent with previous studies which have found that enzyme immobilization in porous materials often results in reduced activity due to slower substrate and product diffusion rates.[20]

The suitability of the PEI-3A-FDH/3A-LDH agarose beads for continuous flow reactions was confirmed by placing the beads in a column and testing their properties under typical flow conditions. The retention of PEI-3A-FDH/3A-LDH particles within the beads was validated by demonstrating the lack of any detectable protein in the outlet solution at high flow rates (1 mL/min) and the absence of any loss in catalytic activity after 60 min. Furthermore, highlighting its potential as a robust immobilization platform, the beads retained their structural integrity and 86% of their catalytic activity for L-*tert*-Leu production even after three months of storage in ddH$_2$O at 4°C.

After characterizing the beads, we then assessed their practicality for continuous flow biosynthesis. The agarose beads containing 220 mg of PEI-3A-FDH/3A-LDH particles placed in the column with an effective reactor volume of 150 mL were supplied with 2 $\mu$mol of NADH and then used to promote the complete conversion of trimethylpyruvate (TMP) to L-*tert*Leu over a period of 5 days without the addition of exogenous NADH (**Figure 22**). To sustain the high conversion for a longer period, the column was recharged with 0.2 $\mu$mol of the cofactor every 5 days. After 30 days, the TTNs of NADH and LDH in the co-immobilized particles were 6,544 and 21,348, respectively, with a productivity of 0.066 mmol/h at room temperature.

To test the generality of this approach for other enzyme processes, we utilized our Cry3Aa-mediated immobilization strategy to co-immobilize FDH[21] and *Bartonella apis* alcohol dehydrogenase (ADH)[22] - two enzymes that can work together to promote the conversion of ethyl acetoacetate (EAA) to ethyl (*R*) 3-hydroxybutyrate (*R*-EHB), a pharmaceutical precursor used in the synthesis of antibiotics, anti-inflammatory agents, and antiviral drugs.[23] Given the slower reaction rate of FDH than ADH, two copies of the 3A-FDH were used to enhance its relative concentration in the particle. The resulting co-immobilized (3A-FDH)$_2$/3A-ADH particles were then modified with PEI, embedded within agarose beads, and then evaluated for the continuous flow conversion of 5 mM EAA to *R*-EHB at a flow rate of 0.1 mL/min and room temperature. Agarose beads containing 180 mg of PEI-(3A-FDH)$_2$/3A-ADH particles pre-incubated with 2 $\mu$mol of NADH were loaded onto a continuous flow column and could be used to promote the biosynthesis of R-EHB over 5 days without addition of exogenous NADH. The conversion was 100% for the first 4 days, but dropped on the fifth day, presumably due to leaching of the NADH - full activity could be recovered by the recharging of the beads with 0.5 $\mu$mol NADH. Following a 30-day period, the TTN of NADH and ADH component within (3A-FDH)$_2$/3A-ADH crystals were recorded as 3,256 and 15,074, respectively, with a productivity of 0.023 mmol/h.

The PEI-3A-FDH/3A-LDH and PEI-(3A-FDH)$_2$/3A-ADH systems described herein illustrate the simplicity of our PEI-modified Cry3Aa-enzyme/NADH co-immobilization technology which we believe to be a significant advancement over existing immobilization approaches. While cofactor covalent attachment methods provide robust cofactor immobilization and resistance to leaching, they typically require complex modifications and support design .[24] For example, a study employing a flexible swinging arm for the covalent attachment of NAD$^+$ achieved a high TTN (turnover number) of 10,839, but the reaction time was limited to 24 h.[25] This necessitated frequent preparation of biocatalysts for extended use, increasing production costs. Entrapment approaches, such as Pickering emulsion droplets, show promise for cofactor recycling but are limited to specific reactions. This limitation arises because both substrates and products must pass through the oil phase, leading to very low flow rates due to the fragility of the droplets. Additionally, there is low control over reaction conditions, along with substrate retention issues, which restrict productivity.[26] While hydrogel entrapment[27, 28] and other ionic absorption methods[11, 14] have been reported, they face challenges stemming from enzyme and cofactor leaching which limits their useful lifetime.

In this study, we present a system designed for long-term cofactor retention and rechargeability, supporting continuous biosynthesis for up to 30 days. Although some cofactor leaching remains, the operational stability of this system establishes it as a robust platform for continuous biocatalysis. The system retains NADH for up to 5 days without the need for exogenous addition and enables recovery of activity through recharging. This method offers superior system longevity (30 days) compared to other approaches. The straightforward preparation process of biocatalysts for the continuous flow reactor facilitates its scalability by simplifying implementation, reducing operational complexity, and ensuring consistent biocatalyst performance over extended use. These features make it a useful system for multiple NADH-dependent enzyme systems, as well as other cofactor-dependent enzyme systems.

**References**

[1] B. Binay, D. Alagöz, D. Yildirim, A. Çelik, S. S. Tükel, Highly Stable and Reusable Immobilized Formate Dehydrogenases: Promising Biocatalysts for In Situ Regeneration of NADH, Beilstein J. Org. Chem. 2016, 12, 271-277.

[2] S. Kim, K. Kwon, G Tae, I. Kwon, Nano-Entrapping Multiple Oxidoreductases and Cofactor for All-In-One Nanoreactors, ACS Sustain. Chem. Eng. 2021, 9, 6741-6747.

[3] X. Ji, Z. Su, P. Wang, G Ma, S. Zhang, Integration of Artificial Photosynthesis System for Enhanced Electronic Energy-Transfer Efficacy: A Case Study for Solar-Energy Driven Bioconversion of Carbon Dioxide to Methanol, Small. 2016, 12, 4753-4762.

[4] J. Grunwald, B. Wirz, M. P. Scollar, A. M. Klibanov, Asymmetric Oxidoreductions Catalyzed by Alcohol Dehydrogenase in Organic Solvents, J. Am. Chem. Soc. 1986, 108, 6732-6734.

[5] J. Wykes, P. Dunnill, M. J. B. Lilly, Bioengineering, Cofactor Recycling in an Enzyme Reactor: A Comparison Using Free and Immobilized Dehydrogenases with Free and Immobilized NAD, Bioeng. 1975, 17, 51-68.

[6] C.-H. Wong, Nicotinamide Cofactor-Requiring Enzymatic Synthesis in Organic Solvent-Water Biphasic Systems, J. Org. Chem. 1987, 29, 197-208.

[7] P. Wang, G Ma, L. Liao, F. Gao, Construction of Multienzyme Bioactivesystems, J. Biotechnol. 2005, 22-41.

[8] S. Wu, R. Snajdrova, J. C. Moore, K. Baldenius, U. T. Bornscheuer, Biocatalysis: Enzymatic Synthesis for Industrial Applications, Catalysts. 2021, 60, 88-119.

[9] S. Mordhorst, J. N. Andexer, Round, Round We Go - Strategies for Enzymatic Cofactor Regeneration, Nat. Prod. Rep. 2020, 37, 1316-1333.

[10] T. Montes, V. Grazú, I. Manso, B. Galán, F. López-Gallego, R. González, J. A. Hermoso, J. L. Garcia, J. M. Guisán, R. Fernández-Lafuente, Improved Stabilization of Genetically Modified Penicillin G Acylase in the Presence of Organic Cosolvents by Co- Immobilization of the Enzyme with Polyethyleneimine, Adv. Synth. Catal. 2007, 349, 459-464.

[11] A. I. Benítez-Mateos, E. San Sebastian, N. Ríos-Lombardía, F. Morís, J. González-Sabin, F. López-Gallego, Asymmetric Reduction of Prochiral Ketones by Using Self-Sufficient Heterogeneous Biocatalysts Based on NADPH-Dependent Ketoreductases, Chem. Eur. J. 2017, 23, 16843-16852.

[12] E. S. da Silva, V. Gómez-Vallejo, J. Llop, F. López-Gallego, Efficient nitrogen-13 radiochemistry catalyzed by a highly stable immobilized biocatalyst, Catal. Sci. Technol. 2015, 5, 2705-2713.

[13] A. I. Benítez-Mateos, C. Huber, B. Nidetzky, J. M. Bolivar, F. López-Gallego, Design of the Enzyme-Carrier Interface to Overcome the O2 and NADH Mass Transfer Limitations of an Immobilized Flavin Oxidase, ACS Appl. Mater. Interfaces. 2020, 12, 56027-56038.

[14] S. Velasco-Lozano, A. I. Benítez-Mateos, F. López-Gallego, Co-immobilized Phosphorylated Cofactors and Enzymes as Self-Sufficient Heterogeneous Biocatalysts for Chemical Processes, Angew. Chem. Int. Ed. Engl. 2017, 56, 771-775.

[15] R. Yekta, X. Xiong, J. Li, B. S. Heater, M. M. Lee, M. K. Chan, Mechanoresponsive Protein Crystals for NADH Recycling in Multicycle Enzyme Reactions, J. Am. Chem. Soc. 2024, 146, 18817-18822.

[16] A. Alehosseini, E.-M. Gomez del Pulgar, M. J. Fabra, L. G Gómez-Mascaraque, A. Benítez-Páez, M. Sarabi-Jamab, B. Ghorani, A. Lopez-Rubio, Agarose-Based Freeze-Dried Capsules Prepared By The Oil-Induced Biphasic Hydrogel Particle Formation Approach For The Protection Of Sensitive Probiotic Bacteria, Food. Hydrocoll. 2019, 87, 487-496.

[17] N. Pernodet, M. Maaloum, B. Tinland, Pore Size of Agarose Gels by Atomic Force Microscopy, Electrophoresis 1997, 18, 55-58.

[18] N. C. Stellwagen, Electrophoresis of DNA in Agarose Gels, Polyacrylamide Gels and in Free Solution, Electrophoresis 2009, 30, 188-195.

[19] J. Narayanan, J.-Y. Xiong, X. Y. Liu, Determination of Agarose Gel Pore Size: Absorbance Measurements vis a vis Other Techniques, J. Phys. Conf. Ser. 2006, 1, 120-128.

[20] L. Bayne, R. V. Ulijn, P. J. Halling, Effect of Pore Size on the Performance of Immobilised Enzymes, Chem. Soc. Rev. 2013, 42, 9000-9010.

[21] H. Choe, J. C. Joo, D. H. Cho, M. H. Kim, S. H. Lee, K. D. Jung, Y. H. Kim, Efficient CO2-Reducing Activity of NAD-Dependent Formate Dehydrogenase from Thiobacillus sp. KNK65MA for Formate Production from CO2 Gas, PLoS One. 2014, 9, 103-111.

[22] Y. H. Zhu, C. Y. Liu, S. Cai, L. B. Guo, I. W. Kim, V. C. Kalia, J. K. Lee, Y. W. Zhang, Cloning, Expression and Characterization of a Highly Active Alcohol Dehydrogenase for Production of Ethyl (S)-4-Chloro-3-Hydroxybutyrate, Indian. J. Microbiol. 2019, 59, 225-233.

[23] J.-Y. He, L.-M. Zhou, P. Wang, L. Zu, Microbial Reduction of Ethyl Acetoacetate to Ethyl (R)-3-Hydroxybutyrate in an Ionic Liquid Containing System, Process. Biochem. 2009, 44, 316-321.

[24] E. Diamanti, S. Velasco-Lozano, D. Grajales-Hernández, A. H. Orrego, D. Di Silvio, J. M. Fraile, F. López-Gallego, Self-Sufficient Heterogeneous Biocatalysis through Boronic Acid-Diol Complexation of Adenylated Cofactors, ACS Sustain. Chem. Eng. 2023, 11, 14409-14421.

[25] C. J. Hartley, C. C. Williams, J. A. Scoble, Q. I. Churches, A. North, N. G French, T. Nebl, G Coia, A. C. Warden, G Simpson, A. R. Frazer, C. N. Jensen, N. J. Turner, C. Scott, Engineered Enzymes that Retain and Regenerate their Cofactors Enable Continuous-Flow Biocatalysis, Nat. Catal. 2019, 2, 1006-1015.

[26] W. Wei, R. Ettelaie, X. Zhang, M. Fan, Y. Dong, Z. Li, H. Yang, Co-compartmentalization of Enzymes and Cofactors within Pickering Emulsion Droplets for Continuous-Flow Catalysis, Angew. Chem. Int. Ed. Engl. 2022, 61, e202211912.

[27] T. Peschke, P. Bitterwolf, S. Gallus, Y. Hu, C. Oelschlaeger, N. Willenbacher, K. S. Rabe, C. M. Niemeyer, Self-Assembling All-Enzyme Hydrogels for Flow Biocatalysis, Angew. Chem., Int. Ed. Engl. 2018, 57, 17028-17032.
[28] Q. Chen, Y. Wang, G Luo, Recycling of Cofactors in Crude Enzyme Hydrogels as Co-immobilized Heterogeneous Biocatalysts for Continuous-Flow Asymmetric Reduction of Ketones, ChemSusChem. 2023, 16, e202201654.

**Example 4: Self-Assembled Protein System for Scalable and Sustainable Cadaverine Biosynthesis**

The growing demand for sustainable and cost-effective biocatalysts for the production of bio-based chemicals as alternatives to petroleum-based raw materials has driven the need for innovative approaches. This study shows the development of a novel biocatalytic platform for cadaverine production using enzyme self-assembled protein systems mediated by Cry1Ab fusion technology. The resulting fusion particles exhibited high catalytic efficiency for cadaverine synthesis, comparable to that of the free enzyme, while eliminating the need for complex purification and immobilization steps for recyclable use. To eliminate dependency on exogenous PLP cofactors, the fusion particles were modified with polyethyleneimine (PEI), enabling stable co-immobilization of PLP. These modified particles were subsequently entrapped in agarose and integrated into a continuous-flow biosynthesis system, achieving high production efficiency. The system sustained robust cadaverine synthesis for over 19 days with minimal PLP supplementation, demonstrating exceptional stability and catalytic longevity. The developed platform achieved turnover numbers (TTN) of 16,938 and 190,609 for PLP and CadA, respectively. This innovative approach streamlines enzyme utilization, enhances operational stability and paves the way for scalable and cost-effective bio-based chemical production, highlighting its potential for large-scale biocatalytic applications.

INTRODUCTION

The production of plastics and wide range of chemicals has mainly relied on petroleum-based raw materials as the primary source, which is ~500 million tons per year. This significant dependency on fossil resources has raised concerns about resource depletion and environmental pollution.[1] In response to this issue, the industrial sector is switching to bio-based resources, which are both environmentally friendly and renewable. It is predicted that more than 75% of petroleum-based materials will be replaced with bio-based materials by 2035, highlighting their remarkable popularity and economic benefits.[1]

Cadaverine, also known as 1,5-pentanediamine, is a crucial precursor in the production of bio-based polymers across various industries, particularly in biochemical and materials engineering.[2] Nylon 5X polymers synthesized from cadaverine exhibit high-performance polyamides with perfect thermal and mechanical properties. For example, bio-nylon 5,6, derived from cadaverine, demonstrates excellent mechanical strength, flame retardancy, moisture absorption, and air permeability, making it a sustainable and favorable choice for plastic and fiber engineering.[3] Another important application of cadaverine is in the synthesis of polyurethanes, a multifunctional polymer that are extensively used across various industries due to its durability, flexibility, and resistance to chemicals and wear. This polymer is traditionally synthesized from petroleum-derived hexamethylene diisocyanate as the preferred precursor.[4, 5] However, bio-based polyurethanes synthesized from cadaverine offer a sustainable and environmentally friendly alternative, featuring lower resource consumption, high reactivity, and enhanced weather resistance.[5, 6] Beyond polymer production, cadaverine has significant applications in medicine, with potential treatments for arrhythmia, hypoglycemia, and dysentery. [1, 7, 8] In agriculture, it functions as a valuable bio-stimulant, improving crop yields and contributing to the modern and sustainable farming strategies.[9, 10] These diverse properties and applications make cadaverine a promising green substitute for manufacturing a wide range of valuable polymers and chemicals, highlighting the importance of developing sustainable and eco-friendly synthesis methods for cadaverine on a large scale to meet global demand.

There are typically two main strategies for synthesizing cadaverine from L-lysine: (i) the chemical method and (ii) biological biosynthesis. Chemo-catalysis offers high conversion, simple separation by using centrifugation, and efficient reusability; however, it suffers from low productivity (2-5 g $L^{-1}$ $h^{-1}$) and selectivity (30-60%) due to the high reactivity of L-lysine, causing side reactions.[1, 11] The use of strong acids in this approach often leads to corrosion issues, while cadaverine's instability in acidic and metal-rich environments further compounds selectivity challenges.[1] On the other hand, enzymatic biosynthesis of cadaverine using lysine decarboxylase (CadA) is highly selective with a 100% conversion rate and demonstrates high productivity (e.g., 204 g $L^{-1}$ $h^{-1}$ for whole-cell systems). [12, 13] This method has significant advantages, including direct synthesis of cadaverine under mild conditions such as room temperature, leading to lower energy consumption and production costs.[2] The industrial application of enzymatic biosynthesis of cadaverine faces several challenges, including enzyme instability, reduced activity due to cadaverine's toxicity, and competition with food resources in whole-cell systems. One major issue is the rise in pH during the decarboxylation process, which causes enzyme instability and limits recyclability and total effectiveness.[14, 15] Another significant challenge is the dependency of CadA on pyridoxal 5'-phosphate (PLP) as a cofactor. Continuous supply of PLP is required, presenting both economic and feasibility concerns, as well as complications in downstream purification.[2, 9] The purification of free CadA is also costly, labor-intensive, and time-consuming, further increasing economic barriers. Therefore, developing technologies to eliminate the complex purification process for cell-free biocatalysts and exploring strategies for co-immobilizing and fixing PLP near the enzyme to enhance its stability and reusability are crucial.

Cry1Ab is a crystal-forming protein from the Cry protein family, produced by *Bacillus thuringiensis* (*Bt*), which kills insects by forming pores in their midgut cells.[16] Recently, our group successfully used this protein as an immobilization platform for the *in vivo* direct co-immobilization of *E. coli* ornithine decarboxylase and human arginase.[17] This approach eliminates the need for routine enzyme purification, simplifying the process to cell lysis, after which the fusion particles can be harvested. Direct enzyme immobilization within the Cry1Ab particles inside the cells also removes the need for post-immobilization steps, such as attaching enzymes to supports or carriers for recyclable use in industry. It has been shown that Cry1Ab-mediated immobilization can significantly improve the thermostability of enzymes, enabling sustainable and efficient biocatalytic reactions.[17]

In this study, we employed Cry1Ab-mediated *in vivo* immobilization technology to directly immobilize CadA inside the cells. To eliminate the continuous need for PLP supplementation, CadA-Cry1Ab fusion particles were chemically modified with polyethyleneimine (PEI), a highly cationic polymer, to facilitate the co-immobilization of the negatively charged PLP cofactor through ionic interactions. The biosystem was further extended to a packed-bed reactor, where the particles were entrapped within agarose beads, enabling the sustainable and efficient production of cadaverine in a continuous-flow mode.

MATERIALS AND METHODS

Pyridoxal 5'-phosphate (PLP), L-lysine hydrochloride, cadaverine dihydrochloride, o-phthalaldehyde (OPA) and MES (2-(N-morpholino)ethanesulfonic acid) were provided from the TCI company. Certified molecular biology agarose from Bio-Rad was provided. Polyethylenimine (PEI) (P3143) at 50% (w/v) in $H_2O$ and all other buffer salts were purchased from Sigma-Aldrich.

**Construction of Expression Plasmids**

To construct the expression plasmid of CadA (Accession Number: NP_418555), the *cadA* gene was amplified by PCR and inserted into a pET28 vector via Gibson Assembly (NEB). The resulting recombinant plasmid was transformed into *E. coli* XL10. All positive colonies were verified by DNA sequencing (BGI).

The plasmid for the expression of CadA-Cry1Ab fusion was prepared by inserting the amplified *cadA* gene into a pET28-Cry1Ab vector via Gibson Assembly (NEB). The resulting recombinant plasmid was transformed into *E. coli* XL10. All positive colonies were verified by DNA sequencing (BGI).

**Expression and Purification of CadA Free Enzyme**

For the expression of free CadA, the corresponding plasmid was transformed into *E. coli* BL21(DE3) via heat shock. The resultant colonies were cultured in 500 mL Lennox Broth (LB, IBI Scientific) with 50 μg/mL kanamycin at 37°C until the $OD_{600}$ reached 0.6-0.8. Then 0.5 mM Isopropyl β-D-1-thiogalactopyranoside (IPTG, IBI Scientific) was added to induce the expression of soluble enzymes at 18°C for 20 h. Cells were harvested by centrifugation, and the pellets were resuspended in buffer A (10 mM HEPES, 300 mM NaCl, pH 7.5) supplemented with 1 mM benzamidine hydrochloride (TCI Chemicals) and 1 mM phenylmethylsulfonyl fluoride (PMSF, Cayman Chemical) and lysed by sonication. The cell lysates were clarified by centrifugation and the resultant supernatants were filtered and applied to a nickel resin column (BioRad) for affinity purification. The target protein was eluted using 250 mM imidazole, and the purity of the eluted fractions was evaluated by SDS-PAGE. Subsequently, the eluted fractions of high purity were dialyzed twice using a SnakeSkin dialysis tubing (MWCO: 10 kDa; Thermo Scientific) in 2 L of buffer A. The dialyzed protein was concentrated using a centrifugal filter (MWCO: 30 kDa; Merck Millipore) and then stored at -80°C after being frozen with liquid nitrogen.

**Expression and Purification of CadA-Cry1Ab Fusion Particles**

Expression of CadA-Cry1Ab fusion protein was achieved by transforming the encoding plasmid into *E. coli* BL21(DE3) by heat shock and the resultant colonies were inoculated into 500 mL Terrific Broth (TB, IBI Scientific) supplemented with 4 mL glycerol and 50 μg/mL kanamycin. Cells were then grown at 37°C until the $OD_{600}$ reached 0.6-0.8, at which point, 2 mM IPTG was added, and the cells were further cultured for 48 h at 30°C. The cell pellets were resuspended in lysis buffer (50 mM Tris, 50 mM EDTA, 15% sucrose, pH 8.0) and incubated with 1 mg/mL lysozyme overnight. The lysed cell pellets were washed twice with $ddH_2O$ and then resuspended in Crystal Wash I solution (0.5 M NaCl, 2 % Triton X-100), followed by sonication and centrifugation. The collected particles were washed twice with Crystal Wash I solution, followed by two washes with 0.5 M NaCl, one wash with 0.25 M NaCl, and three washes with $ddH_2O$. The expression and purity of the target fusion protein particles were confirmed by SDS-PAGE.

All protein concentrations were obtained using the Bradford protein assay (BioRad). To quantify the amount of CadA in CadA-Cry1Ab fusion particle, the total protein concentration (mg/mL) of the particle was converted to its molar concentration by dividing the mass volume concentration (mg/mL) with the combined molecular weight of CadA (81 kDa) and Cry1Ab (130 kDa). As a fusion particle, the molar concentration of the enzyme component was equal to that of the total particle. Vice versa, the mass volume concentration (mg/mL) of CadA component could be calculated by multiplying the molar concentration by its molecular weight (81 kDa).

### Size Exclusion Chromatography (SEC)

To characterize the oligomerization of CadA, 1 mg/mL of the protein in running buffer (10 mM HEPES, 300 mM NaCl, pH 7.5) was injected into a 30 $\mu$L of injection loop and run at 0.1 mL/min. The mixture of SEC standards (Thyroglobulin: 670 kDa; $\gamma$-globulin: 158 kDa; Ovalbumin: 44 kDa; Myoglobin: 17 kDa; Vitamin B12: 1.35 kDa) was diluted twofold with running buffer and then injected into the injection loop and run at 0.1 mL/min.

### Orbitrap Mass Spectrometry

To confirm the identities of the predominant bands derived from SDS-PAGE of free CadA and CadA-Cry1Ab particles, the bands were individually excised from the Coomassie blue-stained gel and fragmented into small pieces. The gel fragments were destained, dehydrated, and subsequently digested with trypsin. The processed samples were analyzed by Orbitrap Fusion Lumos Tribrid Mass Spectrometer (Thermo Fisher Scientific).

### CadA Activity Assay

The activity of CadA was determined based on the L-lysine converted following the ninhydrin method of Chinard *et al.* The reaction solution containing 1 $\mu$M of CadA-Cry1Ab fusion particle (0.211 mg/mL) or CadA enzyme (0.081 mg/mL), 0.1 mM PLP and 50 mM L-lysine HCl was incubated at 37°C with shaking at 1000 rpm for 6 min. The reaction was stopped by the addition of 2 M HClO$_4$. The L-lysine remaining was determined by further reacting the reaction solution with equal volume of 20 mg/mL ninhydrin dissolved in 2-methoxyethanol at 90°C with shaking at 1000 rpm for 30 min. The L-lysine converted was obtained by subtracting the L-lysine remaining from its input amount. Assuming a 1:1 reaction stoichiometry for the conversion of L-lysine to cadaverine, the amount of cadaverine produced equals the L-lysine converted. All protein concentrations were determined using the Bradford protein assay, and the quantification of CadA in CadA-Cry1Ab fusion particle was carried out as described previously. All experiments were performed in triplicate.

### Thermal Stability

200 uL of 2 $\mu$M CadA enzyme or CadA-Cry1Ab particle prepared in 0.1 mM PLP and 50 mM HEPES (pH 8.0) was incubated at varying temperatures (25, 30, 37, 45, 50, 60, 70°C) with shaking at 1000 rpm for 1 h. After that, their residual activities were determined as previously described. The particles were sonicated for 10 sec prior to activity measurement. The residual activity at 25°C was taken as 100%. The data was fitted to a sigmoidal curve and the T$_{50}$ values were calculated using GraphPad Prism software. T$_{50}$ is defined as the temperature at which 50% activity remains after 1 h incubation. The protein concentrations were measured using Bradford protein assay. All experiments were performed in triplicate.

### Optimization of Reaction pH

50 uL of 1 $\mu$M CadA enzyme or 2 $\mu$M CadA-Cry1Ab particle was incubated with equal volume of 100 mM L-lysine HCl in diverse reaction buffers (pH 4 - 5: 0.1 M citric acid/sodium citrate; pH 6: 0.1 M MES; pH 7 - 8: 0.1 M HEPES; pH 9: 0.1 M CAPSO) containing 0.1 mM of PLP at 37°C with shaking at 1000 rpm for 6 min. The L-lysine converted was quantified as previously described, with the assumption that it was 100% under pH 6. All experiments were performed in triplicate.

### Longevity of CadA-Cry1Ab Particles

500 $\mu$L of 2 $\mu$M CadA-Cry1Ab was incubated at pH 6 (0.1 M MES, 0.1 mM PLP) and pH 7 (0.1 M HEPES, 0.1 mM PLP) and 25°C. At day 0, 1, 5 and 15, the residual activities were measured as previously described. The initial activity at day 0 was taken as 100 %. All experiments were performed in triplicate.

**Recyclability of CadA-Cry1Ab Particle for the Conversion of L-Lysine to Cadaverine**

The recyclability of the co-immobilized particles was evaluated by resuspending 45 mg of CadA-Cry1Ab with 10 mL of 1 M L-lysine HCl in reaction buffer (1 M MES, 0.1 mM PLP, pH 6.0), and incubating in 50 mL centrifuge tube at 37°C with shaking at 250 rpm for 1 h. At the end of the incubation, the reaction solution was centrifuged at 8000 rpm for 5 min, and the L-lysine remaining in the supernatant was determined as previously described. The pellets containing PEI-CadA-Cry1Ab-PLP particles were resuspended with 10 mL of fresh 1 M L-lysine HCl in reaction buffer to start the next reaction cycle. All experiments were performed in triplicate.

**PEI-Modification of CadA-Cry1Ab Fusion Particles**

To prepare PEI-modified CadA-Cry1Ab fusion particles, 1 mg/mL of the particles were incubated with a PEI solution (pH 7.0) for 2 h under gentle rotation at room temperature. Subsequently, EDC/NHS crosslinkers (10 mM, 1:2 molar ratio) were gradually added to the mixture and allowed to react for 2 h at 25°C under continuous rotation. To quench the reaction, 200 mM Tris-HCl (pH 7.0) was added at a 1:1 (v/v) ratio, followed by an additional 30 min of rotation at 25°C. The samples were then centrifuged at 8000 rpm for 10 min, and the precipitated particles were washed multiple times with 100 mM NaCl and ddH$_2$O to remove any unbound PEI molecules. The amount of PEI bound to the particles was quantified using the copper sulfate assay.[19, 20]

**Effect of PEI Modification on the Activity of CadA-Cry1Ab Particles**

To evaluate the impact of PEI modification on the activity of CadA-Cry1Ab, the conversion efficiency of the particles before and after PEI modification was compared by incubating the reaction solution containing 1 $\mu$M of CadA-Cry1Ab or PEI-CadA-Cry1Ab, 0.1 mM PLP, and 50 mM L-lysine HCl in 0.1 M MES (pH 6) at 37°C with gentle shaking at 100 rpm for 10 min. The depletion of L-lysine HCl was quantified as described above, with the assumption that it was 100% for CadA-Cry1Ab. All experiments were performed in triplicate.

**Dynamic Light Scattering (DLS)**

To examine the zeta potential and hydrodynamic diameter of CadA-Cry1Ab fusion particles before and after PEI binding, a Malvern Zetasizer Nano ZS90 (Malvern Instruments, UK) was used. The particles (100 $\mu$g/mL) were prepared in ddH$_2$O and PBS (10 mM, pH 7.0) for size distribution and zeta potential analysis, respectively. All measurements were conducted at 25°C.

**Binding Capacity of PLP to PEI-CadA-Cry1Ab Fusion Particles**

For binding analysis, PEI-CadA-Cry1Ab fusion particles (50 $\mu$g/mL) were incubated with increasing concentrations of PLP (0-2 mM) for 25 min under gentle rotation at room temperature. After incubation, the particles were centrifuged at 8000 rpm for 10 min, and the unbound PLP in the supernatant was quantified. A calibration curve, generated from PLP standards treated under identical conditions, was used to determine the amount of PLP bound to the particles. To stabilize the absorbance peak of PLP following Schiff base formation with the protein, 1 M HCl was added to the unbound PLP supernatants in a 1:1 (v/v) ratio. After a 5-minute incubation, the absorbance of the unbound PLP was measured at 294 nm using an Eppendorf Bio-Spectrometer (Eppendorf AG, Germany). The PLP concentration was determined using a standard curve generated for various PLP amounts treated with 1M HCL based on the Beer-Lambert law.

**Batch Recyclability PEI-CadA-Cry1Ab vs. CadA-Cry1Ab**

To evaluate the recyclability of PEI-CadA-Cry1Ab particles, 1 mg/mL of the particles was used in a reaction buffer containing 0.5 M L-lysine in 1 M MES buffer (pH 6.0) at 1000 rpm and 45°C for 20 min. Prior to the first cycle, 0.1 $\mu$mol of PLP was immobilized. To assess the effect of PEI modification, two control groups were included: (i) CadA-Cry1Ab particles with 0.1 $\mu$mol of PLP added before each cycle and (ii) CadA-Cry1Ab particles with 0.1 $\mu$mol of PLP added only before the first cycle. L-lysine depletion was measured for all samples as described above. The conversion achieved in the first cycle was considered as 100%. All experiments were performed in triplicate.

**Oil-induced Entrapment of PEI-Modified CadA-Cry1Ab Fusion Particles in Agarose Beads**

To make PEI-CadA-Cry1Ab fusion particles suitable for continuous flow reactor applications, the particles were encapsulated within an agarose polymer using an oil-biphasic method.[21] First, a 2 wt.% agarose solution was prepared in

ddH$_2$O and heated for 5 minutes in a microwave. The solution was then cooled to 40°C in an incubator. Meanwhile, PEI-CadA-Cry1Ab fusion particles (1 mg/mL) were suspended in ddH$_2$O and gently shaken in an incubator at 37°C. The two solutions were then mixed at an appropriate ratio and dropped into an oil/water biphasic system using a syringe. Upon contact with the oil phase, the droplets took on a spherical shape, and solidification occurred as the mixture cooled. The resulting beads were collected in the water phase and washed to remove the oil. The beads were then rinsed several times with 100 mM NaCl and ddH$_2$O. After that, the beads (containing 200 mg of the particles) were incubated with PLP (0.1 mM) for 1 h under slow rotation at 10°C. The amount of PLP bound to the beads was measured as described above.

**Confocal Microscopy**

To confirm the immobilization of PLP molecules on agarose beads entrapped PEI-CadA-Cry1Ab fusion particles, PEI was first labeled with Alexa Fluor™ 488 NHS Ester, and the fusion particles were tagged with Alexa Fluor™ 568 C5 Maleimide, as previously reported.[19] After separating the PEI-Alexa 488 molecules via centrifugation, they were used to modify the Alexa 568-tagged CadA-Cry1Ab particles. The resulting PEI-Alexa 488-CadA-Cry1Ab-Alexa 568 particles (0.1 mg/mL) were entrapped inside agarose beads and then incubated with PLP (1.0 mM) for 10 min under rotation, then centrifuged and washed with ddH$_2$O. The beads were placed on glass slides for confocal microscopy analysis. Using a Leica SP8 confocal microscope, signals from the bound PLP molecules within the beads were captured via the DAPI channel, and the distribution of the Alexa 488-labeled PEI molecules was visualized.

**Comparison the Cadaverine Production Efficiency of PEI-CadA-Cry1Ab** vs. **PEI-CadA-Cry1Ab-agarose beads**

To compare the efficiency of PEI-CadA-Cry1Ab particles and PEI-CadA-Cry1Ab-agarose beads in cadaverine production, a fixed amount of either free or agarose-entrapped particles (0.211 $\pm$ 0.05 mg) was incubated in a reaction buffer containing 0.1 mM PLP and 50 mM L-lysine HCl in 0.1 M MES (pH 6) at 37°C with gentle shaking at 100 rpm for 10 min. The depletion of L-lysine HCl was then quantified. The cadaverine production by PEI-CadA-Cry1Ab particles was set as 100%. To investigate the effect of the agarose hydrogel on substrate diffusion, the same experiment was conducted using physically fragmented agarose beads. All experiments were performed in triplicate.

**Continuous Flow Biosynthesis of Cadaverine**

PEI-CadA-Cry1Ab-PLP-entrapped agarose beads (4 75 mg of particles containing 4 $\mu$mol of bound PLP) were first loaded into a column and washed with ddH$_2$O for 60 min at a flow rate of 0.5 mL/min. Following this, the reaction solution (50 mM L-lysine, 0.1 M MES, pH 6.0) was continuously injected at a rate of 50 $\mu$L/min into the column, and the product was collected.

RESULTS

We previously demonstrated the successful immobilization and co-immobilization of enzymes (human arginase I and PLP-dependent ornithine decarboxylase) and fluorescent reporter proteins using the Cry1Ab platform in *E. coli*.[17] This approach enhanced the stability of fusion enzymes, enabling the efficient and recyclable conversion of arginine to putrescine, which was subsequently utilized for the synthesis of the thermostable polyamide nylon-4,6. In this study, we extended this technology to the *in vivo* immobilization of another PLP-dependent enzyme, CadA, on Cry1Ab particles for cadaverine biosynthesis. The immobilized particles were further modified with PEI and entrapped in agarose beads, creating a simple, highly stable, and efficient system for the continuous production of cadaverine in a flow reactor.

**Production and Characterization of CadA-Cry1Ab**

To produce CadA-Cry1Ab particles, CadA was genetically fused to the N-terminus of Cry1Ab, and its expression in *E. coli BL21* (*DE3*) was confirmed by SDS-PAGE (**Figure 23**). The purified CadA-Cry1Ab fusion particles were then analyzed using Orbitrap Mass Spectrometry to assess their structural integrity and sequence coverage. The mass spectrometry analysis showed that free CadA exhibited approximately 85% sequence coverage using the CadA protein sequence as a reference, whereas CadA-Cry1Ab displayed 58% sequence coverage using the CadA-Cry1Ab fusion sequence as a reference (**Figure 24**). The detection of CadA peptides within the fusion particles confirmed that the fusion was successfully achieved and that CadA was effectively integrated into the Cry1Ab particles. These results validate the successful expression and purification of CadA-Cry1Ab fusion particles. To compare free CadA with CadA-Cry1Ab particles, free CadA was also expressed, purified, and verified using SDS-PAGE analysis. Additionally, Size Exclusion Chromatography (SEC) was performed to assess the oligomeric state of CadA (**Figure 25**). The SEC profile showed a distinct elution peak corresponding to the expected molecular weight of CadA, indicating its proper folding.

Following the production of free CadA and CadA-Cry1Ab fusion particles, their catalytic activity was evaluated. As shown in **Figure 26,** both exhibited comparable cadaverine production levels, with the fusion particles displaying only a slight decrease in activity (~10%) relative to the free enzyme. Thermostability analysis revealed that the fusion particles exhibited comparable stability at elevated temperatures (**Figure 27**). To determine the optimal reaction conditions, the enzymatic activity of both free CadA and CadA-Cry1Ab particles was assessed across different pH values. The results indicated that the optimal pH range for cadaverine production was 6.0-7.0 (**Figure 28**). The long-term stability of the CadA-Cry1Ab particles was further examined at pH 6.0-7.0 over a 15-day period, revealing high stability with no significant loss of enzymatic activity (**Figure 28**). The recyclability of the particles was also investigated. The particles were efficiently recovered via simple centrifugation and reused for 10 cycles in the conversion of 1 M L-lysine, with only a slight decline in activity observed after the eighth cycle (**Figure 29**). However, the addition of the cofactor PLP in each cycle was required.

**PEI modification of CadA-Cry1Ab fusion particles**

To more efficiently use the cofactor and also reduce the burden of downstream isolation of the target product cadaverine, a cationization approach was explored using PEI, a highly cationic polymer, to hold PLP. As shown in **Figure 31,** PEI modification significantly increased the PLP binding capacity of the particles, from ~100 $\mu$mol/g to ~1300 $\mu$mol/g-a 13-fold enhancement. The modified particles demonstrated prolonged stability, maintaining PLP immobilization for more than 10 cycles in MES buffer (100 mM, pH 6.0), suggesting effective cofactor retention (**Figure 32**). Dynamic light scattering (DLS) analysis revealed that PEI modification altered the zeta potential of the particles, increasing from -50 mV in unmodified particles to +54 mV in PEI-coated CadA-Cry1Ab particles (**Figure 33**), while the overall particle size distribution remained unchanged (**Figure 34**). Furthermore, PLP binding to PEI-modified particles resulted in a decrease in zeta potential from +54 mV to +2 mV, confirming the ionic nature of the PLP-PEI-CadA-Cry1Ab particles' interaction (**Figure 33**).

**PEI-CadA-Cry1Ab Fusion Particle Entrapment within Agarose Beads**

Since the decarboxylation reaction of L-lysine would increase the reaction pH, and CadA is highly sensitive to alkaline conditions that can inactivate the enzyme, thus the PEI-modified particles were entrapped within agarose beads to achieve an appropriate size for a continuous flow reactor, facilitating the continuous collection of the product and thereby enhancing enzyme stability.[22] To assess the distribution of PEI-CadA-Cry1Ab particles within the agarose beads and evaluate the immobilization of PLP, fluorescently labelled PEI-Alexa 488-CadA-Cry1Ab-Alexa 568 particles were used for entrapment. Confocal microscopy was then employed to analyze cross-sections of the beads. As shown in **Figure 35,** the particles were evenly distributed throughout the beads, and successful co-immobilization of PLP within the particles inside the beads was confirmed. Although the activity of the beads decreased by almost 60% after immobilization due to substrate diffusion limitations (**Figure 36**), this approach offers significant advantages. The simplicity and convenience of using immobilized beads in the bioreactor streamline the process while also preventing backpressure and particle leakage through the column issues, which are common challenges associated with free enzyme systems.

**Continuous Flow Biosynthesis of Cadaverine**

After characterization, beads containing 75 mg of immobilized particles were placed inside a continuous flow reactor. A 50 mM L-lysine solution in 0.1 M MES buffer (pH 6.0) was continuously injected at a flow rate of 50 $\mu$L/min to enable sustained cadaverine production. The conversion rate of L-lysine to cadaverine was assessed at the start of the process, confirming complete substrate conversion. The amount of bound PLP within the same quantity of beads was determined to be 4 $\mu$mol. As shown in **Figure 37,** the continuous flow bioreactor operated efficiently for almost three weeks without requiring additional PLP supplementation, achieving maximum substrate conversion efficiency. After 19 days of continuous operation, exogenous PLP was added to determine whether the observed activity loss was due to PLP depletion or enzyme inactivation. The results showed that the conversion recovered but then quickly dropped down, indicating that enzyme deactivation was the primary cause rather than cofactor depletion. This prolonged enzyme stability and operational lifespan in continuous cadaverine production represent a significant advancement in biocatalyst development, demonstrating strong potential for industrial scalability. After 19 days, the turnover number for PLP reached 16,938, while the turnover number for CadA was 190,609, highlighting the high efficiency of the bioreactor system.

DISCUSSION

The co-immobilization of pyridoxal phosphate (PLP) and enzyme stabilization are crucial steps in optimizing biocatalysis for cadaverine synthesis. Wei *et al.*[23] first reported that deacetylated chitin powder could be used for the co-immobilization of both PLP-dependent L-lysine decarboxylase and PLP. However, they faced challenges related to the limited surface area and irregular morphology, which restricted PLP adsorption and hindered its application in continuous-flow systems. To address these challenges, Zhou et al.[24] developed chitin-chitosan microspheres for the co-immobilization of PLP and three different PLP-dependent enzymes-L-lysine decarboxylase, L-dopa decarboxylase, and a transaminase. In this system, the deacetylation of the carrier played a crucial role in enhancing PLP adsorption and enzyme stabilization. They reported that although a high degree of deacetylation significantly improved PLP retention by increasing the number of exposed primary amines, it also weakened the interaction between the chitin-binding domain-fused enzymes and the carrier. Further improvements were achieved by modifying the microspheres with polyethyleneimine (PEI) polymer, which enhanced PLP loading capacity due to the presence of additional amine groups. However, enzyme leakage from the carrier remained a major issue in batch-mode reactions. The transition to a continuous-flow reactor significantly improved enzyme stability, but the biocatalysts retained only 85% of their initial activity after 8 h. In another study, Mi et al.[2] utilized a hybrid system to co-immobilize His-tagged L-lysine decarboxylase and PLP within PEI-modified barium alginate hydrogel microspheres. This system simplified enzyme purification via the His-tag and facilitated the co-immobilization of PLP with PEI. They applied this system in a microreactor and achieved a cadaverine production of 2.16 g after 6 h at a flow rate of 0.07 mL/min in a 10 mL column. However, a noticeable reduction in activity was observed after just 3 h of reaction, highlighting the need for further improvements to enhance its long-term stability and sustainability.

In this study, we developed an enzyme self-assembled protein system mediated by Cry1Ab fusion technology to produce CadA-Cry1Ab fusion particles in *Escherichia coli* as an expression host. The particles were obtained through simple sonication and demonstrated excellent potential as efficient biocatalysts for cadaverine production. However, the need for an exogenous PLP cofactor was identified, prompting the further modification of the particles with PEI polymer to enable stable co-immobilization of PLP, and CadA-Cry1Ab fusion particles. These modified particles were subsequently entrapped within an agarose matrix and employed in the continuous-flow biosynthesis of cadaverine. The resulting biosystem demonstrated high production efficiency, maintaining robust cadaverine synthesis for over almost three weeks with minimal additional PLP required ($TTN_{PLP}$:16,938), confirming its sustained catalytic performance. The straightforward preparation, purification, and PLP co-immobilization processes emphasize the economic feasibility of this technology for large-scale cadaverine production. Given these promising results, this platform has significant potential for scaling up and could be extended to other PLP-dependent enzymes, thereby broadening its applicability for the efficient biocatalytic production of a wide range of value-added chemicals. The versatility and stability of the platform make it an attractive option for industrial bio-transformations, where long-term stability and high catalytic turnover are critical for cost-effective production processes.

**References**

[1] Y. Huang, X. Ji, Z. Ma, M. Łężyk, Y. Xue, H. Zhao, Green chemical and biological synthesis of cadaverine: recent development and challenges, *RSC Advances* **2021,** *11*, 23922-23942.

[2] J. Mi, S. Liu, Y. Du, H. Qi, L. Zhang, Cofactor self-sufficient by co-immobilization of pyridoxal 5'-phosphate and lysine decarboxylase for cadaverine production, Bioresource Technology Reports 2022, 17, 100939.

[3] S. Kind, S. Neubauer, J. Becker, M. Yamamoto, M. Völkert, G Abendroth, O. Zelder, C. Wittmann, From zero to hero - production of bio-based nylon from renewable resources using engineered Corynebacterium glutamicum, Metabolic engineering 2014, 25, 113-123.

[4] W. Ma, K. Chen, Y. Li, N. Hao, X. Wang, P. Ouyang, Advances in Cadaverine Bacterial Production and Its Applications, Engineering 2017, 3, 308-317.

[5] A. Delavarde, G Savin, P. Derkenne, M. Boursier, R. Morales-Cerrada, B. Nottelet, J. Pinaud, S. Caillol, Sustainable polyurethanes: toward new cutting-edge opportunities, Progress in Polymer Science 2024, 151, 101805.

[6] S. Samanta, S. Selvakumar, J. Bahr, D. S. Wickramaratne, M. Sibi, B. J. Chisholm, Synthesis and Characterization of Polyurethane Networks Derived from Soybean-OilBased Cyclic Carbonates and Bioderivable Diamines, ACS Sustainable Chemistry & Engineering 2016, 4, 6551-6561.

[7] L. L. Cruz, B. S. F. da Silva, G G Araujo, T. Leal-Silva, V. G Paula, M. R. Souza, T. S. Soares, R. Q. Moraes-Souza, G C. Monteiro, G P. P. Lima, D. C. Damasceno, G T. Volpato, Phytochemical and antidiabetic analysis of Curatella americana L. aqueous extract on the rat pregnancy, Journal of Ethnopharmacology 2022, 293, 115287.

[8] R. Chen, C. Liao, Q. Guo, L. Wu, L. Zhang, X. Wang, Combined systems pharmacology and fecal metabonomics to study the biomarkers and therapeutic mechanism of type 2 diabetic nephropathy treated with Astragalus and Leech, RSC Adv 2018, 8, 27448-27463.

[9] S. Ozmen, S. Tabur, S. Oney-Birol, Alleviation role of exogenous cadaverine on cell cycle, endogenous polyamines amounts and biochemical enzyme changes in barley seedlings under drought stress, Scientific Reports 2023, 13, 17488.

[10] F. Cassán, S. Maiale, O. Masciarelli, A. Vidal, V. Luna, O. Ruiz, Cadaverine production by Azospirillum brasilense and its possible role in plant growth promotion and osmotic stress mitigation, European Journal of Soil Biology 2009, 45, 12-19.

[11] X. Lv, Z. Ma, X. Li, Y. Zhang, Y. Huang, T. Li, Highly efficient decarboxylation of L-lysine to cadaverine catalyzed by supported ruthenium oxide, Catalysis Communications 2021, 158, 106339.

[12] H. T. Kim, K.-A. Baritugo, Y. H. Oh, S. M. Hyun, T. U. Khang, K. H. Kang, S. H. Jung, B. K. Song, K. Park, I.-K. Kim, M. O. Lee, Y. Kam, Y. T. Hwang, S. J. Park, J. C. Joo, Metabolic Engineering of Corynebacterium glutamicum for the High-Level Production of Cadaverine That Can Be Used for the Synthesis of Biopolyamide 510, ACS Sustainable Chemistry & Engineering 2018, 6, 5296-5305.

[13] H. T. Kim, K.-A. Baritugo, Y. H. Oh, K.-H. Kang, Y. J. Jung, S. Jang, B. K. Song, I.-K. Kim, M. O. Lee, Y. T. Hwang, K. Park, S. J. Park, J. C. Joo, High-Level Conversion of l-lysine into Cadaverine by Escherichia coli Whole Cell Biocatalyst Expressing Hafnia alvei l-lysine Decarboxylase, 2019, 11, 1184.

[14] Y. M. Moon, S. Y. Yang, T. R. Choi, H. R. Jung, H. S. Song, Y. H. Han, H. Y. Park, S. K. Bhatia, R. Gurav, K. Park, J. S. Kim, Y. H. Yang, Enhanced production of cadaverine by the addition of hexadecyltrimethylammonium bromide to whole cell system with regeneration of pyridoxal-5'-phosphate and ATP, Enzyme and microbial technology 2019, 127, 58-64.

[15] U. Kanjee, I. Gutsche, E. Alexopoulos, B. Zhao, M. El Bakkouri, G Thibault, K. Liu, S. Ramachandran, J. Snider, E. F. Pai, W. A. Houry, Linkage between the bacterial acid stress and stringent responses: the structure of the inducible lysine decarboxylase, The EMBO journal 2011, 30, 931-944.

[16] R. Adalat, F. Saleem, N. Crickmore, S. Naz, A. R. Shakoori, In Vivo Crystallization of Three-Domain Cry Toxins, 2017, 9, 80.

[17] X. Xiong, B. S. Heater, W. Y. Ho, H. K. Lee, M. M. Lee, M. K. Chan, Genetically Encoded Multienzyme Particles for the Biosynthesis of Putrescine from l-Arginine, ACS Sustainable Chemistry & Engineering 2024, 12, 773-784.

[18] M. M. Bradford, A rapid and sensitive method for the quantitation of microgram quantities of protein utilizing the principle of protein-dye binding, Anal. Biochem. 1976, 72, 248-254.

[19] R. Yekta, X. Xiong, J. Li, B. S. Heater, M. M. Lee, M. K. Chan, Mechanoresponsive Protein Crystals for NADH Recycling in Multicycle Enzyme Reactions, J. Am. Chem. Soc. 2024, 146, 18817-18822.

[20] T. Wen, F. Qu, N. B. Li, H. Q. Luo, A facile, sensitive, and rapid spectrophotometric method for copper(II) ion detection in aqueous media using polyethyleneimine, Arabian Journal of Chemistry 2017, 10, S1680-S1685.

[21] A. Alehosseini, E.-M. Gomez del Pulgar, M. J. Fabra, L. G Gómez-Mascaraque, A. Benítez-Páez, M. Sarabi-Jamab, B. Ghorani, A. Lopez-Rubio, Agarose-Based Freeze-Dried Capsules Prepared by the Oil-Induced Biphasic Hydrogel Particle Formation Approach for the Protection of Sensitive Probiotic Bacteria, Food Hydrocoll. 2019, 87, 487-496.

[22] A. Alehosseini, E.-M. Gomez del Pulgar, M. J. Fabra, L. G Gómez-Mascaraque, A. Benítez-Páez, M. Sarabi-Jamab, B. Ghorani, A. Lopez-Rubio, Agarose-based freeze-dried capsules prepared by the oil-induced biphasic hydrogel particle formation approach for the protection of sensitive probiotic bacteria, Food Hydrocolloids 2019, 87, 487-496.

[23] G Wei, Y. Chen, N. Zhou, Q. Lu, S. Xu, A. Zhang, K. Chen, P. Ouyang, Chitin biopolymer mediates self-sufficient biocatalyst of pyridoxal 5'-phosphate and L-lysine decarboxylase, Chemical Engineering Journal 2022, 427, 132030.

[24] N. Zhou, G Wei, X. Chen, B. Wu, H. Li, Q. Lu, X. Cao, A. Zhang, K. Chen, P. Ouyang, Self-sufficient biocatalysts constructed using chitin-based microspheres, Chemical Engineering Journal 2023, 459, 141660.

## Example 5: PEI-Cry3Aa delivery of SpCas9 ribonucleoproteins

We previously showed that Pos3Aa crystals were useful tools for delivery of proteins, and so we decided to see whether PEI-Cry3Aa (PEI3A) crystals which are even more positively charged could be used as a possibly better delivery vehicle for proteins. To test this, we decided to try Cas9, an important protein used in gene editing. Here instead of as a fusion, the negatively charged Cas9 ribonucleoprotein (RNP) is bound to the positively charged PEI3A crystals via electrostatic interaction.

We first tested the binding of RNP to PEI3A crystals. Cas9 protein was pre-complexed with single guide RNA (sgRNA) in reduced serum media at room temperature to form RNP. Then, PEI3A crystals equilibrated with the same media were added to RNP at a molar ratio of 10:1 (PEI3A:RNP), and incubated at 4°C for 1 hour to form PEI3A/RNP complex. As shown in **Fig. 38A,** PEI3A crystals could bind a significant amount of RNP, and they could be pelleted along with PEI3A by centrifugation. The binding was nearly abolished without the polyanionic sgRNA complexed with Cas9 protein, indicating the interaction is based on charge.

We then moved to evaluate the intracellular delivery efficiency of RNP into human breast cancer cells MDA-MB0231 by PEI3A. As a positive control, RNP mixed with the commercially available lipofectamine 2000 (L2KRNP), a cationic lipid that has previously been reported to mediate efficient RNP delivery for gene editing, was set up in parallel. Immunostaining using CAS9 antibody against the treated cells shows that the delivery efficiency of PEI3A is comparable to that L2KRNP (**Fig. 38B**).

We thus proceeded to investigate the gene editing efficiency of the PEI3A-delivered RNP. HEK293T cells pretreated with 60 μM chloroquine, a commonly used endosomolytic agent, followed by different concentrations of PEI3A/RNP. The gene editing efficiency was then determined using the T7E1 mismatch assay. The results showed that PEI3A/RNP could efficiently deliver functional RNP into HEK293T and disrupt the AAV1 locus in a dose-dependent, achieving an editing efficiency of 43% for the highest dose tested (**Fig. 39A**). Further optimization experiments indicate that using 10:1 PEI3A:RNP molar ratio to generate the PEI3A/RNP complex results in the highest level of editing efficiency (**Fig. 39B**).

All patents, patent applications, and other publications, including GenBank Accession Numbers, cited in this application are incorporated by reference in the entirety for all purposes.

## SEQUENCE LISTING

SEQ ID NO:1 amino acid sequence of hArg

MSAKSRTIGIIGAPFSKGQPRGGVEEGPTVLRKAGLLEKLKEQECDVKDYGDLPFADIPNDSPFQIVK
NPRSVGKASEQLAGKVAEVKKNGRISLVLGGDHSLAIGSISGHARVHPDLGVIWVDAHTDINTPLTTT
SGNLHGQPVSFLLKELKGKIPDVPGFSWVTPCISAKDIVYIGLRDVDPGEHYILKTLGIKYFSMTEVDRL
GIGKVMEETLSYLLGRKKRPIHLSFDVDGLDPSFTPATGTPVVGGLTYREGLYITEEIYKTGLLSGLDIM
EVNPSLGKTPEEVTRTVNTAVAITLACFGLAREGNHKPIDYLNPPK

SEQ ID NO:2 amino acid sequence of ODC$_{mut}$

MKSMNIAASSELVSRLSSHRRVVALGDTDFTDVAAVVITAADSRSGILALLKRTGFHLPVFLYSEHAVE
LPAGVTAVINGNEQQWLELESAACQYEENLLPPFYDTLTQYVEMGNSTFACPGHQHGAFFKKHPAG
RHFYDFFGENVFRADMCNADVKLGDLLTHTGSAKDAQKFAAKVFHADKTYFVLNGTSAANKVVTNAL
LTRGDLVLFDRNNHKSNHHGALIQAGATPVYLEASRNPFGFIGGIDAHCFNEEYLRQQIRDVAPEKAD
LPRPYRLAIIQLGTYDGTVYNARQVIDTVGHLCDYILFDSAWVGYEQFIPMMADSSPLLLELNENDPGI
FVTQSVHKQQAGFSQTSQIHKKDNHIRGQARFCPHKRLNNAFMLHASTSPFYPLFAALDVNAKIHEG
ESGRRLWAECVEIGIEARKAILARCKLFRPFIPPVVDGKLWQDYPTSVLASDRRFFSFEPGAKWHGFE
GYAADQYFVDPCKLLLTTPGIDAETGEYSDFGVPATILAHYLRENGIVPEKCDLNSILFLLTPAESHEKL
AQLVAMLAQFEQHIEDDSPLVEVLPSVYNKYPVRYRDYTLRQLCQEMHDLYVSFDVKDLQKAMFRQ
QSFPSVVMNPQDAHSAYIRGDVELVRIRDAEGRIAAEGALPYPPGVLCVVPGEVWGGAVQRYFLALE
EGVNLLPGFSPELQGVYSETDADGVKRLYGYVLK

SEQ ID NO:3 amino acid sequence of Cry1Ab

MDNNPNINECIPYNCLSNPEVEVLGGERIETGYTPIDISLSLTQFLLSEFVPGAGFVLGLVDIIWGIFGPS
QWDAFLVQIEQLINQRIEEFARNQAISRLEGLSNLYQIYAESFREWEADPTNPALREEMRIQFNDMNS
ALTTAIPLFAVQNYQVPLLSVYVQAANLHLSVLRDVSVFGQRWGFDAATINSRYNDLTRLIGNYTDHA
VRWYNTGLERVWGPDSRDWIRYNQFRRELTLTVLDIVSLFPNYDSRTYPIRTVSQLTREIYTNPVLEN
FDGSFRGSAQGIEGSIRSPHLMDILNSITIYTDAHRGEYYWSGHQIMASPVGFSGPEFTFPLYGTMGN
AAPQQRIVAQLGQGVYRTLSSTLYRRPFNIGINNQQLSVLDGTEFAYGTSSNLPSAVYRKSGTVDSLD
EIPPQNNNVPPRQGFSHRLSHVSMFRSGFSNSSVSIIRAPMFSWIHRSAEFNNIIPSSQITQIPLTKSTN
LGSGTSVVKGPGFTGGDILRRTSPGQISTLRVNITAPLSQRYRVIRYASTTNLQFHTSIDGRPINQGN
FSATMSSGSNLQSGSFRTVGFTTPFNFSNGSSVFTLSAHVFNSGNEVYIDRIEFVPAEVTFEAEYDLE
RAQKAVNELFTSSNQIGLKTDVTDYHIDQVSNLVECLSDEFCLDEKKELSEKVKHAKRLSDERNLLQD
PNFRGINRQLDRGWRGSTDITIQGGDDVFKENYVTLLGTFDECYPTYLYQKIDESKLKAYTRYQLRGY

IEDSQDLEIYLIRYNAKHETVNVPGTGSLWPLSAPSPIGKCAHHSHHFSLDIDVGCTDLNEDLGVWVIF
KIKTQDGHARLGNLEFLEEKPLVGEALARVKRAEKKWRDKREKLEWETNIVYKEAKESVDALFVNSQ
YDRLQADTNIAMIHAADKRVHSIREAYLPELSVIPGVNAAIFEELEGRIFTAFSLYDARNVIKNGDFNNG
LSCWNVKGHVDVEEQNNHRSVLVVPEWEAEVSQEVRVCPGRGYILRVTAYKEGYGEGCVTIHEIEN
NTDELKFSNCVEEEVYPNNTVTCNDYTATQEEYEGTYTSRNRGYDGAYESNSSVPADYASAYEEKA
YTDGRRDNPCESNRGYGDYTPLPAGYVTKELEYFPETDKVWIEIGETEGTFIVDSVELLLMEE

SEQ ID NO:4 amino acid sequence of Cry3Aa

```
MNPNNRSEHDTIKTTENNEVPTNHVQYPLAETPNPTLEDLNYKEFLRMTADNNTEALDSSTTKDVIQKGISVVGD
LLGVVGFPFGGALVSFYTNFLNTIWPSEDPWKAFMEQVEALMDQKIADYAKNKALAELQGLQNNVEDYVSALSSW
QKNPVSSRNPHSQGRIRELFSQAESHFRNSMPSFAISGYEVLFLTTYAQAANTHLFLLKDAQIYGEEWGYEKEDI
AEFYKRQLKLTQEYTDHCVKWYNVGLDKLRGSSYESWVNFNRYRREMTLTVLDLIALFPLYDVRLYPKEVKTELT
RDVLTDPIVGVNNLRGYGTTFSNIENYIRKPHLFDYLHRIQFHTRFQPGYYGNDSFNYWSGNYVSTRPSIGSNDI
ITSPFYGNKSSEPVQNLEFNGEKVYRAVANTNLAVWPSAVYSGVTKVEFSQYNDQTDEASTQTYDSKRNVGAVSW
DSIDQLPPETTDEPLEKGYSHQLNYVMCFLMQGSRGTIPVLTWTHKSVDFFNMIDSKKITQLPLVKAYKLQSGAS
VVAGPRFTGGDIIQCTENGSAATIYVTPDVSYSQKYRARIHYASTSQITFTLSLDGAPFNQYYFDKTINKGDTLT
YNSFNLASFSTPFELSGNNLQIGVTGLSAGDKVYIDKIEFIPVN
```

SEQ ID NO:5 (amino acid sequence of wild-type ODC)

MKSMNIAASSELVSRLSSHRRVVALGDTDFTDVAAVVITAADSRSGILALLKRTGFH
LPVFLYSEHAVELPAGVTAVINGNEQQWLELESAACQYEENLLPPFYDTLTQYVEM
GNSTFACPGHQHGAFFKKHPAGRHFYDFFGENVFRADMCNADVKLGDLLIHEGSAK
DAQKFAAKVFHADKTYFVLNGTSAANKVVTNALLTRGDLVLFDRNNHKSNHHGAL
IQAGATPVYLEASRNPFGFIGGIDAHCFNEEYLRQQIRDVAPEKADLPRPYRLAIIQLG
TYDGTVYNARQVIDTVGHLCDYILFDSAWVGYEQFIPMMADSSPLLLELNENDPGIF
VTQSVHKQQAGFSQTSQIHKKDNHIRGQARFCPHKRLNNAFMLHASTSPFYPLFAAL
DVNAKIHEGESGRRLWAECVEIGIEARKAILARCKLFRPFIPPVVDGKLWQDYPTSVL
ASDRRFFSFEPGAKWHGFEGYAADQYFVDPCKLLLTTPGIDAETGEYSDFGVPATIL
AHYLRENGIVPEKCDLNSILFLLTPAESHEKLAQLVAMLAQFEQHIEDDSPLVEVLPS
VYNKYPVRYRDYTLRQLCQEMHDLYVSFDVKDLQKAMFRQQSFPSVVMNPQDAHS
AYIRGDVELVRIRDAEGRIAAEGALPYPPGVLCVVPGEVWGGAVQRYFLALEEGVN
LLPGFSPELQGVYSETDADGVKRLYGYVLK

SEQ ID NO:6 (amino acid sequence of wild-type alcohol dehydrogenase ADH)

MRVKDKVAIVTGGAKGLGEATSRLLAKEGAKVVLTDLDEVQGKKVEAEINKAGGI
AKYMKHDVANEQQWKKVVDDTVKLFGKLDILVNNAGVGHSASAEDETLEKWRFL
QSINLDAVFLGTREAIRVMKNQKSGSIINLSSIEGLVGDPTLAAYNASKGGVRLFTKS
AALHCAKSGYGIRVNSVHPGYIWTPMVEESAKESGDVEERKAALTALHPIGKLGVP
DDIAYGILYLASDESGFVTGSELVIDGGYTAQ

SEQ ID NO:7 (amino acid sequence of wild-type formate dehydrogenase FDH)

MAKILCVLYDDPVDGYPKTYARDDLPKIDHYPGGQTLPTPKAIDFTPGQLLGSVSGE
LGLRKYLEANGHTFVVTSDKDGPDSVFEKELVDADVVISQPFWPAYLTPERIAKAKN
LKLALTAGIGSDHVDLQSAIDRGITVAEVTYCNSISVAEHVVMMILGLVRNYIPSHD
WARKGGWNIADCVEHSYDLEGMTVGSVAAGRIGLAVLRRLAPFDVKLHYTDRHRL
PEAVEKELGLVWHDTREDMYPHCDVVTLNVPLHPETEHMINDETLKLFKRGAYIVN
TARGKLADRDAIVRAIESGQLAGYAGDVWFPQPAPKDHPWRTMKWEGMTPHISGT
SLSAQARYAAGTREILECFFEGRPIRDEYLIVQGGALAGTGAHSYSKGNATGGSEEA
AKFKKAG

SEQ ID NO:8 (amino acid sequence of wild-type leucine dehydrogenase LDH)

MTLEIFEYLEKYDYEQVVFCQDKESGLKAIIAIHDTTLGPALGGTRMWTYDSEEAAIE
DALRLAKGMTYKNAAAGLNLGGAKTVIIGDPRKDKSEAMFRALGRYIQGLNGRYIT
AEDVGTTVDDMDIIHEETDFVTGISPSFGSSGNPSPVTAYGVYRGMKAAAKEAFGTD
NLEGKVIAVQGVGNVAYHLCKHLHAEGAKLIVTDINKEAVQRAVEEFGASAVEPNE
IYGVECDIYAPCALGATVNDETIPQLKAKVIAGSANNQLKEDRHGDIIHEMGIVYAPD
YVINAGGVINVADELYGYNRERALKRVESIYDTIAKVIEISKRDGIATYVAADRLAEE
RIASLKNSRSTYLRNGHDIISRR

.

## Claims

1. A protein crystal formed by a Cry protein, a crystal-forming variant thereof, a crystal-forming fragment thereof, or a fusion polypeptide comprising the Cry protein, the variant, or the fragment fused to a heterologous protein, wherein the protein crystal is modified with polyethylenimine (PEI).

2. The protein crystal of claim 1, wherein the Cry protein is Cry3Aa or Cry1Ab.

3. The protein crystal of claim 1,

   which is formed by a fusion polypeptide comprising a Cry protein, a crystal-forming variant of the Cry protein, or a crystal-forming fragment of the Cry protein fused to a heterologous protein, or
   which is formed by a Cry protein, a crystal-forming variant of the Cry protein, or a crystal-forming fragment of the Cry protein and comprises a heterologous protein entrapped within.

4. The protein crystal of claim 3, wherein the heterologous protein is an enzyme, wherein preferably the enzyme is a formate dehydrogenase (FDH), leucine dehydrogenase (LDH), human arginase (hArg), ornithine decarboxylase (ODC), alcohol dehydrogenase (ADH), mandelate dehydrogenase (MDH), carbonyl reductase (CBR), reductive aminase (RedAm), or cytochrome P450 (CYP or P450).

5. The protein crystal of claim 3, wherein the fusion polypeptide comprises a Cry protein, a crystal-forming variant of the Cry protein, or a crystal-forming fragment of the Cry protein fused to an enzyme, wherein preferably the fusion polypeptide is a Cry3Aa-ADH fusion, Cry3Aa-FDH fusion, Cry3Aa-LDH fusion, Cry3Aa-MDH fusion, Cry3Aa-P450 fusion, Cry1Ab-hArg fusion, Cry1Ab-ODC fusion, or Cry1Ab-P450 fusion.

6. The protein crystal of claims 4 or 5,
   wherein the protein crystal is bound with a cofactor required for a chemical reaction catalyzed by the enzyme,

   wherein preferably the cofactor is a phosphorylated cofactor, wherein more preferably the phosphorylated cofactor is nicotinamide adenine dinucleotide (NADH), flavin adenine dinucleotide (FADH), or pyridoxal phosphate (PLP), or
   wherein preferably the enzyme is an ADH, FDH, LDH, MDH, CBR, or RedAm and the phosphorylated cofactor is NADH,
   wherein preferably the enzyme is an ODC and the phosphorylated cofactor is PLP.

7. The protein crystal of claim 1, comprising two fusion polypeptides.

8. A composition comprising the protein crystal of any one of claims 1-7.

9. The composition of claim 8,

   wherein the protein crystal comprises an enzyme capable of catalyzing a substrate in a chemical reaction and a cofactor required for the reaction, and wherein composition further comprises the substrate,
   wherein the protein crystal is formed by a fusion polypeptide comprising a Cry protein fused to an ADH, FDH, LDH, MDH, or P450,
   wherein the protein crystal is formed by a fusion polypeptide comprising a Cry protein fused to an hArg or ODC,
   wherein the protein crystal is formed by two fusion polypeptides.

10. The composition of claim 9, wherein the protein crystal is bound with NADH, or wherein the protein crystal is bound with PLP.

11. A method for performing a chemical reaction, comprising the step of incubating the protein crystal of any one of claims 4-6 with a substrate to the enzyme under conditions permissible for the substrate to be catalyzed by the enzyme in the chemical reaction.

12. The method of claim 11,

    wherein the enzyme is a formate dehydrogenase (FDH), leucine dehydrogenase (LDH), human arginase (hArg), ornithine decarboxylase (ODC), alcohol dehydrogenase (ADH), mandelate dehydrogenase (MDH), carbonyl reductase (CBR), or reductive aminase (RedAm), or cytochrome P450 (CYP or P450,
    wherein the enzyme is an ADH, FDH, LDH, MDH, CBR, or RedAm and the protein crystal is bound with NADH,
    wherein the enzyme is an ODC and the protein crystal is bound with PLP,
    wherein the protein crystal comprises two fusion polypeptides.

13. The method of claim 11 or 12, further comprising, after the reaction is completed, removing the reaction product and reusing the crystal in a second reaction.

14. An *in vitro* method of immobilizing a cofactor by incubating the cofactor with the protein crystal of any one of claims 1-7 in a solution and allowing the cofactor to bind to the protein crystal.

15. The method of claim 14,

    wherein the cofactor is NADH or PLP, or
    further comprising, after the cofactor is bound to the protein crystal, stirring the solution to release the cofactor from the protein crystal, or
    further comprising, after the cofactor is released from the protein crystal, discontinuing stirring the solution to allow the cofactor to again bind to the protein crystal.

16. A method of delivering a protein of interest to a target cell, comprising contacting the target cell with a pre-formed complex between the protein of interest and the protein crystal of any one of claims 1-7.

17. The method of claim 16,

wherein the protein crystal is a PEI-Cry3Aa protein crystal, and wherein the protein of interest is a negatively charged protein or a protein comprising or linked to a negatively charged moiety, or

further comprising, prior to the contacting, incubating the protein of interest with the protein crystal to form a complex, or

wherein the protein of interest is a nuclear protein, and wherein the contacting is performed in the presence of an endosomolytic agent.

# Figure 1

A

Solvent Channel

B

PEI

EDC/NHS

Cry3Aa crystal

3A-PEI crystal

# Figure 2

# Figure 3

# Figure 4

**Bright field**    **Alexa488**    **Alexa 568**

**DAPI**    **Merged**

# Figure 5

**A**

**B**

# Figure 6

Figure 7

# Figure 8

**Figure 9**

## Figure 10

# Figure 11

**A**

**B**

# Figure 12

# Figure 13

Figure 14

# Figure 15

Figure 16

# Figure 17

Figure 18

# Figure 19

# Figure 20

# Figure 21

| Alexa 568 | DAPI | Merged |
|:---:|:---:|:---:|
| 250 µm | 250 µm | 250 µm |

# Figure 22

# Figure 23

# Figure 24

**A**

```
     1          11         21         31         41         51         61         71         81         91
1    MNVIAILNHM GVYFKEEPIR ELHRALERLN FQIVYPNDRD DLLKLIENNA RLCGVIFDWD KYNLELCEEI SKMNENLPLY AFANTYSTLD VSLNDLRLQI
101  SFFEYALGAA EDIANKIKQT TDEYINTILP PLTKALFKYV REGKYTFCTP GHMGGTAFQK SPVGSLFYDF FGPNTMKSDI SISVSELGSL LDHSGPHKEA
201  EQYIARVFNA DRSYMVTNGT STANKIVGMY SAPAGSTILI DRNCHKSLTH LMMMSDVTPI YFRPTRNAYG ILGGIPQSEF QHATIAKRVK ETPNATWPVH
301  AVITNSTYDG LLYNTDFIKK TLDVKSIHFD SAWVPYTNFS PIYEGKCGMS GGRVEGKVIY ETQSTHKLLA AFSQASMIHV KGDVNEETFN EAYMMHTTTS
401  PHYGIVASTE TAAAMMKGNA GKRLINGSIE RAIKFRKEIK RLRTESDGWF FDVWQPDHID TTECWPLRSD STWHGFKNID NEHMYLDPIK VTLLTPGMEK
501  DGTMSDFGIP ASIVAKYLDE HGIVVEKTGP YNLLFLFSIG IDKTKALSLL RALTDFKRAF DLNLRVKNML PSLYREDPEF YENMRIQELA QNIHKLIVHH
601  NLPDLMYRAF EVLPTMVMTP YAAFQKELHG MTEEVYLDEM VGRINANMIL PYPPGVPLVM PGEMITEESR PVLEFLQMLC EIGAHYPGFE TDIHGAYRQA
701  DGRYTVKVLK EESKK
```

**B**

```
      1          11         21         31         41         51         61         71         81         91
1     MNVIAILNHM GVYFKEEPIR ELHRALERLN FQIVYPNDRD DLLKLIENNA RLCGVIFDWD KYNLELCEEI SKMNENLPLY AFANTYSTLD VSLNDLRLQI
101   SFFEYALGAA EDIANKIKQT TDEYINTILP PLTKALFKYV REGKYTFCTP GHMGGTAFQK SPVGSLFYDF FGPNTMKSDI SISVSELGSL LDHSGPHKEA
201   EQYIARVFNA DRSYMVTNGT STANKIVGMY SAPAGSTILI DRNCHKSLTH LMMMSDVTPI YFRPTRNAYG ILGGIPQSEF QHATIAKRVK ETPNATWPVH
301   AVITNSTYDG LLYNTDFIKK TLDVKSIHFD SAWVPYTNFS PIYEGKCGMS GGRVEGKVIY ETQSTHKLLA AFSQASMIHV KGDVNEETFN EAYMMHTTTS
401   PHYGIVASTE TAAAMMKGNA GKRLINGSIE RAIKFRKEIK RLRTESDGWF FDVWQPDHID TTECWPLRSD STWHGFKNID NEHMYLDPIK VTLLTPGMEK
501   DGTMSDFGIP ASIVAKYLDE HGIVVEKTGP YNLLFLFSIG IDKTKALSLL RALTDFKRAF DLNLRVKNML PSLYREDPEF YENMRIQELA QNIHKLIVHH
601   NLPDLMYRAF EVLPTMVMTP YAAFQKELHG MTEEVYLDEM VGRINANMIL PYPPGVPLVM PGEMITEESR PVLEFLQMLC EIGAHYPGFE TDIHGAYRQA
701   DGRYTVKVLK EESKKASMDN NPNINECIPY NCLSNPEVEV LGGERIETGY TPIDISLSLT QFLLSEFVPG AGFVLGLVDI IWGIFGPSQW DAFLVQIEQL
801   INQRIEEFAR NQAISRLEGL SNLYQIYAES FREWEADPTN PALREEMRIQ FNDMNSALTT AIPLFAVQNY QVPLLSVYVQ AANLHLSVLR DVSVFGQRWG
901   FDAATINSRY NDLTRLIGNY TDHAVRWYNT GLERVWGPDS RDWIRYNQFR RELTLTVLDI VSLFPNYDSR TYPIRTVSQL TREIYTNPVL ENFDGSFRGS
1001  AQGIEGSIRS PHLMDILNSI TIYTDAHRGE YYWSGHQIMA SPVGFSGPEF TFPLYGTMGN AAPQQRIVAQ LGQGVYRTLS STLYRRPFNI GINNQQLSVL
1101  DGTEFAYGTS SNLPSAVYRK SGTVDSLDEI PPQNNNVPPR QGFSHRLSHV SMFRSGFSNS SVSIIRAPMF SWIHRSAEFN NIIPSSQITQ IPLTKSTNLG
1201  SGTSVVKGPG FTGGDILRRT SPGQISTLRV NITAPLSQRY RVRIRYASTT NLQFHTSIDG RPINQGNFSA TMSSGSNLQS GSFRTVGFTT PFNFSNGSSV
1301  FTLSAHVFNS GNEVYIDRIE FVPAEVTFEA EYDLERAQKA VNELFTSSNQ IGLKTDVTDY HIDQVSNLVE CLSDEFCLDE KKELSEKVKH AKRLSDERNL
1401  LQDPNFRGIN RQLDRGWRGS TDITIQGGDD VFKENYVTLL GTFDECYPTY LYQKIDESKL KAYTRYQLRG YIEDSQDLEI YLIRYNAKHE TVNVPGTGSL
1501  WPLSAPSPIG KCAHHSHHFS LDIDVGCTDL NEDLGVWVIF KIKTQDGHAR LGNLEFLEEK PLVGEALARV KRAEKKWRDK REKLEWETNI VYKEAKESVD
1601  ALFVNSQYDR LQADTNIAMI HAADKRVHSI REAYLPELSV IPGVNAAIFE ELEGRIFTAF SLYDARNVIK NGDFNNGLSC WNVKGHVDVE EQNNHRSVLV
1701  VPEWEAEVSQ EVRVCPGRGY ILRVTAYKEG YGEGCVTIHE IENNTDELKF SNCVEEEVYP NNTVTCNDYT ATQEEYEGTY TSRNRGYDGA YESNSSVPAD
1801
```

# Figure 25

# Figure 26

Figure 27

Figure 28

**Figure 29**

## Figure 30

Figure 31

Figure 32

**Figure 33**

# Figure 34

Figure 35

# Figure 36

Figure 37

# Figure 38

**A.**

| | Cry3Aa/PEI +RNP | Pos3Aa +RNP | Cry3Aa/PEI +Cas9 | Pos3Aa +Cas9 |
|---|---|---|---|---|
| % bound | 83.8 | 48.1 | 8.7 | 86.7 |

**B.**

**Figure 39**

A.

| | PEI3A/RNP (1pmol RNP) | PEI3A/RNP (5pmol RNP) |
|---|---|---|
| % editing | 20.38 | 43.17 |

B.

| PEI3A: (pmol) | 0.5 | 1 | 2 | 5 | 10 |
|---|---|---|---|---|---|
| PEI3A:RNP (molar ratio) | 1:2 | 1:1 | 2:1 | 5:1 | 10:1 |
| % editing | 2.53 | 5.77 | 12.54 | 16.33 | 34.50 |

## EUROPEAN SEARCH REPORT

**Application Number**

EP 25 17 0849

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HEATER BRADLEY S. ET AL: "In Vivo Enzyme Entrapment in a Protein Crystal", JOURNAL OF THE AMERICAN CHEMICAL SOCIETY, vol. 142, no. 22, 14 May 2020 (2020-05-14), pages 9879-9883, XP093316379, ISSN: 0002-7863, DOI: 10.1021/jacs.9b13462 Retrieved from the Internet: URL:https://pubs.acs.org/doi/pdf/10.1021/jacs.9b13462> * the whole document * ----- | 1-17 | INV. C07K14/325 C12N11/00 C12N15/62 C12P13/04 |
| A,D | SUN QIAN ET AL: "Cry3Aa*SpyCatcher Fusion Crystals Produced in Bacteria as Scaffolds for Multienzyme Coimmobilization", BIOCONJUGATE CHEMISTRY, vol. 33, no. 2, 31 January 2022 (2022-01-31), pages 386-396, XP093316862, US ISSN: 1043-1802, DOI: 10.1021/acs.bioconjchem.2c00003 Retrieved from the Internet: URL:https://pubs.acs.org/doi/pdf/10.1021/acs.bioconjchem.2c00003> * the whole document * ----- | 1-17 | |
| | | | TECHNICAL FIELDS SEARCHED (IPC) |
| A | REN SIZHU ET AL: "Co-immobilization multienzyme nanoreactor with co-factor regeneration for conversion of CO2", INTERNATIONAL JOURNAL OF BIOLOGICAL MACROMOLECULES, vol. 155, July 2020 (2020-07), pages 110-118, XP093316408, NL ISSN: 0141-8130, DOI: 10.1016/j.ijbiomac.2020.03.177 * the whole document * ----- | 1-17 | C07K C12N C12P |

-/--

The present search report has been drawn up for all claims

| Place of search | Date of completion of the search | Examiner |
|---|---|---|
| The Hague | 23 September 2025 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 25 17 0849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ANDERSSON M M ET AL: "Protein stabilising effect of polyethyleneimine", JOURNAL OF BIOTECHNOLOGY, ELSEVIER, AMSTERDAM NL, vol. 72, no. 1-2, 11 June 1999 (1999-06-11), pages 21-31, XP004172884, ISSN: 0168-1656, DOI: 10.1016/S0168-1656(99)00050-4 * the whole document * ----- | 1-17 | |
| A | MICHAEL R. SAWAYA ET AL: "Protein crystal structure obtained at 2.9 A resolution from injecting bacterial cells into an X-ray free-electron laser beam", PROCEEDINGS OF THE NATIONAL ACADEMY OF SCIENCES (PNAS), vol. 111, no. 35, 2 September 2014 (2014-09-02), pages 12769-12774, XP055746571, ISSN: 0027-8424, DOI: 10.1073/pnas.1413456111 * the whole document * ----- | 1-17 | |

**TECHNICAL FIELDS SEARCHED (IPC)**

The present search report has been drawn up for all claims

| Place of search | Date of completion of the search | Examiner |
|---|---|---|
| The Hague | 23 September 2025 | Oderwald, Harald |

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63636314
- US 20100322977
- US 20100322977 A
- WO 2020216322 A
- WO 2020211782 A
- WO 2018028371 A
- WO 02086075 A

### Non-patent literature cited in the description

- **PARK et al.** *Appl Environ Microbiol*, 1998, vol. 64, 3932-3938
- **SCHNEPF et al.** *Microbiol Mol Biol Rev*, 1998, vol. 62, 775-806
- **WHITELEY** ; **SCHNEPF**. *Annu Rev Microbiol*, 1986, vol. 40, 549-576
- **NAIR et al.** *PLoS One*, 2015, vol. 10, e0127669
- **BATZER et al.** *Nucleic Acid Res.*, 1991, vol. 19, 5081
- **OHTSUKA et al.** *J. Biol. Chem.*, 1985, vol. 260, 2605-2608
- **ROSSOLINI et al.** *Mol. Cell. Probes*, 1994, vol. 8, 91-98
- **CREIGHTON**. Proteins. W. H. Freeman and Co., 1984
- **SMITH** ; **WATERMAN**. *Adv. Appl. Math.*, 1981, vol. 2, 482
- **NEEDLEMAN** ; **WUNSCH**. *J. Mol. Biol.*, 1970, vol. 48, 443
- **PEARSON** ; **LIPMAN**. *Proc. Nat'l. Acad. Sci. USA*, 1988, vol. 85, 2444
- Current Protocols in Molecular Biology. 1995
- **ALTSCHUL et al.** *J. Mol. Biol.*, 1990, vol. 215, 403-410
- **ALTSCHUL et al.** *Nucleic Acids Res.*, 1977, vol. 25, 3389-3402
- **HENIKOFF** ; **HENIKOFF**. *Proc. Natl. Acad. Sci. USA*, 1989, vol. 89, 10915
- **KARLIN** ; **ALTSCHUL**. *Proc. Nat'l. Acad. Sci. USA*, 1993, vol. 90, 5873-5787
- **SAMBROOK** ; **RUSSELL**. Molecular Cloning, A Laboratory Manual. 2001
- **KRIEGLER**. Gene Transfer and Expression: A Laboratory Manual. 1990
- Current Protocols in Molecular Biology. 1994
- **BEAUCAGE** ; **CARUTHERS**. *Tetrahedron Lett.*, 1981, vol. 22, 1859-1862
- **VAN DEVANTER**. *Nucleic Acids Res.*, 1984, vol. 12, 6159-6168
- **PEARSON** ; **REANIER**. *J. Chrom.*, 1983, vol. 255, 137-149
- **WALLACE et al.** *Gene*, 1981, vol. 16, 21-26
- **COLLEY et al.** *J. Biol. Chem.*, 1989, vol. 264, 17619-17622
- Guide to Protein Purification. Methods in Enzymology. 1990, vol. 182
- **MORRISON**. *J. Bact.*, 1977, vol. 132, 349-351
- **CLARK-CURTISS** ; **CURTISS et al.** Methods in Enzymology. 1983, vol. 101, 347-362
- **PATRA et al.** *Protein Expression and Purification*, 2000, vol. 18, 182-190
- **HERNANDEZ-MONTELONGO et al.** *Mater. Sci. Eng. C.*, 2017, vol. 71, 718-724
- **VICENNATI et al.** *A. Curr. Med. Chem.*, 2008, vol. 15, 2826-2839
- **ROCHE, J. et al.** NADH regenerated using immobilized FDH in a continuously supplied reactor - Application to l-lactate synthesis. *Chemical Engineering Journal*, 2014, vol. 239, 216-225
- **SABA, T. et al.** NADH Regeneration: A Case Study of Pt-Catalyzed NAD+ Reduction with H2.. *ACS Catalysis*, 2021, vol. 11 (1), 283-289
- **ALI, I.** ; **B. SOOMRO** ; **S. OMANOVIC**. Electrochemical regeneration of NADH on a glassy carbon electrode surface: The influence of electrolysis potential.. *Electrochemistry Communications*, 2011, vol. 13 (6), 562-565
- **WANG, X. et al.** Cofactor NAD(P)H Regeneration Inspired by Heterogeneous Pathways. *Chem*, 2017, vol. 2 (5), 621-654
- **VELASCO-LOZANO, S.** ; **A.I. BENÍTEZ-MATEOS** ; **F. LÓPEZ-GALLEGO**. Co-immobilized Phosphorylated Cofactors and Enzymes as Self-Sufficient Heterogeneous Biocatalysts for Chemical Processes.. *Angew Chem Int Ed Engl*, 2017, vol. 56 (3), 771-775

- **JI, X. et al.** Tethering of Nicotinamide Adenine Dinucleotide Inside Hollow Nanofibers for High-Yield Synthesis of Methanol from Carbon Dioxide Catalyzed by Coencapsulated Multienzymes.. *ACS Nano*, 2015, vol. 9 (4), 4600-4610
- **LI, P. et al.** Hierarchically Engineered Mesoporous Metal-Organic Frameworks toward Cell-free Immobilized Enzyme Systems.. *Chem*, 2018, vol. 4 (5), 1022-1034
- **LIANG, J. et al.** *Hierarchically Porous Biocatalytic MOF Microreactor as a Versatile Platform towards Enhanced Multienzyme and Cofactor-Dependent Biocatalysis*, 2021, vol. 60 (10), 5421-5428
- **VELASCO-LOZANO, S.** ; **A.I. BENÍTEZ-MATEOS** ; **F. LÓPEZ-GALLEGO**. *Co-immobilized Phosphorylated Cofactors and Enzymes as Self-Sufficient Heterogeneous Biocatalysts for Chemical Processes.*, 2017, vol. 56 (3), 771-775
- **FU, J. et al.** Multi-enzyme complexes on DNA scaffolds capable of substrate channelling with an artificial swinging arm.. *Nat Nanotechnol*, 2014, vol. 9 (7), 531-6
- **HARTLEY, C.J. et al.** Engineered enzymes that retain and regenerate their cofactors enable continuous-flow biocatalysis.. *Nature Catalysis*, 2019, vol. 2 (11), 1006-1015
- **HEATER, B.S. et al.** Directed evolution of a genetically encoded immobilized lipase for the efficient production of biodiesel from waste cooking oil.. *Biotechnol Biofuels*, 2019, vol. 12, 165
- **HEATER, B.S. et al.** In Vivo Enzyme Entrapment in a Protein Crystal.. *Journal of the American Chemical Society*, 2020, vol. 142 (22), 9879-9883
- **EMSLEY, P.** ; **K. COWTAN**. Coot: model-building tools for molecular graphics.. *Acta Crystallogr D Biol Crystallogr*, 2004, vol. 60 (12,1), 2126-32
- **YANG, Z.** ; **M.M.M. LEE** ; **M.K. CHAN**. Efficient intracellular delivery of p53 protein by engineered protein crystals restores tumor suppressing function in vivo. *Biomaterials*, 2021, vol. 271, 120759
- **MOUNSEF, J.R. et al.** A simple method for the separation of Bacillus thuringiensis spores and crystals.. *J Microbiol Methods*, 2014, vol. 107, 147-9
- **UNGARO, F. et al.** Spectrophotometric determination of polyethylenimine in the presence of an oligonucleotide for the characterization of controlled release formulations.. *Journal of Pharmaceutical and Biomedical Analysis*, 2003, vol. 31 (1), 143-149
- **WEN, T. et al.** A facile, sensitive, and rapid spectrophotometric method for copper(II) ion detection in aqueous media using polyethyleneimine. *Arabian Journal of Chemistry*, 2017, vol. 10, S1680-S1685
- **OTWINOWSKI, Z.** ; **W. MINOR**. Processing of X-ray diffraction data collected in oscillation mode.. *Methods Enzymol*, 1997, vol. 276, 307-26
- **MCCOY, A.J. et al.** Phaser crystallographic software.. *J Appl Crystallogr*, 2007, vol. 40 (4), 658-674
- **WINN, M.D.** An overview of the CCP4 project in protein crystallography: an example of a collaborative project.. *J Synchrotron Radiat*, 2003, vol. 10 (1), 23-5
- **MURSHUDOV, GN. et al.** REFMAC5 for the refinement of macromolecular crystal structures.. *Acta Crystallogr D Biol Crystallogr*, 2011, vol. 67 (4), 355-67
- **LIEBSCHNER, D. et al.** Macromolecular structure determination using X-rays, neutrons and electrons: recent developments in Phenix.. *Acta Crystallogr D Struct Biol*, 2019, vol. 75 (10), 861-877
- **GOSCIANSKA, J.** ; **A. OLEJNIK** ; **I. NOWAK**. APTES-functionalized mesoporous silica as a vehicle for antipyrine - adsorption and release studies.. *Colloids and Surfaces A: Physicochemical and Engineering Aspects*, 2017, vol. 533, 187-196
- **BOURKAIB, M.C. et al.** APTES modified SBA15 and meso-macro silica materials for the immobilization of aminoacylases from Streptomyces ambofaciens.. *Microporous and Mesoporous Materials*, 2021, vol. 323, 111226
- **NAIR, M.S. et al.** Cry Protein Crystals: A Novel Platform for Protein Delivery.. *PLOS ONE*, 2015, vol. 10 (6), e0127669
- **SEMENOVA, A. et al.** Copper-Binding Properties of Polyethylenimine-Silica Nanocomposite Particles.. *Langmuir*, 2022, vol. 38 (34), 10585-10600
- **LEE, K.Y** ; **M.C. PETERS** ; **D.J. MOONEY**. *Controlled Drug Delivery from Polymers by Mechanical Signals*, 2001, vol. 13 (11), 837-839
- **LIN, H.-Y** ; **J.L. THOMAS**. PEG-Lipids and Oligo(ethylene glycol) Surfactants Enhance the Ultrasonic Permeabilizability of Liposomes.. *Langmuir*, 2003, vol. 19 (4), 1098-1105
- **KÜNG, R. et al.** *Mechanochemical Release of Non-Covalently Bound Guests from a Polymer-Decorated Supramolecular Cage.*, 2021, vol. 60 (24), 13626-13630
- **WHITE, S.R. et al.** Autonomic healing of polymer composites.. *Nature*, 2001, vol. 409 (6822), 794-7
- **LI, Z.A. et al.** *Highly Sensitive Built-In Strain Sensors for Polymer Composites: Fluorescence Turn-On Response through Mechanochemical Activation*, 2016, vol. 28 (31), 6592-6597
- **IMATO, K. et al.** Fluorescent supramolecular mechanophores based on charge-transfer interactions.. *Chemical Communications*, 2020, vol. 56 (57), 7937-7940
- **AERTS, A. et al.** *Pyranine Based Ion-Paired Complex as a Mechanophore in Polyurethanes.*, 2021, vol. 42 (1), 2000476

- **MICHAEL, P.** ; **W.H. BINDER**. *AMechanochemically Triggered "Click" Catalyst.*, 2015, vol. 54 (47), 13918-13922
- **GROOTE, R. et al.** Unfolding and Mechanochemical Scission of Supramolecular Polymers Containing a Metal-Ligand Coordination Bond.. *Macromolecules*, 2011, vol. 44 (23), 9187-9195
- **PIERMATTEI, A.** ; **S. KARTHIKEYAN** ; **R.P. SIJBESMA**. Activating catalysts with mechanical force.. *Nature Chemistry*, 2009, vol. 1 (2), 133-137
- **MICHAEL, P.** ; **S.K. SHEIDAEE MEHR** ; **W.H. BINDER**. *Synthesis and characterization of polymer linked copper(I) bis(N-heterocyclic carbene) mechanocatalysts.*, 2017, vol. 55 (23), 3893-3907
- **SHAN, C.A. et al.** Effects of nano-pore system characteristics on CH4 adsorption capacity in anthracite.. *Frontiers of Earth Science*, 2019, vol. 13 (1), 75-91
- **LIU, W. et al.** Effect of Pore Size Distribution and Amination on Adsorption Capacities of Polymeric Adsorbents.. *Molecules*, 2021, vol. 26 (17)
- **SURESH KUMAR, P. et al.** Effect of pore size distribution and particle size of porous metal oxides on phosphate adsorption capacity and kinetics.. *Chemical Engineering Journal*, 2019, vol. 358, 160-169
- **FERRATI, S. et al.** Leveraging nanochannels for universal, zero-order drug delivery in vivo.. *Journal of Controlled Release*, 2013, vol. 172 (3), 1011-1019
- **ZHANG, B. et al.** New insights into the effects of porosity, pore length, pore shape and pore alignment on drug release from extrusionbased additive manufactured pharmaceuticals.. *Additive Manufacturing*, 2021, vol. 46, 102196
- **GAO, Y. et al.** Effect of pore size of three-dimensionally ordered macroporous chitosan-silica matrix on solubility, drug release, and oral bioavailability of loaded-nimodipine.. *Drug Dev Ind Pharm*, 2016, vol. 42 (3), 464-72
- **LI, J. et al.** Effects of pore size on in vitro and in vivo anticancer efficacies of mesoporous silica nanoparticles.. *RSC Advances*, 2018, vol. 8 (43), 24633-24640
- **SUN, Q. et al.** Cry3Aa*SpyCatcher Fusion Crystals Produced in Bacteria as Scaffolds for Multienzyme Coimmobilization.. *Bioconjugate Chemistry*, 2022, vol. 33 (2), 386-396
- **SHELDON, R.A.** ; **S. VAN PELT**. Enzyme immobilisation in biocatalysis: why, what and how.. *Chem Soc Rev*, 2013, vol. 42 (15), 6223-35
- **ROCHA-MARTÍN, J. et al.** Rational Co-Immobilization of Bi-Enzyme Cascades on Porous Supports and their Applications. *Bio-Redox Reactions with In Situ Recycling of Soluble Cofactors*, 2012, vol. 4 (9), 1279-1288
- **EL-ZAHAB, B.** ; **D. DONNELLY** ; **P. WANG**. Particle-tethered NADH for production of methanol from CO(2) catalyzed by coimmobilized enzymes.. *Biotechnol Bioeng*, 2008, vol. 99 (3), 508-14
- **DICOSIMO, R. et al.** Industrial use of immobilized enzymes.. *Chem Soc Rev*, 2013, vol. 42 (15), 6437-74
- **JI, X. et al.** Enabling multi-enzyme biocatalysis using coaxial-electrospun hollow nanofibers: redesign of artificial cells.. *J Mater Chem B*, 2014, vol. 2 (2), 181-190
- **BOLIVAR, J.M. et al.** Modulation of the distribution of small proteins within porous matrixes by smart-control of the immobilization rate.. *J Biotechnol*, 2011, vol. 155 (4), 412-20
- **BENÍTEZ-MATEOS, A.I. et al.** Design of the Enzyme-Carrier Interface to Overcome the O2 and NADH Mass Transfer Limitations of an Immobilized Flavin Oxidase.. *ACS Applied Materials & Interfaces*, 2020, vol. 12 (50), 56027-56038
- **CANTONE, S. et al.** Efficient immobilisation of industrial biocatalysts: criteria and constraints for the selection of organic polymeric carriers and immobilisation methods.. *Chemical Society Reviews*, 2013, vol. 42 (15), 6262-6276
- **LIESE, A.** ; **L. HILTERHAUS**. Evaluation of immobilized enzymes for industrial applications.. *Chemical Society Reviews*, 2013, vol. 42 (15), 6236-6249
- **ZHOU, N. et al.** Self-sufficient biocatalysts constructed using chitin-based microspheres.. *Chemical Engineering Journal*, 2023, vol. 459, 141660
- **WANG, Z. et al.** Durable cofactor immobilization in sol-gel bio-composite thin films for reagentless biosensors and bioreactors using dehydrogenases.. *Biosensors and Bioelectronics*, 2012, vol. 32 (1), 111-117
- **ORREGO, A.H. et al.** Self-sufficient asymmetric reduction of β-ketoesters catalysed by a novel and robust thermophilic alcohol dehydrogenase co-immobilised with NADH.. *Catal Sci Technol*, 2021, vol. 11 (9), 3217-3230
- **LI, D.** ; **XIONG, Q.** ; **LIANG, L.** ; **DUAN, H.** Multienzyme nanoassemblies: from rational design to biomedical applications.. *Biomater. Sci.*, 2021, vol. 9, 7323-7342
- **BUGADA, L. F.** ; **SMITH, M. R.** ; **WEN, F.** Engineering spatially organized multienzyme assemblies for complex chemical transformation. *ACS Catal.*, 2018, vol. 8, 7898-7906
- **ADRIO, J. L.** ; **DEMAIN, A. L.** Microbial enzymes: tools for biotechnological processes.. *Biomolecules*, 2014, vol. 4, 117-139
- **SHELDON, R. A.** ; **BASSO, A.** ; **BRADY, D.** New frontiers in enzyme immobilisation: robust biocatalysts for a circular bio-based economy.. *Chem. Soc. Rev.*, 2021, vol. 50, 5850-5862

- **ARANA-PEÑA, S.** ; **CARBALLARES, D.** ; **MORELLON-STERLLING, R.** ; **BERENGUER-MURCIA, Á.** ; **ALCÁNTARA, A. R.** ; **RODRIGUES, R. C.** ; **FERNANDEZ-LAFUENTE, R.** Enzyme co-immobilization: Always the biocatalyst designers' choice... or not?. *Biotechnol. Adv.*, 2021, vol. 51, 107584

- **SANTIAGO-ARCOS, J.** ; **VELASCO-LOZANO, S.** ; **LÓPEZ-GALLEGO, F.** Multienzyme coimmobilization on triheterofunctional supports.. *Biomacromolecules*, 2023, vol. 24, 929-942

- **ZHANG, G** ; **QUIN, M. B.** ; **SCHMIDT-DANNERT, C.** Self-assembling protein scaffold system for easy in vitro coimmobilization of biocatalytic cascade enzymes.. *ACS Catal.*, 2018, vol. 8, 5611-5620

- **RICCA, E.** ; **BRUCHER, B.** ; **SCHRITTWIESER, J. H.** Multi- enzymatic cascade reactions: overview and perspectives.. *Adv. Synth. Catal.*, 2011, vol. 353, 2239-2262

- **SCHNEIDER, J.** ; **WENDISCH, V. F.** Putrescine production by engineered Corynebacterium glutamicum.. *Appl. Microbiol. Biotechnol.*, 2010, vol. 88, 859-868

- **HANDA, A. K.** ; **FATIMA, T.** ; **MATTOO, A. K.** Polyamines: bio-molecules with diverse functions in plant and human health and disease.. *Front. Chem.*, 2018, vol. 6

- **CHOI, H.** ; **KYEONG, H.-H.** ; **CHOI, J. M.** ; **KIM, H.-S.** Rational design of ornithine decarboxylase with high catalytic activity for the production of putrescine.. *Appl. Microbiol. Biotechnol.*, 2014, vol. 98, 7483-7490

- **CHUNG, H.** ; **YANG, J. E.** ; **HA, J. Y** ; **CHAE, T. U.** ; **SHIN, J. H.** ; **GUSTAVSSON, M.** ; **LEE, S. Y.** Bio-based production of monomers and polymers by metabolically engineered microorganisms.. *Curr. Opin. Biotechnol.*, 2015, vol. 36, 73-84

- **LI, G H.** ; **HUANG, D. X.** ; **WANG, L.** ; **DENG, Y.** Highly efficient whole-cell biosynthesis of putrescine by recombinant Escherichia coli.. *Biochem. Eng. J.*, 2021, vol. 166

- **ROERDINK, E.** ; **WARNIER, J. M. M.** Preparation and properties of high molar mass nylon-4, 6: a new development in nylon polymers.. *Polymer*, 1985, vol. 26, 1582-1588

- **SHAKIBA, M.** ; **REZVANI, G** ; **KHOSRAVI, F.** ; **JOUYBAR, S.** ; **BIGHAM, A.** ; **ZARE, M.** ; **ABDOUSS, M.** ; **MOAREF, R.** ; **RAMAKRISHNA, S.** Nylon-A material introduction and overview for biomedical applications.. *Polym. Adv. Technol.*, 2021, vol. 32, 16

- **QIAN, Z. G** ; **XIA, X. X.** ; **LEE, S. Y.** Metabolic engineering of Escherichia coli for the production of putrescine: a four carbon diamine.. *Biotechnol. Bioeng.*, 2009, vol. 104, 651-662

- **SANDERS, J.** ; **SCOTT, E.** ; **WEUSTHUIS, R.** ; **MOOIBROEK, H.** Bio- refinery as the bio- inspired process to bulk chemicals.. *Macromol. Biosci.*, 2007, vol. 7, 105-117

- **KONG, C.-C.** ; **WEI, X.** ; **LIU, G-L.** ; **CHI, Z.-M.** ; **CHI, Z.** Metabolic engineering of Aureobasidium melanogenum for the overproduction of putrescine by improved L-ornithine biosynthesis.. *Microbiol. Res.*, 2022, vol. 260, 127041

- **LI, L.** ; **ZOU, D.** ; **JI, A.** ; **HE, Y.** ; **LIU, Y.** ; **DENG, Y.** ; **CHEN, S.** ; **WEI, X.** Multilevel metabolic engineering of Bacillus amyloliquefaciens for production of the platform chemical putrescine from sustainable biomass hydrolysates.. *ACS Sustain. Chem. Eng.*, 2020, vol. 8, 2147-2157

- **THONGBHUBATE, K.** ; **IRIE, K.** ; **SAKAI, Y** ; **ITOH, A.** ; **SUZUKI, H.** Improvement of putrescine production through the arginine decarboxylase pathway in Escherichia coli K-12.. *AMB Express*, 2021, vol. 11

- **HUI, H.** ; **BAI, Y.** ; **FAN, T.** ; **ZHENG, X.** ; **CAI, Y.** Biosynthesis of putrescine from L-arginine using engineered Escherichia coli whole cells.. *Catalysts*, 2020, vol. 10, 947

- **YANG, S. C.** ; **TING, W. W.** ; **NG, I. S.** Effective whole cell biotransformation of arginine to a four-carbon diamine putrescine using engineered Escherichia coli.. *Biochem. Eng. J.*, 2022, vol. 185

- **YANG, F. Y** ; **QIU, K. L.** ; **ZHU, Y. C.** ; **ZHANG, X.** ; **YANG, T. W.** ; **YI, G F.** ; **XU, M. J.** ; **RAO, Z. M.** Production of putrescine in metabolic engineering Corynebacterium crenatum by mixed sugar fermentation.. *ACS Sustain. Chem. Eng.*, 2022, vol. 10, 14407-14416

- **SCHNEIDER, J.** ; **EBERHARDT, D.** ; **WENDISCH, V. F.** Improving putrescine production by Corynebacterium glutamicum by fine-tuning ornithine transcarbamoylase activity using a plasmid addiction system.. *Appl. Microbiol. Biotechnol.*, 2012, vol. 95, 169-178

- **BOLIVAR, J. M.** ; **WOODLEY, J. M.** ; **FERNANDEZ-LAFUENTE, R.** Is enzyme immobilization a mature discipline? Some critical considerations to capitalize on the benefits of immobilization.. *Chem. Soc. Rev.*, 2022

- **RODRIGUES, R. C.** ; **BERENGUER-MURCIA, Á.** ; **CARBALLARES, D.** ; **MORELLON-STERLING, R.** ; **FERNANDEZ-LAFUENTE, R.** Stabilization of enzymes via immobilization: Multipoint covalent attachment and other stabilization strategies.. *Biotechnol. Adv.*, 2021, vol. 52, 107821

- **MATEO, C.** ; **PALOMO, J. M.** ; **FERNANDEZ-LORENTE, G** ; **GUISAN, J. M.** ; **FERNANDEZ-LAFUENTE, R.** Improvement of enzyme activity, stability and selectivity via immobilization techniques.. *Enzyme Microb. Technol.*, 2007, vol. 40, 1451-1463

- **RODRIGUES, R. C.** ; **ORTIZ, C.** ; **BERENGUER-MURCIA, Á.** ; **TORRES, R.** ; **FERNÁNDEZ-LAFUENTE, R.** Modifying enzyme activity and selectivity by immobilization.. *Chem. Soc. Rev.*, 2013, vol. 42, 6290-6307

- **GUISAN, J. M.** ; **FERNANDEZ-LORENTE, G** ; **ROCHA-MARTIN, J.** ; **MORENO-GAMERO, D.** Enzyme immobilization strategies for the design of robust and efficient biocatalysts.. *Curr. Opin. Green Sustain. Chem.*, 2022, vol. 35, 100593

- **MAGHRABY, Y. R.** ; **EL-SHABASY, R. M.** ; **IBRAHIM, A. H.** ; **AZZAZY, H. M. E.-S.** Enzyme immobilization technologies and industrial applications.. *ACS Omega*, 2023, vol. 8, 5184-5196

- **OLCUCU, G** ; **KLAUS, O.** ; **JAEGER, K.-E.** ; **DREPPER, T.** ; **KRAUSS, U.** Emerging solutions for in vivo biocatalyst immobilization: tailor-made catalysts for industrial biocatalysis.. *ACS Sustain. Chem. Eng.*, 2021, vol. 9, 8919-8945

- **LI, J.** ; **CARROLL, J.** ; **ELLAR, D. J.** Crystal structure of insecticidal δ-endotoxin from Bacillus thuringiensis at 2.5 Å resolution.. *Nature*, 1991, vol. 353, 815-821

- **SCHNEPF, E.** ; **CRICKMORE, N.** ; **VAN RIE, J.** ; **LERECLUS, D.** ; **BAUM, J.** ; **FEITELSON, J.** ; **ZEIGLER, D. R.** ; **DEAN, D.** Bacillus thuringiensis and its pesticidal crystal proteins.. *Microbiol. Mol. Biol. Rev.*, 1998, vol. 62, 775-806

- **LOUTFI, H.** ; **FAYAD, N.** ; **PELLEN, F.** ; **LE JEUNE, B.** ; **CHAKROUN, M.** ; **BENFARHAT, D.** ; **LTEIF, R.** ; **KALLASSY, M.** ; **LE BRUN, G** ; **ABBOUD, M.** Morphological study of Bacillus thuringiensis crystals and spores.. *Appl. Sci.*, 2021, vol. 11, 155

- **HEATER, B. S.** ; **LEE, M. M.** ; **CHAN, M. K.** Direct production of a genetically-encoded immobilized biodiesel catalyst.. *Sci. Rep.*, 2018, vol. 8, 1-10

- **HEATER, B. S.** ; **CHAN, W. S.** ; **LEE, M. M.** ; **CHAN, M. K.** Directed evolution of a genetically encoded immobilized lipase for the efficient production of biodiesel from waste cooking oil.. *Biotechnol. Biofuels*, 2019, vol. 12, 1-14

- **SUN, Q.** ; **HEATER, B. S.** ; **LI, T. L.** ; **YE, W.** ; **GUO, Z.** ; **CHAN, M. K.** Cry3Aa* SpyCatcher fusion crystals produced in bacteria as scaffolds for multienzyme coimmobilization.. *Bioconjug. Chem.*, 2022, vol. 33, 386-396

- **NAIR, M. S.** ; **LEE, M. M.** ; **BONNEGARDE-BERNARD, A.** ; **WALLACE, J. A.** ; **DEAN, D. H.** ; **OSTROWSKI, M. C.** ; **BURRY, R. W.** ; **BOYAKA, P. N.** ; **CHAN, M. K.** Cry protein crystals: a novel platform for protein delivery.. *PLoS ONE*, 2015, vol. 10, e0127669

- **SUN, Q.** ; **CHENG, S. W.** ; **CHEUNG, K.** ; **LEE, M. M.** ; **CHAN, M. K.** Cry protein crystal-immobilized metallothioneins for bioremediation of heavy metals from water.. *Crystals*, 2019, vol. 9

- **PEÑA-CARDEÑA, A.** ; **GRANDE, R.** ; **SÁNCHEZ, J.** ; **TABASHNIK, B. E.** ; **BRAVO, A.** ; **SOBERÓN, M.** ; **GÓMEZ, I.** The C-terminal protoxin region of Bacillus thuringiensis Cry1Ab toxin has a functional role in binding to GPI-anchored receptors in the insect midgut.. *J. Biol. Chem.*, 2018, vol. 293, 20263-20272

- **OEDA, K.** ; **INOUYE, K.** ; **IBUCHI, Y.** ; **OSHIE, K.** ; **SHIMIZU, M.** ; **NAKAMURA, K.** ; **NISHIOKA, R.** ; **TAKADA, Y.** ; **OHKAWA, H.** Formation of crystals of the insecticidal proteins of Bacillus thuringiensis subsp. aizawai IPL7 in Escherichia coli.. *J. Bacteriol.*, 1989, vol. 171, 3568-3571

- Study on Escherichia coli-expressed Cry1Ab towards the development of a targeted protein delivery system.. **LEE, T. F.** Master's thesis. The Chinese University of Hong Kong, 2017

- **KUDO, K.** ; **MOCHIZUKI, M.** ; **KIRIYAMA, S.** ; **WATANABE, M.** ; **HIRAMI, M.** Studies on the structure and properties of nylon 46 fiber. I. Dimensional stability.. *J. Appl. Polym. Sci.*, 1994, vol. 52, 861-867

- **BRADFORD, M. M.** A rapid and sensitive method for the quantitation of microgram quantities of protein utilizing the principle of protein-dye binding.. *Anal. Biochem.*, 1976, vol. 72, 248-254

- **RAHMATULLAH, M.** ; **BOYDE, T. R. C.** Improvements in the determination of urea using diacetyl monoxime; methods with and without deproteinisation.. *Clin. Chim. Acta*, 1980, vol. 107, 3-9

- **CHINARD, F. P.** Photometric estimation of proline and ornithine.. *J. Biol. Chem.*, 1952, vol. 199, 91-95

- **PEREIRA, V.** ; **PONTES, M.** ; **CÂMARA, J.** ; **MARQUES, J. C.** Simultaneous analysis of free amino acids and biogenic amines in honey and wine samples using in loop orthophthalaldeyde derivatization procedure.. *J. Chromatogr. A*, 2008, vol. 1189, 435-443

- Cry3Aa-inspired enzyme immobilization: from biocatalysis to therapeutics.. **HEATER, B. S.** Doctoral thesis. The Chinese University of Hong Kong, 2018

- **HEATER, B. S.** ; **YANG, Z.** ; **LEE, M. M.** ; **CHAN, M. K.** In Vivo Enzyme Entrapment in a Protein Crystal.. *J. Am. Chem. Soc.*, 2020, vol. 142, 9879-9883

- **ALARCÓN, R.** ; **ORELLANA, M. S.** ; **NEIRA, B.** ; **URIBE, E.** ; **GARCIA, J. R.** ; **CARVAJAL, N.** Mutational analysis of substrate recognition by human arginase type I- agmatinase activity of the N130D variant.. *FEBS J.*, 2006, vol. 273, 5625-5631

- **MASHINO, T.** ; **FRIDOVICH, I.** Effects of urea and trimethylamine-N-oxide on enzyme activity and stability.. *Arch. Biochem. Biophys.*, 1987, vol. 258, 356-360

- **CHILAKA, F. C.** ; **NWAMBA, C. O.** Kinetic analysis of urea-inactivation of β-galactosidase in the presence of galactose.. *J. Enzyme Inhib. Med. Chem.*, 2008, vol. 23, 7-15

- **GROBBEN, Y.** ; **UITDEHAAG, J. C. M.** ; **WILLEMSEN-SEEGERS, N.** ; **TABAK, W. W. A.** ; **DE MAN, J.** ; **BUIJSMAN, R. C.** ; **ZAMAN, G J. R.** Structural insights into human Arginase-1 pH dependence and its inhibition by the small molecule inhibitor CB-1158.. *J. Struct. Biol.: X*, 2020, vol. 4

- **STONE, E. M.** ; **GLAZER, E. S.** ; **CHANTRANUPONG, L.** ; **CHERUKURI, P.** ; **BREECE, R. M.** ; **TIERNEY, D. L.** ; **CURLEY, S. A.** ; **IVERSON, B. L.** ; **GEORGIOU, G.** Replacing Mn2+ with Co2+ in human arginase I enhances cytotoxicity toward L-arginine auxotrophic cancer cell lines.. *ACS Chem. Biol.*, 2010, vol. 5, 333-342

- **BASSO, A.** ; **SERBAN, S.** Industrial applications of immobilized enzyme-A review.. *Mol. Catal.*, 2019, vol. 479, 110607

- **ZHANG, Z.** ; **HUANG, K.** ; **LIU, Z.** Synthesis of high molecular weight nylon 46 in supercritical carbon dioxide.. *Macromolecules*, 2011, vol. 44, 820-825

- **YAMANOBE, T.** ; **KURIHARA, Y.** ; **UEHARA, H.** ; **KOMOTO, T.** Structure and characterization of nylon 46.. *J. Mol. Struct.*, 2007, vol. 829, 80-87

- **CONG, P.** ; **XIANG, F.** ; **LIU, X.** ; **LI, T.** Morphology and microstructure of polyamide 46 wear debris and transfer film: In relation to wear mechanisms.. *Wear*, 2008, vol. 265, 1100-1105

- **B. BINAY** ; **D. ALAGÖZ** ; **D. YILDIRIM** ; **A. ÇELIK** ; **S. S. TÜKEL.** Highly Stable and Reusable Immobilized Formate Dehydrogenases: Promising Biocatalysts for In Situ Regeneration of NADH. *Beilstein J. Org. Chem.*, 2016, vol. 12, 271-277

- **S. KIM** ; **K. KWON** ; **G TAE** ; **I. KWON.** Nano-Entrapping Multiple Oxidoreductases and Cofactor for All-In-One Nanoreactors. *ACS Sustain. Chem. Eng.*, 2021, vol. 9, 6741-6747

- **X. JI** ; **Z. SU** ; **P. WANG** ; **G MA** ; **S. ZHANG.** Integration of Artificial Photosynthesis System for Enhanced Electronic Energy-Transfer Efficacy: A Case Study for Solar-Energy Driven Bioconversion of Carbon Dioxide to Methanol. *Small.*, 2016, vol. 12, 4753-4762

- **J. GRUNWALD** ; **B. WIRZ** ; **M. P. SCOLLAR** ; **A. M. KLIBANOV.** Asymmetric Oxidoreductions Catalyzed by Alcohol Dehydrogenase in Organic Solvents. *J. Am. Chem. Soc.*, 1986, vol. 108, 6732-6734

- **J. WYKES** ; **P. DUNNILL** ; **M. J. B. LILLY.** Bioengineering, Cofactor Recycling in an Enzyme Reactor: A Comparison Using Free and Immobilized Dehydrogenases with Free and Immobilized NAD. *Bioeng.*, 1975, vol. 17, 51-68

- **C.-H. WONG.** Nicotinamide Cofactor-Requiring Enzymatic Synthesis in Organic Solvent-Water Biphasic Systems. *J. Org. Chem.*, 1987, vol. 29, 197-208

- **P. WANG** ; **G MA** ; **L. LIAO** ; **F. GAO.** Construction of Multienzyme Bioactivesystems. *J. Biotechnol.*, 2005, 22-41

- **S. WU** ; **R. SNAJDROVA** ; **J. C. MOORE** ; **K. BALDENIUS** ; **U. T. BORNSCHEUER.** Biocatalysis: Enzymatic Synthesis for Industrial Applications. *Catalysts.*, 2021, vol. 60, 88-119

- **S. MORDHORST** ; **J. N. ANDEXER.** Round, Round We Go - Strategies for Enzymatic Cofactor Regeneration. *Nat. Prod. Rep.*, 2020, vol. 37, 1316-1333

- **T. MONTES** ; **V. GRAZÚ** ; **I. MANSO** ; **B. GALÁN** ; **F. LÓPEZ-GALLEGO** ; **R. GONZÁLEZ** ; **J. A. HERMOSO** ; **J. L. GARCIA** ; **J. M. GUISÁN** ; **R. FERNÁNDEZ-LAFUENTE.** Improved Stabilization of Genetically Modified Penicillin G Acylase in the Presence of Organic Cosolvents by Co- Immobilization of the Enzyme with Polyethyleneimine. *Adv. Synth. Catal.*, 2007, vol. 349, 459-464

- **A. I. BENÍTEZ-MATEOS** ; **E. SAN SEBASTIAN** ; **N. RÍOS-LOMBARDÍA** ; **F. MORÍS** ; **J. GONZÁLEZ-SABIN** ; **F. LÓPEZ-GALLEGO.** symmetric Reduction of Prochiral Ketones by Using Self-Sufficient Heterogeneous Biocatalysts Based on NADPH-Dependent Ketoreductases. *Chem. Eur. J.*, 2017, vol. 23, 16843-16852

- **E. S. DA SILVA** ; **V. GÓMEZ-VALLEJO** ; **J. LLOP** ; **F. LÓPEZ-GALLEGO.** Efficient nitrogen-13 radiochemistry catalyzed by a highly stable immobilized biocatalyst. *Catal. Sci. Technol.*, 2015, vol. 5, 2705-2713

- **A. I. BENÍTEZ-MATEOS** ; **C. HUBER** ; **B. NIDETZKY** ; **J. M. BOLIVAR** ; **F. LÓPEZ-GALLEGO.** Design of the Enzyme-Carrier Interface to Overcome the O2 and NADH Mass Transfer Limitations of an Immobilized Flavin Oxidase. *ACS Appl. Mater. Interfaces.*, 2020, vol. 12, 56027-56038

- **S. VELASCO-LOZANO** ; **A. I. BENÍTEZ-MATEOS** ; **F. LÓPEZ-GALLEGO.** Co-immobilized Phosphorylated Cofactors and Enzymes as Self-Sufficient Heterogeneous Biocatalysts for Chemical Processes. *Angew. Chem. Int. Ed. Engl.*, 2017, vol. 56, 771-775

- **R. YEKTA** ; **X. XIONG** ; **J. LI** ; **B. S. HEATER** ; **M. M. LEE** ; **M. K. CHAN.** Mechanoresponsive Protein Crystals for NADH Recycling in Multicycle Enzyme Reactions. *J. Am. Chem. Soc.*, 2024, vol. 146, 18817-18822

- **A. ALEHOSSEINI ; E.-M. GOMEZ DEL PULGAR ; M. J. FABRA ; L. G GÓMEZ-MASCARAQUE ; A. BENÍTEZ-PÁEZ ; M. SARABI-JAMAB ; B. GHOR-ANI ; A. LOPEZ-RUBIO**. Agarose-Based Freeze-Dried Capsules Prepared By The Oil-Induced Biphasic Hydrogel Particle Formation Approach For The Protection Of Sensitive Probiotic Bacteria. *Food. Hydrocoll.*, 2019, vol. 87, 487-496
- **N. PERNODET ; M. MAALOUM ; B. TINLAND**. Pore Size of Agarose Gels by Atomic Force Microscopy. *Electrophoresis*, 1997, vol. 18, 55-58
- **N. C. STELLWAGEN**. Electrophoresis of DNA in Agarose Gels, Polyacrylamide Gels and in Free Solution. *Electrophoresis*, 2009, vol. 30, 188-195
- **J. NARAYANAN ; J.-Y. XIONG ; X. Y. LIU**. Determination of Agarose Gel Pore Size: Absorbance Measurements vis a vis Other Techniques. *J. Phys. Conf. Ser.*, 2006, vol. 1, 120-128
- **L. BAYNE ; R. V. ULIJN ; P. J. HALLING**. Effect of Pore Size on the Performance of Immobilised Enzymes. *Chem. Soc. Rev.*, 2013, vol. 42, 9000-9010
- **H. CHOE ; J. C. JOO ; D. H. CHO ; M. H. KIM ; S. H. LEE ; K. D. JUNG ; Y. H. KIM**. Efficient CO2-Reducing Activity of NAD-Dependent Formate Dehydrogenase from Thiobacillus sp. KNK65MA for Formate Production from CO2 Gas. *PLoS One.*, 2014, vol. 9, 103-111
- **Y. H. ZHU ; C. Y. LIU ; S. CAI ; L. B. GUO ; I. W. KIM ; V. C. KALIA ; J. K. LEE ; Y. W. ZHANG**. Cloning, Expression and Characterization of a Highly Active Alcohol Dehydrogenase for Production of Ethyl (S)-4-Chloro-3-Hydroxybutyrate, Indian.. *J. Microbiol.*, 2019, vol. 59, 225-233
- **J.-Y. HE ; L.-M. ZHOU ; P. WANG ; L. ZU**. Microbial Reduction of Ethyl Acetoacetate to Ethyl (R)-3-Hydroxybutyrate in an Ionic Liquid Containing System, Process.. *Biochem.*, 2009, vol. 44, 316-321
- **E. DIAMANTI ; S. VELASCO-LOZANO ; D. GRA-JALES-HERNÁNDEZ ; A. H. ORREGO ; D. DI SILVIO ; J. M. FRAILE ; F. LÓPEZ-GALLEGO**. Self-Sufficient Heterogeneous Biocatalysis through Boronic Acid-Diol Complexation of Adenylated Cofactors, ACS Sustain.. *Chem. Eng.*, 2023, vol. 11, 14409-14421
- **C. J. HARTLEY ; C. C. WILLIAMS ; J. A. SCOBLE ; Q. I. CHURCHES ; A. NORTH ; N. G FRENCH ; T. NEBL ; G COIA ; A. C. WARDEN ; G SIMPSON**. Engineered Enzymes that Retain and Regenerate their Cofactors Enable Continuous-Flow Biocatalysis. *Nat. Catal.*, 2019, vol. 2, 1006-1015
- **W. WEI ; R. ETTELAIE ; X. ZHANG ; M. FAN ; Y. DONG ; Z. LI ; H. YANG**. Co-compartmentalization of Enzymes and Cofactors within Pickering Emulsion Droplets for Continuous-Flow Catalysis. *Angew. Chem. Int. Ed. Engl.*, 2022, vol. 61, e202211912
- **T. PESCHKE ; P. BITTERWOLF ; S. GALLUS ; Y. HU ; C. OELSCHLAEGER ; N. WILLENBACHER ; K. S. RABE ; C. M. NIEMEYER**. Self-Assembling All-Enzyme Hydrogels for Flow Biocatalysis. *Angew. Chem., Int. Ed. Engl.*, 2018, vol. 57, 17028-17032
- **Q. CHEN ; Y. WANG ; G LUO**. Recycling of Cofactors in Crude Enzyme Hydrogels as Co-immobilized Heterogeneous Biocatalysts for Continuous-Flow Asymmetric Reduction of Ketones. *ChemSusChem*, 2023, vol. 16, e202201654
- **J. MI ; S. LIU ; Y. DU ; H. QI ; L. ZHANG**. Cofactor self-sufficient by co-immobilization of pyridoxal 5'-phosphate and lysine decarboxylase for cadaverine production. *Bioresource Technology Reports*, 2022, vol. 17, 100939
- **S. KIND ; S. NEUBAUER ; J. BECKER ; M. YAMAMOTO ; M. VÖLKERT ; G ABENDROTH ; O. ZELDER ; C. WITTMANN**. From zero to hero - production of bio-based nylon from renewable resources using engineered Corynebacterium glutamicum. *Metabolic engineering*, 2014, vol. 25, 113-123
- **W. MA ; K. CHEN ; Y. LI ; N. HAO ; X. WANG ; P. OUYANG**. Advances in Cadaverine Bacterial Production and Its Applications. *Engineering*, 2017, vol. 3, 308-317
- **A. DELAVARDE ; G SAVIN ; P. DERKENNE ; M. BOURSIER ; R. MORALES-CERRADA ; B. NOT-TELET ; J. PINAUD ; S. CAILLOL**. Sustainable polyurethanes: toward new cutting-edge opportunities. *Progress in Polymer Science*, 2024, vol. 151, 101805
- **S. SAMANTA ; S. SELVAKUMAR ; J. BAHR ; D. S. WICKRAMARATNE ; M. SIBI ; B. J. CHISHOLM**. Synthesis and Characterization of Polyurethane Networks Derived from Soybean-OilBased Cyclic Carbonates and Bioderivable Diamines. *ACS Sustainable Chemistry & Engineering*, 2016, vol. 4, 6551-6561
- **L. L. CRUZ ; B. S. F. DA SILVA ; G G ARAUJO ; T. LEAL-SILVA ; V. G PAULA ; M. R. SOUZA ; T. S. SOARES ; R. Q. MORAES-SOUZA ; G C. MON-TEIRO ; G P. P. LIMA**. Phytochemical and antidiabetic analysis of Curatella americana L. aqueous extract on the rat pregnancy. *Journal of Ethnopharmacology*, 2022, vol. 293, 115287

- **R. CHEN** ; **C. LIAO** ; **Q. GUO** ; **L. WU** ; **L. ZHANG** ; **X. WANG**. Combined systems pharmacology and fecal metabonomics to study the biomarkers and therapeutic mechanism of type 2 diabetic nephropathy treated with Astragalus and Leech. *RSC Adv*, 2018, vol. 8, 27448-27463
- **S. OZMEN** ; **S. TABUR** ; **S. ONEY-BIROL**. Alleviation role of exogenous cadaverine on cell cycle, endogenous polyamines amounts and biochemical enzyme changes in barley seedlings under drought stress. *Scientific Reports*, 2023, vol. 13, 17488
- **F. CASSÁN** ; **S. MAIALE** ; **O. MASCIARELLI** ; **A. VIDAL** ; **V. LUNA** ; **O. RUIZ**. Cadaverine production by Azospirillum brasilense and its possible role in plant growth promotion and osmotic stress mitigation. *European Journal of Soil Biology*, 2009, vol. 45, 12-19
- **X. LV** ; **Z. MA** ; **X. LI** ; **Y. ZHANG** ; **Y. HUANG** ; **T. LI**. Highly efficient decarboxylation of L-lysine to cadaverine catalyzed by supported ruthenium oxide. *Catalysis Communications*, 2021, vol. 158, 106339
- **H. T. KIM** ; **K.-A. BARITUGO** ; **Y. H. OH** ; **S. M. HYUN** ; **T. U. KHANG** ; **K. H. KANG** ; **S. H. JUNG** ; **B. K. SONG** ; **K. PARK** ; **I.-K. KIM**. Metabolic Engineering of Corynebacterium glutamicum for the High-Level Production of Cadaverine That Can Be Used for the Synthesis of Biopolyamide 510. *ACS Sustainable Chemistry & Engineering*, 2018, vol. 6, 5296-5305
- **H. T. KIM** ; **K.-A. BARITUGO** ; **Y. H. OH** ; **K.-H. KANG** ; **Y. J. JUNG** ; **S. JANG** ; **B. K. SONG** ; **I.-K. KIM** ; **M. O. LEE** ; **Y. T. HWANG**. *High-Level Conversion of l-lysine into Cadaverine by Escherichia coli Whole Cell Biocatalyst Expressing Hafnia alvei l-lysine Decarboxylase*, 2019, vol. 11, 1184
- **Y. M. MOON** ; **S. Y. YANG** ; **T. R. CHOI** ; **H. R. JUNG** ; **H. S. SONG** ; **Y. H. HAN** ; **H. Y. PARK** ; **S. K. BHATIA** ; **R. GURAV** ; **K. PARK**. Enhanced production of cadaverine by the addition of hexadecyltrimethylammonium bromide to whole cell system with regeneration of pyridoxal-5'-phosphate and ATP. *Enzyme and microbial technology*, 2019, vol. 127, 58-64
- **U. KANJEE** ; **I. GUTSCHE** ; **E. ALEXOPOULOS** ; **B. ZHAO** ; **M. EL BAKKOURI** ; **G THIBAULT** ; **K. LIU** ; **S. RAMACHANDRAN** ; **J. SNIDER** ; **E. F. PAI**. Linkage between the bacterial acid stress and stringent responses: the structure of the inducible lysine decarboxylase. *The EMBO journal*, 2011, vol. 30, 931-944
- **R. ADALAT** ; **F. SALEEM** ; **N. CRICKMORE** ; **S. NAZ** ; **A. R. SHAKOORI**. *In Vivo Crystallization of Three-Domain Cry Toxins*, 2017, vol. 9, 80
- **X. XIONG** ; **B. S. HEATER** ; **W. Y. HO** ; **H. K. LEE** ; **M. M. LEE** ; **M. K. CHAN**. Genetically Encoded Multi-enzyme Particles for the Biosynthesis of Putrescine from l-Arginine. *ACS Sustainable Chemistry & Engineering*, 2024, vol. 12, 773-784
- **M. M. BRADFORD**. A rapid and sensitive method for the quantitation of microgram quantities of protein utilizing the principle of protein-dye binding. *Anal. Biochem.*, 1976, vol. 72, 248-254
- **T. WEN** ; **F. QU** ; **N. B. LI** ; **H. Q. LUO**. A facile, sensitive, and rapid spectrophotometric method for copper(II) ion detection in aqueous media using polyethyleneimine. *Arabian Journal of Chemistry*, 2017, vol. 10, S1680-S1685
- **A. ALEHOSSEINI** ; **E.-M. GOMEZ DEL PULGAR** ; **M. J. FABRA** ; **L. G GÓMEZ-MASCARAQUE** ; **A. BENÍTEZ-PÁEZ** ; **M. SARABI-JAMAB** ; **B. GHORANI** ; **A. LOPEZ-RUBIO**. Agarose-Based Freeze-Dried Capsules Prepared by the Oil-Induced Biphasic Hydrogel Particle Formation Approach for the Protection of Sensitive Probiotic Bacteria. *Food Hydrocoll.*, 2019, vol. 87, 487-496
- **A. ALEHOSSEINI** ; **E.-M. GOMEZ DEL PULGAR** ; **M. J. FABRA** ; **L. G GÓMEZ-MASCARAQUE** ; **A. BENÍTEZ-PÁEZ** ; **M. SARABI-JAMAB** ; **B. GHORANI** ; **A. LOPEZ-RUBIO**. Agarose-based freeze-dried capsules prepared by the oil-induced biphasic hydrogel particle formation approach for the protection of sensitive probiotic bacteria. *Food Hydrocolloids*, 2019, vol. 87, 487-496
- **G WEI** ; **Y. CHEN** ; **N. ZHOU** ; **Q. LU** ; **S. XU** ; **A. ZHANG** ; **K. CHEN** ; **P. OUYANG**. Chitin biopolymer mediates self-sufficient biocatalyst of pyridoxal 5'-phosphate and L-lysine decarboxylase. *Chemical Engineering Journal*, 2022, vol. 427, 132030
- **N. ZHOU** ; **G WEI** ; **X. CHEN** ; **B. WU** ; **H. LI** ; **Q. LU** ; **X. CAO** ; **A. ZHANG** ; **K. CHEN** ; **P. OUYANG**. Self-sufficient biocatalysts constructed using chitin-based microspheres. *Chemical Engineering Journal*, 2023, vol. 459, 141660